# EUROPEAN PATENT APPLICATION

(11) **EP 2 019 102 A1**
(43) Date of publication of application: **28.01.2009**
(21) Application number: 07075641.6
(22) Date of filing: 24.07.2007
(51) Int. Cl.: C07D 405/12, C07D 209/14, A61K 31/404, A61P 15/16

(54) **Alkylacetylene substituted Acyltryptophanols**

(71) Applicant: Bayer Schering Pharma AG, 13353 Berlin (DE)
(72) Inventor: KOPPITZ, Dr. Marcus, 10115 Berlin (DE); WORTMANN, Dr. Lars, 10435 Berlin (DE); SCHREY, Anna Katharina, 10179 Berlin (DE); LIESENER, Dr. Florian Peter, 83308 Trostberg (DE); FRENZEL, Dr. Thomas, 65719 Hofheim (Taunus) (DE); MUHN, Dr. Hans-Peter, 13465 Berlin (DE); KUEHNE, Ronald, 12683 Berlin (DE)

(57) **Abstract**

The present invention relates to acyltryptophanols of the general formula I, 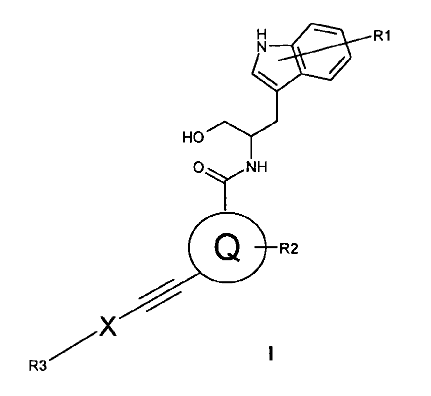 in which R1, R2, R3, Q and X have the meaning as defined in the description.

The compounds according to the invention are effective FSH antagonists and can be used for example for fertility control in men or in women, or for the prevention and/or treatment of osteoporosis.

## Description

The present invention relates to novel alkylacetylene substituted acyltryptophanols with FSH-receptor antagonist activity. The present invention also relates to a process for their preparation, pharmaceutical compositions comprising the compounds according to the invention, and the use thereof for fertility control in men or women, for the treatment and/or prevention of osteoporosis.

Follicle-stimulating hormone (FSH) and luteinizing hormone (LH) are together responsible for the control of male and female fertility and of the production of sex steroids.

In the female mammal, FSH controls the early ripening of ovarian primary follicles and the biosynthesis of sex steroids. In the advanced stage of differentiation (preantral follicles), the influence of LH becomes increasingly important for further development of the follicles until ovulation occurs.

In male mammals, FSH is primarily responsible for the differentiation and stimulation of Sertoli cells. Their function consists of assisting spermatogenesis on many levels. LH is primarily responsible for stimulating the Leydig cells and thus androgen production. FSH, LH and TSH (thyrotropic hormone) together form the group of glycoprotein hormones which are formed in the pituitary and are secreted from there. Whereas the alpha subunit is common to the three hormones, their specificity of action is determined by the beta chain which is unique in each case. The molecular weight of FSH including the sugar portion is about 30 kD.

FSH and the other glycoprotein hormones act specifically via their selectively expressed G protein-coupled receptor (GPCR). FSH stimulates, through binding to its receptor, the association thereof with a stimulating G protein (Gₛ) which is thereby stimulated to hydrolyse guanosine triphosphate (GTP) and to activate the membrane-associated adenylate cyclase. Cyclic adenosine monophosphate (cAMP) is accordingly an important and readily quantifiable secondary messenger substance of FSH (G. Vassart, L. Pardo, S. Costagliola, Trends Biochem. Sci. 2004, 29, 119-126).

The importance of FSH for male fertility is the subject of intensive research. It has been possible to show that FSH influences several processes of spermatogenesis such as the proliferation of spermatogonia, the antiapoptotic effect on spermatogonia and spermatocytes and the stimulation of sperm maturation including motility thereof.

The following arguments are also in favour of the FSH receptor as target for male fertility control:
1. The FSH receptor is exclusively expressed on Sertoli cells (high specificity).
2. Contraceptive vaccination against FSH beta chain or the FSH receptor induces infertility in male primates (N. R. Mougdal, M. Jeyakumar, H. N. Krishnamurthy, S. Sridhar, H. Krishnamurthy, F. Martin, Human Reproduction Update 1997, 3, 335-346).
3. Naturally occurring mutations in the FSH receptor or the FSH beta chain may lead to sub- or infertility in men (I. Huhtaniemi, Journal of Reproduction and Fertility 2000, 119, 173-186; L. C. Layman, P. G. McDonough, Molecular and Cellular Endocrinology 2000, 161, 9-17).
4. Neutralizing FSH antiserum has no effect on testis weight and testosterone production (V. Sriraman, A. J. Rao, Molecular and Cellular Endocrionology 2004, 224, 73-82). Adverse effects of FSH blockade on androgen production therefore appear unlikely.

In line with these arguments, FSH antagonists are expected to be suitable for spermatogenesis inhibition (prevention) in men. Moreover, a suitable FSH antagonist may just as well lead to infertility in women, because it suppresses follicle ripening and thus also ovulation. On the other hand, the skilled person expects advantages from non-peptidergic FSH agonists when used to promote fertility in women (stimulation of follicle ripening). There are no reports of experience on the use of FSH or FSH agonists in male infertility, but specific indications are also conceivable in this connection.
New findings demonstrate that there is also a direct effect of FSH on cells of bone metabolism. There are two fundamentally different cell types in bones: osteoclasts and osteoblasts. While osteoclasts play a central role in bone resorption (breakdown of bone), osteoblasts simulate bone density (anabolic effect).
FSH receptors have been detected in osteoclasts but not in osteoblasts. In vitro, FSH stimulates bone resorption by mouse osteoclasts ( Li Sun et al. Cell 2006; 125: 247-60). A clinical correlation between the serum FSH level and low bone density has been observed in postmenopausal women (Devleta et al, J. Bone Miner. Metab. 2004, 22: 360-4).

These findings among others suggest that FSH stimulates loss of bone mass, and consequently FSH antagonists will display an antiresorptive effect on bone and are therefore suitable for the therapy and/or prevention of peri- and postmenopausal loss of bone mass and osteoporosis.
FSH receptor modulators are compounds that have a mixed profile of both FSH receptor antagonistic and FSH receptor agonistic properties. FSH receptor modulators of various compound classes of low molecular weight, have been reported on recently. FSH receptor modulators are disclosed in WO 2004/056779, WO 2004/056780; J. Med. Chem. 2005, 48, 1697 [tetrahydroquinolines]; WO 02/70493, Bioorg. Med. Chem. Lett. 2004, 14, 1713 and 1717 [diketopiperazines]; WO 01/47875 [sulphonamides] and EP07090087.3 [hydroxyethyltryptamines].
FSH receptor agonists are disclosed in WO 02/09706; J. Comb. Chem. 2004, 6, 196 [Thiazolidinones]; WO 2003/020726 and WO 03/20727, Chem. Biochem. 2002, 10, 1023 {thieno[2,3-d] pyrimidines)}; WO 01/87287 [pyrazoles]; WO 00/08015 [carbazoles]; WO 06/117023, WO 06/117368, WO 06/117370 and WO 06/117371 [hexahydroquinolines].
FSH receptor antagonists are disclosed in WO 03/004028 [tetrahydroquinolines], WO 02/09705 [thiazolidinones], WO 00/58277, Bioorg. Med. Chem. 2002, 10, 639 [sulphonic acids]; WO 00/58276, Endocr. 2002, 143, 3822; Synth. Comm. 2002, 32, 2695 [azo compounds]; US 2006/0199806, US 2006/0258644, US 2006/0258645, US 2006/0287522 [pyrrolobenzodiazepines], WO 2007/017289 [acyltryptophanols], EP06090223.6 [1,2-diarylacetylene derivatives of acyltryptophanols], EP06077263.9 [bicyclic acyltryptophanols], EP07090034.5 [sulfonyltryptophanols] and EP07090059.2 [tetrahydrocarbazoles].

WO 2007/017289 is considered to be the closest prior art.

In view of the prior art, the objective technical problem to be solved according to the present invention may therefore be seen in providing alternative compounds having a FSH receptor antagonistic activity.

The technical problem has been solved according to the present invention by the provision of novel compounds of the formula I 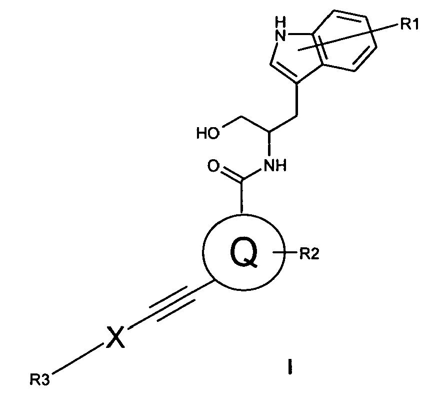 in which
- R1: is hydrogen, halogen, cyano, -SO₂Me, C₁-C₆-alkyl, C₂-C₆-alkenyl, C₂-C₆-alkynyl or C₁-C₆-alkyloxy,
where the hydrocarbon chains therein may optionally be substituted one or more times by fluorine and/or cyano;
- R2: is hydrogen, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano or hydroxy;
- R3: is hydroxy, amino, cyano, C₁-C₆-alkyl, C₂-C₆-alkenyl or C₂-C₆-alkynyl, C₃-C₇-cycloalkyl, C₃-C₇-cycloalkenyl, C₃-C₇-heterocycloalkyl,
where the hydrocarbon chains therein may optionally be substituted one or more times by fluorine or cyano
or hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cycloalkyl-C₁- C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino, C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneamino-carbonyl, C₁-C₆-alkylcarbonyl, C₃-C₇-cycloalkylcarbonyl, C₁-C₆-acyloxy, carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl, -N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl, -C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine, -S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine, -O-C₂-C₆-alkylene-(C₁-C₆-alkyl)amine, -O-C₂-C₆-alkylene-[di(C₁-C₆-alkylene)]amine, or the radicals: 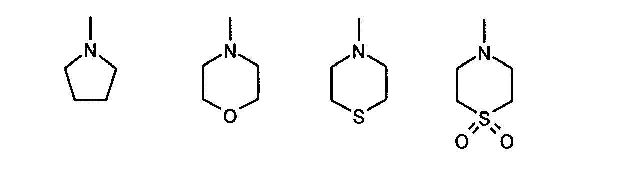 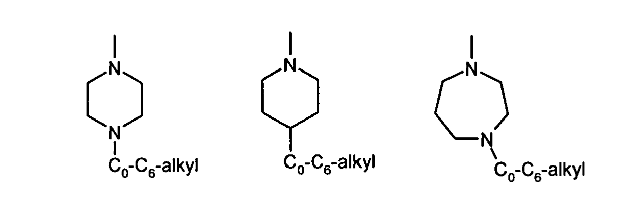 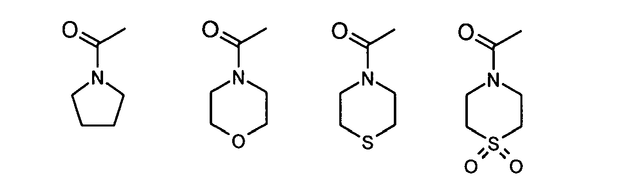 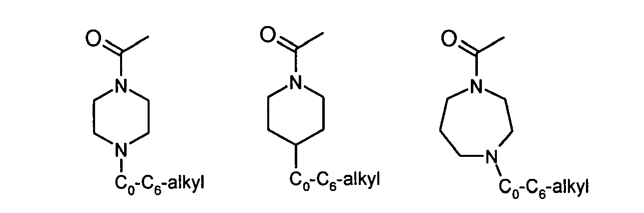 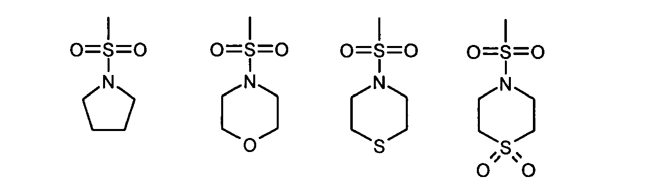 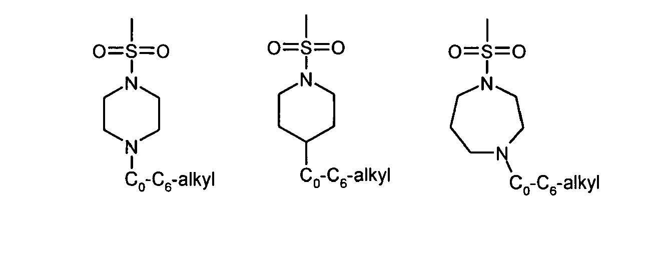 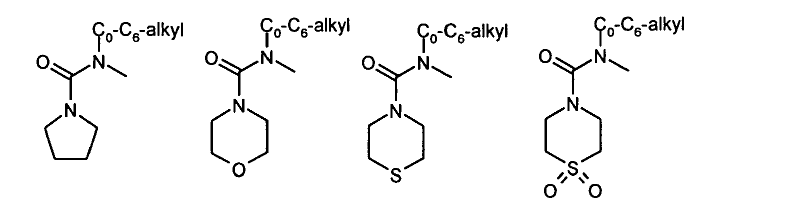 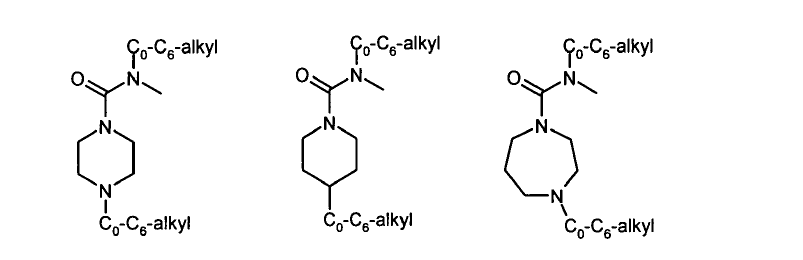 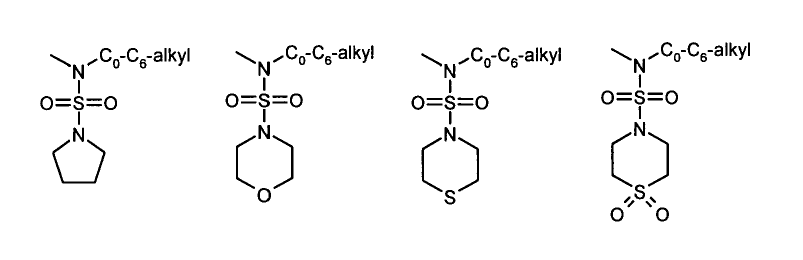 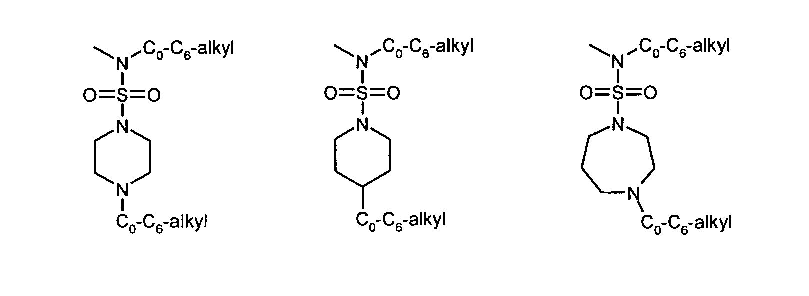 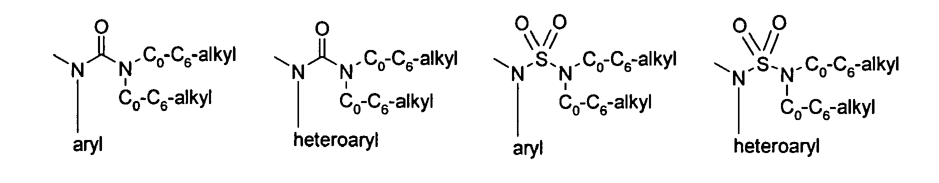 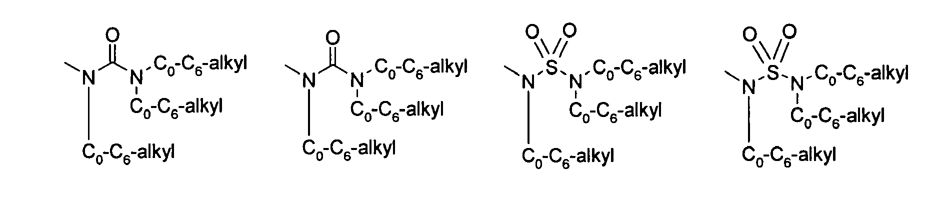 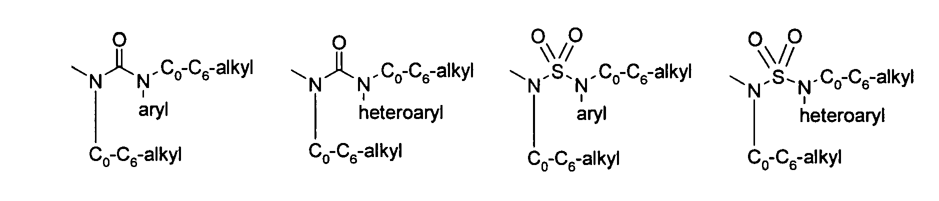 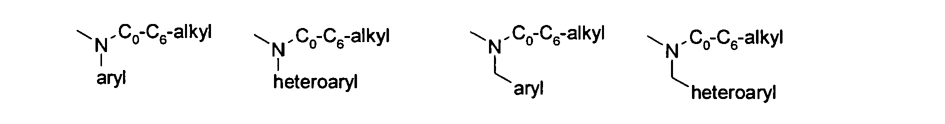 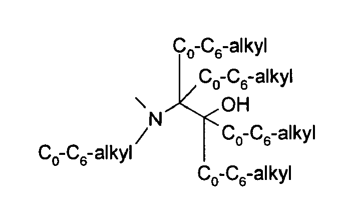 in which the aryl or heteroarylgroup may optionally be substituted with halogen, cyano, SO₂Me, C₁-C₆-alkyl, C₂-C₆-alkenyl, C₂-C₆-alkynyl or C₁-C₆-alkyloxy, where the hydrocarbon chains therein may optionally be substituted one or more times by fluorine and/or cyano;
and
- X: is a bond, C₁-C₄-alkylene, C₂-C₄-alkenylene or C₂-C₄-alkynylene;
- Q: is an aryl or heteroaryl group, or the group AV 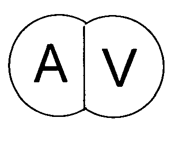
in which
- A: is a monocyclic aryl or a monocyclic heteroaryl group;
- V: is a cycloalkylen, cycloalkenylen, heterocycloalkylen or heterocycloalkenylen group;
where
- R1: substitutes one or more positions of the aryl or heteroaryl ring in the indole residue;
- R2: substitutes one or more positions of the aryl or heteroaryl ring in the radicals Q, A or V;
- R3: substitutes one or more positions of the group X.

The present invention relates to both possible enantiomeric forms at the stereocentre of the tryptophanol residue.

The unbranched C₁-C₆-alkyl groups for the radicals R1 to R3 may be for example a methyl, ethyl, propyl, butyl, pentyl or a hexyl group; and the branched C₃-C₆-alkyl groups for the radicals R1 to R3 may be an isopropyl, isobutyl, *sec*-butyl, *tert*-butyl, isopentyl, 2-methylbutyl, 1-methylbutyl, 1-ethylpropyl, *neopent*yl*,* 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or a 1,2-dimethylbutyl group.
The branched or unbranched C₂-C₆-alkenyl groups for the radicals R1 to R3 may be for example a vinyl, allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-en-yl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methyl-pent-3-enyl, (*E*)-2-methylpent-3-enyl, (*Z*)-2-methylpent-3-enyl, (*E*)-1-methylpent-3-enyl, (*Z*)-1-methylpent-3-enyl, (*E*)-4-methylpent-2-enyl, (Z)-4-methylpent-2-enyl, (*E*)-3-methyl-pent-2-enyl, (*Z*)-3-methylpent-2-enyl, (*E*)-2-methylpent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methyl-pent-1-enyl, (*E*)-3-methylpent-1-enyl, (*Z*)-3-methylpent-1-enyl, (*E*)-2-methylpent-1-enyl, (*Z*)-2-methylpent-l-enyl, (*E*)-1-methylpent-l-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethylbut-2-enyl, (*Z*)-1-ethylbut-2-enyl, (*E*)-3-ethylbut-l-enyl, (*Z*)-3-ethylbut-l-enyl, 2-ethylbut-1-enyl, (*E*)-1-ethylbut-1-enyl, (Z)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propyl-prop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-en-yl, (*Z*)-3,3-dimethylprop-1-enyl- or a 1-(1,1-dimethylethyl)ethenyl group.

The C₂-C₆-alkynyl groups for the radicals R1 to R3 may be for example an ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethyl-but-3-ynyl, 1-ethylbut-2-ynyl, 1-propylprop-2-ynyl, 1-isopropylprop-2-ynyl, 2,2-dimethyl-but-3-ynyl, 1,1-dimethylbut-3-ynyl, 1,1-dimethylbut-2-ynyl or a 3,3-dimethylbut-1-ynyl group.

The C₁-C₆-alkyloxy groups for the radicals R1 to R3 may be for example a methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy group.

The halogens for the radicals R1 to R3 are fluorine, chlorine, bromine or iodine.

The C₁-C₃-alkylsulphanyl groups for the radical R3 may be for example a methyl-sulphanyl (CH₃S-), ethylsulphanyl (CH₃CH₂S-), propylsulphanyl, isopropylsulphanyl group.

The C₁-C₆-alkylaminocarbonyl groups for the radical R3 may be for example a methylaminocarbonyl-, ethylaminocarbonyl-, propylaminocarbonyl-, *iso*propylaminocarbonyl-, butylaminocarbonyl-, *iso*butylaminocarbonyl-, *sec*-butylaminocarbonyl-, *tert-*butylaminocarbonyl-, pentylaminocarbonyl-, isopentylaminocarbonyl-, (2-methylbutyl)-aminocarbonyl-, (1-methylbutyl)aminocarbonyl-, (1-ethylpropyl)aminocarbonyl-, *neo-pent*ylaminocarbonyl-, (1,1-dimethylpropyl)aminocarbonyl-, hexylaminocarbonyl-, (4-methylpentyl)aminocarbonyl-, (3-methylpentyl)aminocarbonyl-, (2-methylpentyl)aminocarbonyl-, (1-methylpentyl)aminocarbonyl-, (1-ethylbutyl)aminocarbonyl-, (2-ethylbutyl)-aminocarbonyl-, (3,3-dimethylbutyl)aminocarbonyl-, (2,2-dimethylbutyl)aminocarbonyl-, (1,1-dimethylbutyl)aminocarbonyl-, (2,3-dimethylbutyl)aminocarbonyl-, (1,3-dimethylbutyl)aminocarbonyl- or a (1,2-dimethylbutyl)aminocarbonyl group.
The hydroxy-C₁-C₆-alkylene groups for the radical R3 may be a hydroxymethyl (HOCH₂-), 2-hydroxyethyl (HOCH₂CH₂-), 1-hydroxyethyl [CH₃CH(OH)-], 3-hydroxypropyl (HOCH₂CH₂CH₂-), 2-hydroxypropyl [CH₃CH(OH)CH₂-], 1-hydroxypropyl [CH₃CH₂CH(OH)-], 2-hydroxy-1-methylethyl [HOCH₂CH(CH₃)-], 1-hydroxy-1-methylethyl [(CH₃)₂C(OH)-], 4-hydroxybutyl (HOCH₂CH₂CH₂CH₂-), 3-hydroxybutyl [CH₃CH(OH)CH₂CH₂-], 2-hydroxybutyl [CH₃CH₂CH(OH)CH₂-], 1-hydroxybutyl [CH₃CH₂CH₂CH(OH)-], 3-hydroxy-1-methylpropyl [HOCH₂CH₂CH(CH₃)-], 2-hydroxy-1-methylpropyl [CH₃CH(OH)CH(CH₃)-], 1-hydroxy-1-methylpropyl [CH₃CH₂C(CH₃)(OH)-], 1-(hydroxymethyl)propyl [CH₃CH(CH₂OH)-], 3-hydroxy-2-methylpropyl [HOCH₂CH(CH₃)CH₂-], 2-hydroxy-2-methylpropyl [(CH₃)₂C(OH)CH₂-], 1-hydroxy-2-methylpropyl [CH₃CH(CH₃)CH(OH)-] or a 2-hydroxy-1,1-dimethylethyl group [HOCH₂C(CH₃)₂-].

The C₃-C₇-cycloalkyl groups for the radicals R2 to R3 may be for example a cyclopropyl, cyclobutyl, cyclopentyl, cyclohexyl, cycloheptyl group.

The C₃-C₇-heterocycloalkyl groups for the radicals R2 to R3 may be for example a cyclopropyl, cyclobutyl, cycopentyl, cyclohexyl, cycloheptyl group in which one or two carbon atoms of the ring are replaced independently of one another by an oxygen, nitrogen or sulphur atom.

The monocyclic aryl group for A may be for example a phenyl group which is linked via substitutable positions
The aryl group for Q or R3 may be for example a phenyl, naphthyl group which is linked via substitutable positions.
The monocylic heteroaryl group for A may be for example a pyridinyl, pyrimidinyl, furanyl, thienyl, oxazolyl, isoxazolyl, thiazolyl, pyrrolyl, pyrazolyl or an imidazolyl group which is linked via substitutable positions.

The heteroaryl group for Q or R3 may be for example a pyridinyl, pyrimidinyl, quinolinyl, isoquinolinyl, quinazolinyl, quinoxalinyl, phthalazinyl, 1.5-naphthyridinyl, 1,6-naphthyridinyl, 1,7-naphthyridinyl, 1,8-naphthyridinyl, benzofuranyl, benzothienyl, 1,3-benzo-dioxolyl, 2,1,3-benzothiadiazolyl, indolyl, indazolyl, furanyl, thienyl, oxazolyl, isoxazolyl, thiazolyl, pyrrolyl, pyrazolyl or an imidazolyl group which is linked via substitutable positions.

The heterocycloalkylen groups for V may be for example the following groups:

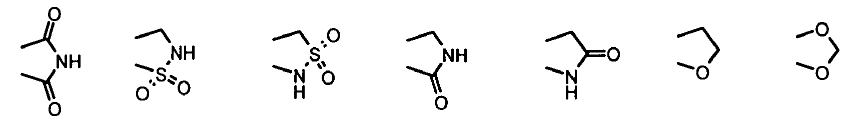

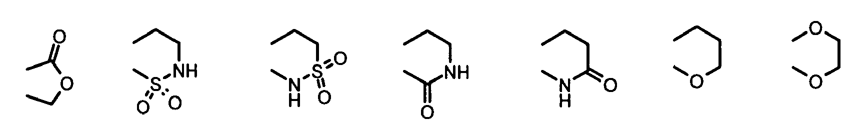

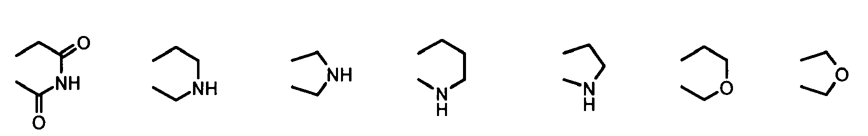

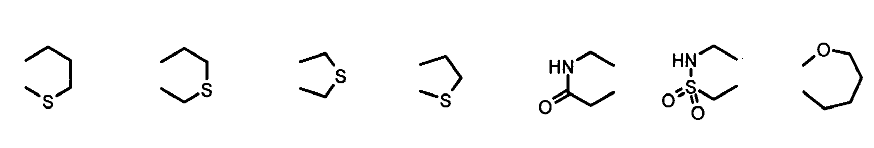

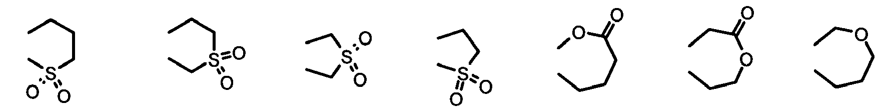

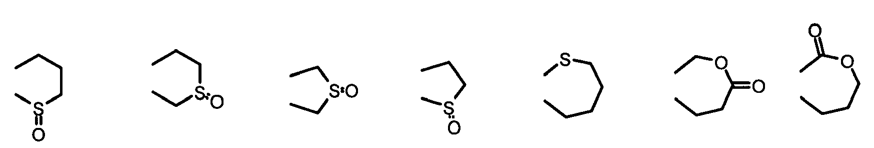

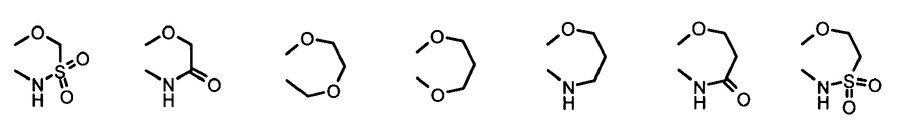

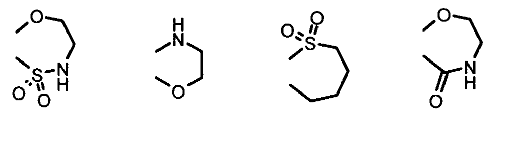

The heterocycloalkenylen groups for V may be for example the following groups:

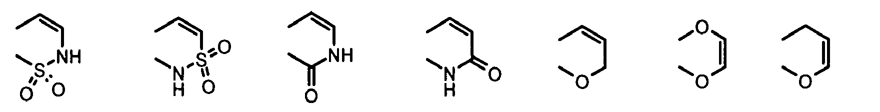

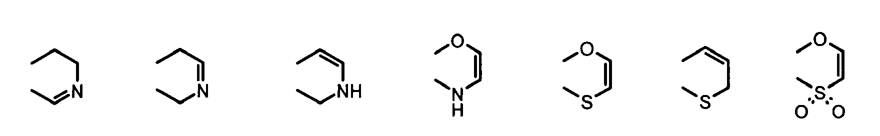

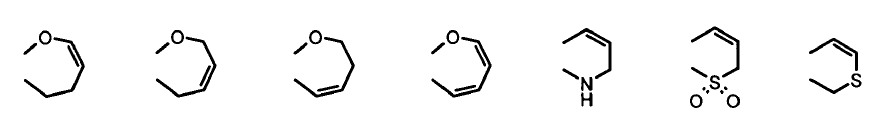

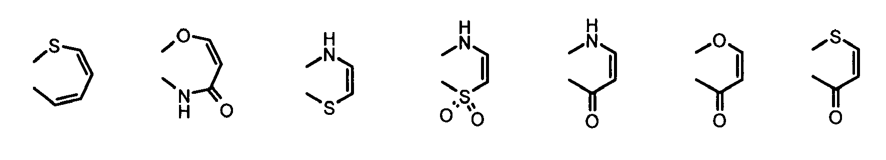

The cycloalkylen groups for V may be for example the following groups:

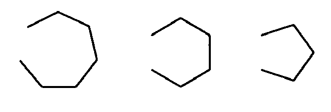

The cycloalkenylen groups for V may be for example the following groups:

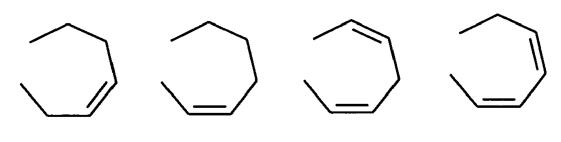

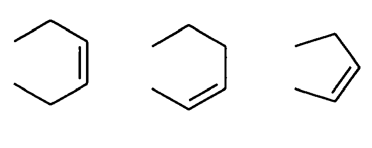

The C₁-C₄-alkylene groups for the radical X may be for example a methylene (-CH₂-), ethylidene [-CH(CH₃)-], ethylene (-CH₂CH₂-), prop-1,3-ylene (-CH₂CH₂CH₂-), prop-1,2-ylene [-CH₂CH(CH₃)-], but-1,4-ylene (-CH₂CH₂CH₂CH₂-), but-1,3-ylene [-CH₂CH₂CH(CH₃)-], but-1,2-ylene [-CH₂CH(CH₂CH₃)-], but-2,3-ylene [-CHCH(CH₃)-], 2-methylprop-1,2-ylene [-CH₂C(CH₃)₂-] or a 2-methylprop-1,3-ylene group [-CH₂CH(CH₃)CH₂-].

The C₂-C₄-alkenylene groups for the radical X may be for example an ethen-1,2-ylidene (-CH=CH-), prop-2-en-1,3-ylidene (-CH₂-CH=CH-), prop-1-en-1,3-ylidene (-CH=CH-CH₂-), but-1-en-1,4-ylidene (-CH=CH-CH₂-CH₂-), but-2-en-1,4-ylidene (-CH₂-CH=CH-CH₂-) or a but-3-en-1,4-ylidene group (-CH₂-CH₂-CH=CH-).

The C₂-C₄-alkynylene groups for the radical X may be for example an ethyn-1 ,2-ylidene (-C≡C-), prop-2-yn-1,3-ylidene (-CH₂-C≡C-), prop-1-yn-1,3-ylidene (-C≡C-CH₂-), but-1-yn-1,4-ylidene (-C≡C-CH₂-CH₂-), but-2-yn-1,4-ylidene (-CH₂-C≡C-CH₂-) or a but-3-yn-1,4-ylidene group (-CH₂-CH₂-C≡C-).

The C₃-C₇-cycloalkyloxy groups for the radical R1 to R3 may be for example a cyclopropyloxy, cyclobutyloxy, cyclopentyloxy, cyclohexyloxy, cycloheptyloxy group.
The C₁-C₆-alkylamino groups for the radicals R1 to R6 may be for example methyl-amino, ethylamino, propylamino, *iso*propylamino, butylamino, *iso*butylamino, *sec*-butylamino, *tert*-butylamino, pentylamino, *iso*pentylamino, (2-methylbutyl)amino, (1-methylbutyl)amino, (1-ethylpropyl)amino, *neo*pentylamino, (1,1-dimethylpropyl)amino, hexyl-amino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino group.
In the di(C₁-C₆-alkyl)amino groups for the radicals R1 to R6, each of the two radicals on the nitrogen atom of the dialkylamino group may be chosen independently of one another from the following radicals: possible examples are a methyl, ethyl, propyl, *iso*propyl, butyl, *iso*butyl, *sec*-butyl, *tert*-butyl, pentyl, *iso*pentyl, (2-methylbutyl), (1-methylbutyl), (1-ethylpropyl), *neo*pentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the C₃-C₇-cycloalkyl-C₁-C₆-alkyleneoxy groups for the radicals R2 to R3 it is possible to combine each of the C₃-C₇-cycloalkyl groups of the C₃-C₇-cycloalkyl-C₁-C₆-alkyleneoxy group, for example of a cyclopropyl, cyclobutyl, cyclopentyl, cyclohexyl or cycloheptyl group, independently of one another with each C0-C6-alkyleneoxy group, for example with a methyleneoxy, ethyleneoxy, propyleneoxy, butyleneoxy, pentyleneoxy, hexyleneoxy group.

In the hydroxy-C₃-C₆-alkenylene groups for the radicals R1 to R6 it is possible for the hydroxy group to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-Pent-2-enyl-, (*Z*)-Pent-2-enyl-, (*E*)-Pent-1-enyl-, (*Z*)-Pent-1-enyl-, hex-5-enyl-, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethyl-prop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E*)-3-methylpent-3-enyl, (*Z*)-3-methylpent-3-enyl, (*E*)-2-methylpent-3-enyl, (*Z*)-2-methylpent-3-enyl, (*E*)-1-methyl-pent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methylpent-2-enyl, (*Z*)-2-methyl-pent-2-enyl, (*E*)-1-methylpent-2-enyl, (*Z*)-1-methylpent-2-enyl, (*E*)-4-methylpent-1-enyl, (Z)-4-methylpent-1-enyl, (*E*)-3-methylpent-1-enyl, (*Z*)-3-methylpent-1-enyl, (*E*)-2-methyl-pent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethyl-but-2-enyl, (*E*)-2-ethylbut-2-enyl, (*Z*)-2-ethylbut-2-enyl, (*E*)-1-ethylbut-2-enyl, (*Z*)-1-ethyl-but-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-iso-propylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group, and to be combined independently of one another.
In the hydroxy-C₃-C₆-alkynyl groups for the radicals R2 to R3 it is possible for the hydroxy group to be located at any desired position of the C₃-C₆-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group.
In the C₁-C₆-alkyloxy-C₃-C₆-alkenylene groups for the radicals R2 to R3 it is possible for the C₁-C₆-alkyloxy group, for example a methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1 ,3-dimethylbutyl)oxy or a (1,2-dimethylbutyl)oxy group, to be located on any desired position of the C₃-C₆-alkenyl group, for example of an allyl, (*E*)-2-methylvinyl, (*Z*)-2-methylvinyl, homoallyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methyl-prop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z)*-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (*E)*-3-methylpent-3-enyl, (*Z*)-3-methylpent-3-enyl, (*E*)-2-methylpent-3-enyl, (*Z*)-2-methyl-pent-3-enyl, (*E*)-1-methylpent-3-enyl, (*Z*)-1-methylpent-3-enyl, (*E*)-4-methylpent-2-enyl, (*Z*)-4-methylpent-2-enyl, (*E*)-3-methylpent-2-enyl, (*Z*)-3-methylpent-2-enyl, (*E*)-2-methyl-pent-2-enyl, (*Z*)-2-methylpent-2-enyl, (*E*)-1-methylpent-2-enyl, (*Z*)-1-methylpent-2-enyl, (*E*)-4-methylpent-1-enyl, (*Z*)-4-methylpent-1-enyl, (*E*)-3-methylpent-1-enyl, (*Z*)-3-methyl-pent-1-enyl, (*E*)-2-methylpent-1-enyl, (*Z*)-2-methylpent-1-enyl, (*E*)-1-methylpent-1-enyl, (*Z*)-1-methylpent-1-enyl, 3-ethylbut-3-enyl, 2-ethylbut-3-enyl, 1-ethylbut-3-enyl, (*E*)-3-ethylbut-2-enyl, (*Z*)-3-ethylbut-2-enyl, (*E*)-2-ethylbut-2-enyl, (*Z*)-2-ethylbut-2-enyl, (*E*)-1-ethylbut-2-enyl, (*Z*)-1-ethylbut-2-enyl, (*E*)-3-ethylbut-1-enyl, (*Z*)-3-ethylbut-1-enyl, 2-ethylbut-1-enyl, (*E*)-1-ethylbut-1-enyl, (*Z*)-1-ethylbut-1-enyl, 2-propylprop-2-enyl, 1-propylprop-2-enyl, 2-isopropylprop-2-enyl, 1-isopropylprop-2-enyl, (*E*)-2-propylprop-1-enyl, (*Z*)-2-propylprop-1-enyl, (*E*)-1-propylprop-1-enyl, (*Z*)-1-propylprop-1-enyl, (*E*)-2-isopropylprop-1-enyl, (*Z*)-2-isopropylprop-1-enyl, (*E*)-1-isopropylprop-1-enyl, (*Z*)-1-isopropylprop-1-enyl, (*E*)-3,3-dimethylprop-1-enyl, (*Z*)-3,3-dimethylprop-1-enyl or a 1-(1,1-dimethylethyl)ethenyl group and to be combined independently of one another.
In the C₁-C₆-alkyloxy-C₃-C₆-alkynylene groups for the radicals R1 to R6 it is possible for the C₁-C₆-alkyloxy group, for example a methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)oxy, (1 ,3-dimethylbutyl)oxy or a (1,2-dimethylbutyl)oxy group, to be located at any desired position of the C3-C6-alkynyl group, for example of a prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methyl-but-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-yn-yl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or a 3,3-dimethylbut-1-ynyl group, and to be combined independently of one another.
In the C₁-C₆-alkyloxyphenyl-C₁-C₆-alkylene groups for the radical R2 to R3 it is possible for the C₁-C₆-alkyloxy group to be selected independently of one another from methyloxy, ethyloxy, propyloxy, isopropyloxy, butyloxy, isobutyloxy, *sec*-butyloxy, *tert*-butyloxy, pentyloxy, isopentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy, and to be combined independently of one another with C₁-C₆-alkylene groups such as, for example, methylene, ethylene, propylene, butylene, pentylene, hexylene.
In the C₃-C₇-cycloalkyl-(C₀-C₆)-alkyleneamino groups of the radicals R2 to R3 it is possible for each of the C₃-C₇-cycloalkyl groups of the C₃-C₇-cycloalkyl-(C₀-C₆)-alkylene-amino group, for example of a cyclopropyl, cyclobutyl, cyclopentyl, cyclohexyl or cycloheptyl group, to be combined independently of one another with each C0-C6-alkylene group, for example with a bond, a methylene, ethylene, propylene, butylene, pentylene, hexylene group.
In the C₁-C₆-alkyloxy-C₁-C₆-alkylene groups for the radical R2 to R3, it is possible for the C₁-C₆-alkyloxy group to be selected independently for example from methyloxy, ethyloxy, propyloxy, *iso*propyloxy, butyloxy, *iso*butyloxy, *sec*-butyloxy, *tert*-butyloxy, pentyloxy, *iso*pentyloxy, (2-methylbutyl)oxy, (1-methylbutyl)oxy, (1-ethylpropyl)oxy, *neo*pentyloxy, (1,1-dimethylpropyl)oxy, hexyloxy, (4-methylpentyl)oxy, (3-methylpentyl)-oxy, (2-methylpentyl)oxy, (1-methylpentyl)oxy, (1-ethylbutyl)oxy, (2-ethylbutyl)oxy, (3,3-dimethylbutyl)oxy, (2,2-dimethylbutyl)oxy, (1,1-dimethylbutyl)oxy, (2,3-dimethylbutyl)-oxy, (1,3-dimethylbutyl)oxy or a (1,2-dimethylbutyl)oxy and to be combined independently of one another with C₁-C₆-alkylene groups such as, for example, methylene, ethylene, propylene, butylene, pentylene, hexylene.
In the di(C₁-C₆-alkyl)amino-C₁-C₆-alkylene group for the radical R3 it is possible for each of the two radicals on the nitrogen atom of the amino group to be selected independently for example from methyl, ethyl, propyl, *iso*propyl, butyl, *iso*butyl, *sec*-butyl, *tert*-butyl, pentyl, *iso*pentyl, (2-methylbutyl), (1-methylbutyl), (1-ethylpropyl), *neo*pentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, and to be combined with C₁-C₆-alkylene groups such as, for example, methylene, ethylene, propylene, butylene, pentylene, hexylene.
The C₃-C₇-cycloalkyl-C₁-C₆-alkylene groups for the radicals R2 to R3 may be for example a cyclopropyloxymethylene, cyclopropyloxyethylene, cyclopropyloxypropylene, cyclopropyloxybutylene, cyclopropyloxypentylene, cyclopropyloxyhexylene, cyclobutyloxymethylene, cyclobutyloxyethylene, cyclobutyloxypropylene, cyclobutyloxybutylene, cyclobutyloxypentylene, cyclobutyloxyhexylene, cyclopentyloxymethylene, cyclopentyloxyethylene, cyclopentyloxypropylene, cyclopentyloxybutylene, cyclopentyloxypentylene, cyclopentyloxyhexylene, cyclohexyloxymethylene, cyclohexyloxyethylene, cyclohexyloxypropylene, cyclohexyloxybutylene, cyclohexyloxypentylene, cyclohexyloxyhexylene, cycloheptyloxymethylene, cycloheptyloxyethylene, cycloheptyloxypropylene, cycloheptyloxybutylene, cycloheptyloxypentylene, cycloheptyloxyhexylen group.
In the C₁-C₆-alkylamino-C₁-C₆-alkylene groups for the radicals R2 to R3 it is possible for the C₁-C₆-alkylamino group to be selected independently for example from methyl-amino, ethylamino, propylamino, *iso*propylamino, butylamino, *iso*butylamino, *sec*-butylamino, *tert*-butylamino, pentylamino, *iso*pentylamino, (2-methylbutyl)amino, (1-methylbutyl)amino, (1-ethylpropyl)amino, *neopent*ylamino, (1,1-dimethylpropyl)amino, hexyl-amino, (4-methylpentyl)amino, (3-methylpentyl)amino, (2-methylpentyl)amino, (1-methylpentyl)amino, (1-ethylbutyl)amino, (2-ethylbutyl)amino, (3,3-dimethylbutyl)amino, (2,2-dimethylbutyl)amino, (1,1-dimethylbutyl)amino, (2,3-dimethylbutyl)amino, (1,3-dimethylbutyl)amino or a (1,2-dimethylbutyl)amino and to be combined with C₁-C₆-alkylene groups such as, for example, methylene, ethylene, propylene, butylene, pentylene, hexylene.
The phenyloxy-C₁-C₆-alkylene groups for the radical R2 may be for example a phenyloxymethyl, phenyloxyethyl, phenyloxypropyl, phenyloxybutyl, phenyloxypentyl, phenyloxyhexyl group.
In the C₁-C₆-acyl-(C₀-C₆-alkyl)amido groups for the radical R3, it is possible for each of the C₁-C₆-acyl groups, for example a formyl, acetyl, propionyl, 2-methylpropionyl, 2,2-dimethylpropionyl, butyryl, 2-methylbutyryl, 3-methylbutyryl, 2,2-dimethylbutyryl, 2-ethyl-butyryl, pentanoyl, 2-methylpentanoyl, 3-methylpentanoyl, 4-methylpentanoyl or a hexanoyl group, to be combined independently of one another with each (C₀-C₆-alkyl)-amido group, for example a hydrogen atom, a methylamido, ethylamido, propylamido, isopropylamido, butylamido, isobutylamido, sec-butylamido, tert-butylamido, pentylamido, isopentylamido, (2-methylbutyl)amido, (1-methylbutyl)amido, (1-ethylpropyl)-amido, neopentylamido, (1,1-dimethylpropyl)amido, hexylamido, (4-methylpentyl)amido, (3-methylpentyl)amido, (2-methylpentyl)amido, (1-methylpentyl)amido, (1-ethylbutyl)-amido, (2-ethylbutyl)amido, (3,3-dimethylbutyl)amido, (2,2-dimethylbutyl)amido, (1,1-dimethylbutyl)amido, (2,3-dimethylbutyl)amido, (1,3-dimethylbutyl)amido or a (1,2-dimethylbutyl)amido group.
The C₁-C₆-alkylaminocarbonyl groups for the radicals R4 to R6 may be for example a methylaminocarbonyl, ethylaminocarbonyl, propylaminocarbonyl, isopropylaminocarbonyl, butylaminocarbonyl, isobutylaminocarbonyl, sec-butylaminocarbonyl, tert-butylaminocarbonyl, pentylaminocarbonyl, isopentylaminocarbonyl, (2-methylbutyl)-aminocarbonyl, (1-methylbutyl)aminocarbonyl, (1-ethylpropyl)aminocarbonyl, neopentylaminocarbonyl, (1,1-dimethylpropyl)aminocarbonyl, hexylaminocarbonyl, (4-methylpentyl)aminocarbonyl, (3-methylpentyl)aminocarbonyl, (2-methylpentyl)aminocarbonyl, (1-methylpentyl)aminocarbonyl, (1-ethylbutyl)aminocarbonyl, (2-ethylbutyl)aminocarbonyl, (3,3-dimethylbutyl)aminocarbonyl, (2,2-dimethylbutyl)aminocarbonyl, (1,1-dimethylbutyl)aminocarbonyl, (2,3-dimethylbutyl)aminocarbonyl, (1,3-dimethylbutyl)-aminocarbonyl or a (1,2-dimethylbutyl)aminocarbonyl group.
In the di(C₁-C₆-alkyl)aminocarbonyl groups for the radical R3, each of the two C₁-C₆-alkyl radicals on the nitrogen atom of the di(C₁-C₆-alkyl)aminocarbonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
The (C₃-C₇-cycloalkyl)aminocarbonyl groups for the radical R3 may be for example a cyclopropylaminocarbonyl, cyclobutylaminocarbonyl, cyclopentylaminocarbonyl, cyclohexylaminocarbonyl or cycloheptylaminocarbonyl group.

In the di(C₃-C₇-cycloalkyl)aminocarbonyl groups for the radical R3, each of the two C₃-C₇-cycloalkyl radicals on the nitrogen atom of the di(C₃-C₇-cycloalkyl)aminocarbonyl group may be independently of one another for example a cyclopropyl, cyclobutyl, cyclopentyl, cyclohexyl or cycloheptyl group.
In the C₃-C₇-cycloalkyl-C₁-C₆-alkyleneaminocarbonyl groups of the radical R3 it is possible for each of the C₃-C₇-cycloalkyl groups of the C₃-C₇-cycloalkyl-C₁-C₆-alkylene-aminocarbonyl groups, for example of a cyclopropyl, cyclobutyl, cyclopentyl, cyclohexyl or cycloheptyl group, to be combined independently of one another with each C₁-C₆-alkyleneaminocarbonyl group, for example with a methyleneaminocarbonyl, ethylene-aminocarbonyl, propyleneaminocarbonyl, butyleneaminocarbonyl, pentyleneaminocarbonyl, hexyleneaminocarbonyl group.
The C₁-C₆-alkylcarbonyl groups for the radical R3 may be for example a methylcarbonyl, ethylcarbonyl, propylcarbonyl, isopropylcarbonyl, butylcarbonyl, isobutylcarbonyl, sec-butylcarbonyl, tert-butylcarbonyl, pentylcarbonyl, isopentylcarbonyl, (2-methylbutyl)-carbonyl, (1-methylbutyl)carbonyl, (1-ethylpropyl)carbonyl, neopentylcarbonyl, (1,1-dimethylpropyl)carbonyl, hexylcarbonyl, (4-methylpentyl)carbonyl, (3-methylpentyl)-carbonyl, (2-methylpentyl)carbonyl, (1-methylpentyl)carbonyl, (1-ethylbutyl)carbonyl, (2-ethylbutyl)carbonyl, (3,3-dimethylbutyl)carbonyl, (2,2-dimethylbutyl)carbonyl, (1,1-dimethylbutyl)carbonyl, (2,3-dimethylbutyl)carbonyl, (1,3-dimethylbutyl)carbonyl or a (1,2-dimethylbutyl)carbonyl group.
The C₃-C₇-cycloalkylcarbonyl groups for the radicals R4 to R6 may be for example a cyclopropylcarbonyl, cyclobutylcarbonyl, cyclopentylcarbonyl, cyclohexylcarbonyl or cycloheptylcarbonyl group.
The C₁-C₆-alkyloxycarbonyl groups for the radical R3 may be for example a methyloxycarbonyl, ethyloxycarbonyl, propyloxycarbonyl, isopropyloxycarbonyl, butyloxycarbonyl, isobutyloxycarbonyl, sec-butyloxycarbonyl, tert-butyloxycarbonyl, pentyloxycarbonyl, isopentyloxycarbonyl, (2-methylbutyl)oxycarbonyl, (1-methylbutyl)oxycarbonyl, (1-ethylpropyl)oxycarbonyl, neopentyloxycarbonyl, (1,1-dimethylpropyl)oxycarbonyl, hexyloxycarbonyl, (4-methylpentyl)oxycarbonyl, (3-methylpentyl)oxycarbonyl, (2-methylpentyl)oxycarbonyl, (1-methylpentyl)oxycarbonyl, (1-ethylbutyl)oxycarbonyl, (2-ethylbutyl)oxycarbonyl, (3,3-dimethylbutyl)oxycarbonyl, (2,2-dimethylbutyl)oxycarbonyl, (1,1-dimethylbutyl)oxycarbonyl, (2,3-dimethylbutyl)oxycarbonyl, (1,3-dimethylbutyl)oxycarbonyl or a (1,2-dimethylbutyl)oxycarbonyl group.

The C₁-C₆-alkylsulphonyl groups for the radical R3 may be for example a methylsulphonyl, ethylsulphonyl, propylsulphonyl, isopropylsulphonyl, butylsulphonyl, isobutylsulphonyl, sec-butylsulphonyl, tert-butylsulphonyl, pentylsulphonyl, isopentylsulphonyl, (2-methylbutyl)sulphonyl, (1-methylbutyl)sulphonyl, (1-ethylpropyl)sulphonyl, neopentylsulphonyl, (1,1-dimethylpropyl)sulphonyl, hexylsulphonyl, (4-methylpentyl)sulphonyl, (3-methylpentyl)sulphonyl, (2-methylpentyl)sulphonyl, (1-methylpentyl)sulphonyl, (1-ethylbutyl)sulphonyl, (2-ethylbutyl)sulphonyl, (3,3-dimethylbutyl)sulphonyl, (2,2-dimethylbutyl)sulphonyl, (1,1-dimethylbutyl)sulphonyl, (2,3-dimethylbutyl)sulphonyl, (1,3-dimethylbutyl)sulphonyl or a (1,2-dimethylbutyl)sulphonyl group.
The C₃-C₇-cycloalkylsulphonyl groups for the radical R3 may be for example a cyclopropylsulphonyl, cyclobutylsulphonyl, cyclopentylsulphonyl, cyclohexylsulphonyl or cycloheptylsulphonyl group.
In the C₃-C₇-cycloalkyl-C₁-C₆-alkylenesulphonyl groups of the radical R3 it is possible for each of the C₃-C₇-cycloalkyl groups of the C₃-C₇-cycloalkyl-C₁-C₆-alkylenesulphonyl groups, for example of a cyclopropyl, cyclobutyl, cyclopentyl, cyclohexyl or cycloheptyl group, to be combined independently of one another with each C₁-C₆-alkylenesulphonyl group, for example with a methylenesulphonyl, ethylenesulphonyl, propylenesulphonyl, butylenesulphonyl, pentylenesulphonyl, hexylenesulphonyl group.
The C₁-C₆-alkylaminosulphonyl groups for the radical R3 may be for example a methyl-aminosulphonyl, ethylaminosulphonyl, propylaminosulphonyl, isopropylaminosulphonyl, butylaminosulphonyl, isobutylaminosulphonyl, sec-butylaminosulphonyl, tert-butylaminosulphonyl, pentylaminosulphonyl, isopentylaminosulphonyl, (2-methylbutyl)-aminosulphonyl, (1-methylbutyl)aminosulphonyl, (1-ethylpropyl)aminosulphonyl, neo-pentylaminosulphonyl, (1,1-dimethylpropyl)aminosulphonyl, hexylaminosulphonyl, (4-methylpentyl)aminosulphonyl, (3-methylpentyl)aminosulphonyl, (2-methylpentyl)aminosulphonyl, (1-methylpentyl)aminosulphonyl, (1-ethylbutyl)aminosulphonyl, (2-ethylbutyl)-aminosulphonyl, (3,3-dimethylbutyl)aminosulphonyl, (2,2-dimethylbutyl)aminosulphonyl, (1,1-dimethylbutyl)aminosulphonyl, (2,3-dimethylbutyl)aminosulphonyl, (1,3-dimethylbutyl)aminosulphonyl or a (1,2-dimethylbutyl)aminosulphonyl group.
In the di(C₁-C₆-alkyl)aminosulphonyl groups for the radical R3, each of the two C₁-C₆-alkyl radicals on the nitrogen atom of the di(C₁-C₆-alkyl)aminosulphonyl group may be independently of one another for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
The (C₃-C₇-cycloalkyl)aminosulphonyl groups for the radical R3 may be for example a cyclopropylaminosulphonyl, cyclobutylaminosulphonyl, cyclopentylaminosulphonyl, cyclohexylaminosulphonyl or cycloheptylaminosulphonyl group.
In the di(C₃-C₇-cycloalkyl)aminosulphonyl groups for the radical R3, each of the two C₃-C₇-cycloalkyl radicals on the nitrogen atom of the di(C₃-C₇-cycloalkyl)aminosulphonyl group may be independently of one another for example a cyclopropyl, cyclobutyl, cyclopentyl, cyclohexyl or cycloheptyl group.
In the C₃-C₇-cycloalkyl-C₁-C₆-alkyleneaminosulphonyl groups of the radical R3, each of the C₃-C₇-cycloalkyl groups of the C₃-C₇-cycloalkyl-C₁-C₆-alkyleneaminosulphonyl groups, for example of a cyclopropyl, cyclobutyl, cyclopentyl, cyclohexyl or cycloheptyl group, can be combined independently of one another with each C₁-C₆-alkyleneaminosulphonyl group, for example with a methyleneaminosulphonyl, ethyleneaminosulphonyl, propyleneaminosulphonyl, butyleneaminosulphonyl, pentyleneaminosulphonyl, hexyleneaminosulphonyl group.
The C₁-C₆-alkylsulphonylamido groups for the radical R3 may be for example a methylsulphonylamido, ethylsulphonylamido, propylsulphonylamido, isopropylsulphonylamido, butylsulphonylamido, isobutylsulphonylamido, sec-butylsulphonylamido, tert-butylsulphonylamido, pentylsulphonylamido, isopentylsulphonylamido, (2-methylbutyl)-sulphonylamido, (1-methylbutyl)sulphonylamido, (1-ethylpropyl)sulphonylamido, neo-pentylsulphonylamido, (1,1-dimethylpropyl)sulphonylamido, hexylsulphonylamido, (4-methylpentyl)sulphonylamido, (3-methylpentyl)sulphonylamido, (2-methylpentyl)-sulphonylamido, (1-methylpentyl)sulphonylamido, (1-ethylbutyl)sulphonylamido, (2-ethylbutyl)sulphonylamido, (3,3-dimethylbutyl)sulphonylamido, (2,2-dimethylbutyl)-sulphonylamido, (1,1-dimethylbutyl)sulphonylamido, (2,3-dimethylbutyl)sulphonylamido, (1,3-dimethylbutyl)sulphonylamido or a (1,2-dimethylbutyl)sulphonylamido group.
In the -N(C₀-C₆-alkyl)-C(O)-C₁-C₆-alkyl groups of the radicals R4 to R6, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₁-C₆-alkyl group on the carbonyl group of the amide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -N-(C₀-C₆-alkyl)-C(O)-C₃-C₇-cycloalkyl groups of the radical R3, each of the (C₀-C₆-alkyl) groups on the nitrogen atom of the -N(C₀-C₆-alkyl)-C(O)-C₁-C₆-alkyl groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the carbonyl group of the amide, for example with a cyclopropyl, cyclobutyl, cyclopentyl, cyclohexyl or cycloheptyl group.
In the -N(C₀-C₆-alkyl)-C(O)-N-di(C₀-C₆-alkyl) groups of the radical R3, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -N(C₀-C₆-alkyl)-C(O)-O-(C₀-C₆-alkyl) groups of the radical R3, both (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -N(C₀-C₆-alkyl)-C(O)-NH-(C₃-C₇-cycloalkyl) groups of the radical R3, each of the (C₀-C₆-alkyl) groups on the nitrogen atom of the -N(C₀-C₆-alkyl)-C(O)-NH-(C₃-C₇-cycloalkyl) groups, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₃-C₇-cycloalkyl group on the terminal nitrogen atom of the urea, for example with a cyclopropyl, cyclobutyl, cyclopentyl, cyclohexyl or cycloheptyl group.
In the -N(C₀-C₆-alkyl)-SO₂-(C₁-C₆-alkyl) groups of the radical R3, each of the (C₀-C₆-alkyl) groups on the nitrogen atom of the -N(C₀-C₆-alkyl)-SO₂-(C₁-C₆-alkyl) group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may independently of one another be combined with each C₁-C₆-alkyl group on the sulphonyl group of the sulphonamide, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -N(C₀-C₆-alkyl)-SO₂-C₃-C₇-cycloalkyl groups of the radical R3, each of the (C₀-C₆-alkyl) groups on the nitrogen atom of the -N(C₀-C₆-alkyl)-SO₂-C₃-C₇-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group on the sulphonyl group, for example with a cyclopropyl, cyclobutyl, cyclopentyl, cyclohexyl or cycloheptyl group.
In the -N(C₀-C₆-alkyl)-SO₂-N-di(C₀-C₆-alkyl) groups of the radical R3, all three (C₀-C₆-alkyl) groups may be independently of one another a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -N(C₀-C₆-alkyl)-SO₂-NH-(C₃-C₇)-cycloalkyl groups of the radical R3, the C₀-C₆-alkyl group of the -N(C₀-C₆-alkyl)-SO₂-NH-(C₃-C₇)-cycloalkyl group, for example a hydrogen, a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group, may be combined independently of one another with each C₃-C₇-cycloalkyl group, for example with a cyclopropyl, cyclobutyl, cyclopentyl, cyclohexyl or cycloheptyl group.
In the -C(O)-N(H)-C₂-C₆-alkylene-(C₁-C₆-alkyl)amine groups of the radical R3, each of the C₂-C₆-alkylene groups on the nitrogen atom of the -C(O)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -C(O)-N(H)-C₂-C₆-alkylene-[di(C₁-C₆-alkyl)]amine groups of the radical R3, each of the C₂-C₆-alkylene groups on the nitrogen atom of the -C(O)-N(H)-C₂-C₆-alkylene-[di(C₁-C₆-alkyl)]amine group, for example an ethylene, propylene, butylene, pentylene or hexylene group, may be combined independently of one another with each of the two identically or different C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -C(O)-N(H)-C₂-C₆-alkylene-(C₃-C₇-cycloalkyl)amine groups of the radical R3, each of the (C₂-C₆-alkylene) groups of the -C(O)-N(H)-C₂-C₆-alkylene-(C₃-C₇-cycloalkyl)amine group, for example an ethylene, propylene, butylene, pentylene or hexylene group, may be combined independently of one another with each C₃-C₇-cycloalkyl group on the amine, for example with a cyclopropyl, cyclobutyl, cyclopentyl, cyclohexyl or cycloheptyl group.
In the -C(O)-N(H)-C₂-C₆-alkylene-(C₃-C₇-cycloalkyl-C₁-C₆-alkylene)amine groups of the radical R3, each of the (C₂-C₆-alkylene) groups of the -C(O)-N(H)-C2-C6-alkylene-(C3-C6-cycloalkyl-C1-C6-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylene or cycloheptylhexylene group.

In the -S(O₂)-N(H)-C₂-C₆-alkylene-(C₁-C₆-alkyl)amine groups of the radical R3, the (C₂-C₆-alkylene) groups of the -S(O₂)-N(H)-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -S(O₂)-N(H)-C₂-C₆-alkylene-[di(C₁-C₆-alkyl)]amine groups of the radical R3, the C₂-C₆-alkylene group of the -S(O₂)-N(H)-C₂-C₆-alkylene-[di(C₁-C₆-alkyl)]amine group, for example an ethylene, propylene, butylene, pentylene or hexylene group, may be combined independently of one another with each of the two C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

In the -S(O₂)-N(H)-C₂-C₆-alkylene-(C₃-C₇-cycloalkyl)amine groups of the radical R3, the C₂-C₆-alkylene group of the -S(O₂)-N(H)-C₂-C₆-alkylene-(C₃-C₇-cycloalkyl)amine group, for example an ethylene, propylene, butylene, pentylene or hexylene group, may be combined independently of one another with each C₃-C₇-cycloalkyl group on the amino group, for example with a cyclopropyl, cyclobutyl, cyclopentyl, cyclohexyl or cycloheptyl group.
In the -S(O₂)-N(H)-C₂-C₆-alkylene-(C₃-C₇-cycloalkyl-C₁-C₆-alkylene)amine groups of the radical R3, each C₂-C₆-alkylene group of the -S(O₂)-N(H)-C₂-C₆-alkylene-(C₃-C₇-cycloalkyl-C₁-C₆-alkylene)amine group, for example an ethylene, propylene, butylene, pentylene or hexylene group, may be combined independently of one another with each C₃-C₇-cycloalkyl-C₁-C₆-alkylene group on the amine, for example with a cyclopropylmethylene, cyclopropylethylene, cyclopropylpropylene, cyclopropylbutylene, cyclopropylpentylene, cyclopropylhexylene, cyclobutylmethylene, cyclobutylethylene, cyclobutylpropylene, cyclobutylbutylene, cyclobutylpentylene, cyclobutylhexylene, cyclopentylmethylene, cyclopentylethylene, cyclopentylpropylene, cyclopentylhexylene, cyclohexylmethylene, cyclohexylethylene, cyclohexylpropylene, cyclohexylbutylene, cyclohexylpentylene, cyclohexylhexylene, cycloheptylmethylene, cycloheptylethylene, cycloheptylpropylene, cycloheptylbutylene, cycloheptylpentylen or cycloheptylhexylene group.
In the -O-C₂-C₆-alkylene-(C₁-C₆-alkyl)amine groups of the radical R3, the C₂-C₆-alkylene group of the -O-C₂-C₆-alkylene-(C₁-C₆-alkyl)amine group, for example an ethylene, propylene, butylene, pentylene or hexylene group, may be combined independently of one another with each C₁-C₆-alkyl group on the amino group, for example a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.
In the -O-C₂-C₆-alkylene-[di(C₁-C₆-alkyl)]amine groups of the radical R3, the C₂-C₆-alkylene group of the -O-C₂-C₆-alkylene-[di(C₁-C₆-alkyl)]amine group, for example an ethylene, propylene, butylene, pentylene or hexylene group, may be combined independently of one another with two freely selectable C₁-C₆-alkyl groups on the amino group, for example with a methyl, ethyl, propyl, isopropyl, butyl, isobutyl, sec-butyl, tert-butyl, pentyl, isopentyl, (2-methylbutyl), (1-methylbutyl), (1-ethylpropyl), neopentyl, (1,1-dimethylpropyl), hexyl-, (4-methylpentyl), (3-methylpentyl), (2-methylpentyl), (1-methylpentyl), (1-ethylbutyl), (2-ethylbutyl), (3,3-dimethylbutyl), (2,2-dimethylbutyl), (1,1-dimethylbutyl), (2,3-dimethylbutyl), (1,3-dimethylbutyl) or a (1,2-dimethylbutyl) group.

Compounds preferred according to the present invention are those of the formulae II and III 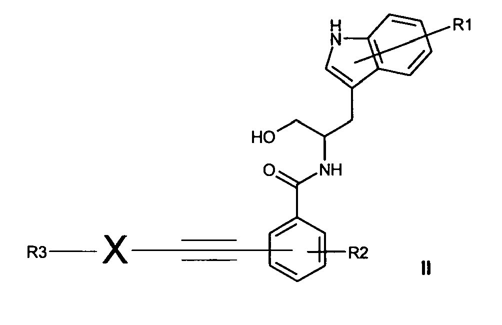 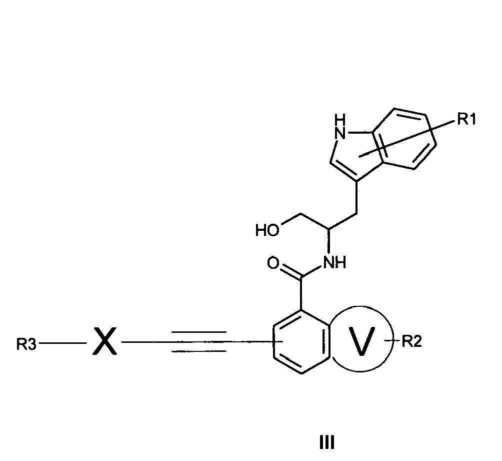 in which the radicals R1 to R3, X and V have the same meaning as defined in formula I.

Compounds particularly preferred according to the present invention are those of the formula IV and V 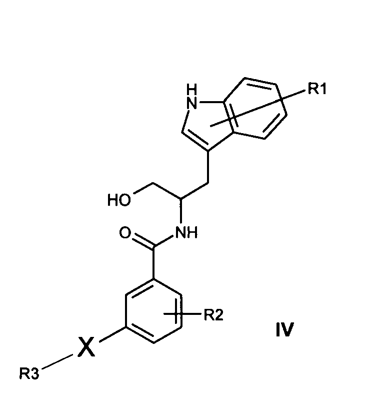 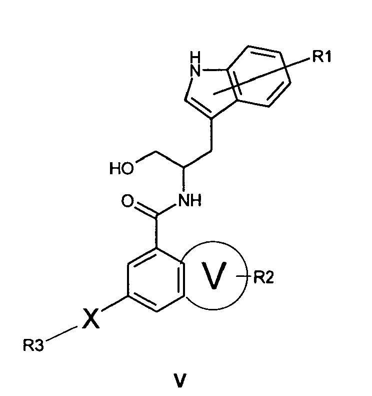 in which the radicals R1 to R3, X and V have the same meaning as defined in formula I.

The following compounds are very particularly preferred:
**1** 5-(3-Cyclopentyl-prop-1-ynyl)-N-[(R)-1-hydroxymethyl-2-(1H-indol-3-yl)-ethyl]-2-isopropoxy-benzamide;
**2** 5-Cyclopentylethynyl-N-[(R)-1-hydroxymethyl-2-(1H-indol-3-yl)-ethyl]-2-isopropoxy-benzamide;
**3** 5-(3,3-Dimethyl-but-1-ynyl)-N-[(R)-1-hydroxymethyl-2-(1H-indol-3-yl)-ethyl]-2-isopropoxy-benzamide;
**4** 5-(4-Hydroxy-but-1-ynyl)-N-[(R)-1-hydroxymethyl-2-(1H-indol-3-yl)-ethyl]-2-isopropoxy-benzamide;
**5** 5-[3-(1,1-Dioxo-1lambda*6*-thiomorpholin-4-yl)-prop-1-ynyl]-N-[(R)-2-hydroxy-1-(1H-indol-3-ylmethyl)-ethyl]-2-isopropoxy-benzamide;
**6** 6-{3-[(R)-1-Hydroxymethyl-2-(1H-indol-3-yl)-ethylcarbamoyl]-4-isopropoxy-phenyl}-hex-5-ynoic acid methyl ester;
**7** N-[(R)-1-Hydroxymethyl-2-(1H-indol-3-yl)-ethyl]-2-isopropoxy-5-[3-(3-p-tolyl-ureido)-prop-1-ynyl]-benzamide;
**8** N-[(R)-1-Hydroxymethyl-2-(1H-indol-3-yl)-ethyl]-5-(5-hydroxy-pent-1-ynyl)-2-isopropoxy-benzamide;
**9** 5-(5-Cyano-pent-1-ynyl)-N-[(R)-1-hydroxymethyl-2-(1H-indol-3-yl)-ethyl]-2-isopropoxy-benzamide;
**10** 5-[3-(1,3-Dimethyl-ureido)-prop-1-ynyl]-N-[(R)-1-hydroxymethyl-2-(1H-indol-3-yl)-ethyl]-2-isopropoxy-benzamide;
**11** N-[2-(5-Fluoro-1H-indol-3-yl)-1-hydroxymethyl-ethyl]-5-(3-hydroxy-prop-1-ynyl)-2-propoxy-benzamide;
**12** N-[2-(5-Fluoro-1 H-indol-3-yl)-1-hydroxymethyl-ethyl]-5-(4-hydroxy-pent-1-ynyl)-2-propoxy-benzamide;
**13** 5-(3-Dimethylamino-prop-1-ynyl)-N-[2-(5-fluoro-1H-indol-3-yl)-1-hydroxymethyl-ethyl]-2-propoxy-benzamide;
**14** 5-(5-Cyano-pent-1-ynyl)-N-[2-(5-fluoro-1H-indol-3-yl)-1-hydroxymethyl-ethyl]-2-propoxy-benzamide;
**15** N-[2-(5-Fluoro-1H-indol-3-yl)-1-hydroxymethyl-ethyl]-5-(5-hydroxy-pent-1-ynyl)-2-propoxy-benzamide;
**16** N-[2-(5-Fluoro-1H-indol-3-yl)-1-hydroxymethyl-ethyl]-5-(4-hydroxy-but-1-ynyl)-2-propoxy-benzamide;
**17** N-[2-(5-Fluoro-1H-indol-3-yl)-1-hydroxymethyl-ethyl]-5-{3-[((1R,2R)-2-hydroxy-1-methyl-propyl)-methyl-amino]-prop-1-ynyl}-2-propoxy-benzamide;
**18** 5-[4-(1,3-Dimethyl-ureido)-but-1-ynyl]-N-[2-(5-fluoro-1H-indol-3-yl)-1-hydroxymethyl-ethyl]-2-propoxy-benzamide;
**19** N-[2-(5-Fluoro-1H-indol-3-yl)-1-hydroxymethyl-ethyl]-5-(4-methylcarbamoyl-but-1-ynyl)-2-propoxy-benzamide;
**20** N-[2-(5-Fluoro-1 H-indol-3-yl)-1-hydroxymethyl-ethyl]-5-(5-methylcarbamoyl-pent-1-ynyl)-2-propoxy-benzamide;
**21** N-[(R)-1-Hydroxymethyl-2-(1H-indol-3-yl)-ethyl]-5-(3-hydroxy-prop-1-ynyl)-2-isopropoxy-benzamide;
**22** N-[(R)-1-Hydroxymethyl-2-(1H-indol-3-yl)-ethyl]-5-(4-hydroxy-pent-1-ynyl)-2-isopropoxy-benzamide;
**23** 5-[3-(Benzyl-methyl-amino)-prop-1-ynyl]-N-[(R)-1-hydroxymethyl-2-(1H-indol-3-yl)-ethyl]-2-isopropoxy-benzamide;
**24** 5-(3-Dimethylamino-prop-1-ynyl)-N-[(R)-1-hydroxymethyl-2-(1H-indol-3-yl)-ethyl]-2-isopropoxy-benzamide;
**25** 5-[3-(1,3-Dimethyl-ureido)-prop-1-ynyl]-N-[(R)-1-hydroxymethyl-2-(1H-indol-3-yl)-ethyl]-2-isopropoxy-benzamide;
**26** 6-{3-[(R)-1-Hydroxymethyl-2-(1H-indol-3-yl)-ethylcarbamoyl]-4-isopropoxy-phenyl}-hex-5-ynoic acid methyl ester;
**27** 5-(6-Hydroxy-hex-1-ynyl)-N-[(R)-1-hydroxymethyl-2-(1H-indol-3-yl)-ethyl]-2-isopropoxy-benzamide;
**28** 5-(5-Cyano-pent-1-ynyl)-N-[(R)-1-hydroxymethyl-2-(1H-indol-3-yl)-ethyl]-2-isopropoxy-benzamide;
**29** N-[(R)-1-Hydroxymethyl-2-(1H-indol-3-yl)-ethyl]-5-(5-hydroxy-pent-1-ynyl)-2-isopropoxy-benzamide;
**30** 5-(4-Hydroxy-but-1-ynyl)-N-[(R)-1-hydroxymethyl-2-(1H-indol-3-yl)-ethyl]-2-isopropoxy-benzamide;
**31** Acetic acid 3-{3-[(R)-1-hydroxymethyl-2-(1H-indol-3-yl)-ethylcarbamoyl]-4-isopropoxyphenyl}-prop-2-ynyl ester;
**32** N-[(R)-1-Hydroxymethyl-2-(1H-indol-3-yl)-ethyl]-5-{3-[((1R,2R)-2-hydroxy-1-methylpropyl)-methyl-amino]-prop-1-ynyl}-2-isopropoxy-benzamide;
**33** 9-{3-[(R)-1-Hydroxymethyl-2-(1H-indol-3-yl)-ethylcarbamoyl]-4-isopropoxy-phenyl}-non-8-ynoic acid;
**34** 5-[4-(1,3-Dimethyl-ureido)-but-1-ynyl]-N-[(R)-1-hydroxymethyl-2-(1H-indol-3-yl)-ethyl]-2-isopropoxy-benzamide;
**35** N-[(R)-1-Hydroxymethyl-2-(1H-indol-3-yl)-ethyl]-2-isopropoxy-5-(4-methylcarbamoyl-but-1-ynyl)-benzamide;
**36** N-[(R)-1-Hydroxymethyl-2-(1H-indol-3-yl)-ethyl]-2-isopropoxy-5-(5-methylcarbamoyl-pent-1-ynyl)-benzamide;
**37** N-[(R)-1-Hydroxymethyl-2-(1H-indol-3-yl)-ethyl]-3-(3-hydroxy-prop-1-ynyl)-benzamide;
**38** N-[(R)-1-Hydroxymethyl-2-(1H-indol-3-yl)-ethyl]-3-(3-hydroxy-prop-1-ynyl)-benzamide;
**39** 3-[3-(Benzyl-methyl-amino)-prop-1-ynyl]-N-[(R)-1-hydroxymethyl-2-(1H-indol-3-yl)-ethyl]-benzamide;
**40** 3-(3-Dimethylamino-prop-1-ynyl)-N-[(R)-1-hydroxymethyl-2-(1H-indol-3-yl)-ethyl]-benzamide;
**41** 3-[3-(1,3-Dimethyl-ureido)-prop-1-ynyl]-N-[(R)-1-hydroxymethyl-2-(1H-indol-3-yl)-ethyl]-benzamide;
**42** 6-{3-[(R)-1-Hydroxymethyl-2-(1H-indol-3-yl)-ethylcarbamoyl]-phenyl}-hex-5-ynoic acid;
**43** 6-{3-[(R)-1-Hydroxymethyl-2-(1H-indol-3-yl)-ethylcarbamoyl]-phenyl}-hex-5-ynoic acid; methyl ester;
**44** 3-(6-Hydroxy-hex-1-ynyl)-N-[(R)-1-hydroxymethyl-2-(1H-indol-3-yl)-ethyl]-benzamide;
**45** 3-(5-Cyclohexyl-pent-1-ynyl)-N-[(R)-1-hydroxymethyl-2-(1H-indol-3-yl)-ethyl]-benzamide;
**46** 3-(5-Cyano-pent-1-ynyl)-N-[(R)-l-hydroxymethyl-2-(1H-indol-3-yl)-ethyl]-benzamide;
**47** N-[(R)-1-Hydroxymethyl-2-(1H-indol-3-yl)-ethyl]-3-(5-hydroxy-pent-1-ynyl)-benzamide;
**48** 3-(4-Hydroxy-but-1-ynyl)-N-[(R)-1-hydroxymethyl-2-(1H-indol-3-yl)-ethyl]-benzamide;
**49** N-[(R)-1-Hydroxymethyl-2-(1H-indol-3-yl)-ethyl]-3-{3-[((1R,2R)-2-hydroxy-1-methylpropyl)-methyl-amino]-prop-1-ynyl}-benzamide;
**50** N-[(R)-1-Hydroxymethyl-2-(1H-indol-3-yl)-ethyl]-3-[3-(3-methyl-ureido)-prop-1-ynyl]-benzamide;
**51** 9-{3-[(R)-1-Hydroxymethyl-2-(1H-indol-3-yl)-ethylcarbamoyl]-phenyl}-non-8-ynoic acid;
**52** 3-[4-(1,3-Dimethyl-ureido)-but-1-ynyl]-N-[(R)-1-hydroxymethyl-2-(1H-indol-3-yl)-ethyl]-benzamide;
**53** N-[(R)-1-Hydroxymethyl-2-(1H-indol-3-yl)-ethyl]-3-[4-(3-methyl-ureido)-but-1-ynyl]-benzamide;
**54** N-[(R)-1-Hydroxymethyl-2-(1H-indol-3-yl)-ethyl]-3-(4-methylcarbamoyl-but-1-ynyl)-benzamide;
**55** N-[(R)-1-Hydroxymethyl-2-(1H-indol-3-yl)-ethyl]-3-(5-methylcarbamoyl-pent-1-ynyl)-benzamide;
**56** 5-[3-(Benzyl-methyl-amino)-prop-1-ynyl]-2-bromo-N-[(R)-1-hydroxymethyl-2-(1H-indol-3-yl)-ethyl]-benzamide;
**57** 2-Bromo-5-(3-dimethylamino-prop-1-ynyl)-N-[(R)-1-hydroxymethyl-2-(1H-indol-3-yl)-ethyl]-benzamide;
**58** 2-Bromo-5-[3-(1,3-dimethyl-ureido)-prop-1-ynyl]-N-[(R)-2-hydroxy-1-(1H-indol-3-ylmethyl)-ethyl]-benzamide;
**59** 2-Bromo-5-(5-cyano-pent-1-ynyl)-N-[(R)-1-hydroxymethyl-2-(1H-indol-3-yl)-ethyl]-benzamide;
**60** 2-Bromo-N-[(R)-1-hydroxymethyl-2-(1H-indol-3-yl)-ethyl]-5-(5-methylcarbamoyl-pent-1-ynyl)-benzamide;
**61** 7-(3-Hydroxy-prop-1-ynyl)-2,3,4,5-tetrahydro-benzo[b]oxepine-9-carboxylic acid [(R)-1-hydroxymethyl-2-(1H-indol-3-yl)-ethyl]-amide;
**62** 7-(4-Hydroxy-pent-1-ynyl)-2,3,4,5-tetrahydro-benzo[b]oxepine-9-carboxylic acid [(R)-1-hydroxymethyl-2-(1H-indol-3-yl)-ethyl]-amide;
**63** 7-[3-(Benzyl-methyl-amino)-prop-1-ynyl]-2,3,4,5-tetrahydro-benzo[b]oxepine-9-carboxylic acid [(R)-1-hydroxymethy)-2-(1H-indol-3-yl)-ethyl]-amide;
**64** 7-(3-Dimethylamino-prop-1-ynyl)-2,3,4,5-tetrahydro-benzo[b]oxepine-9-carboxylic acid [(R)-1-hydroxymethyl-2-(1H-indol-3-yl)-ethyl]-amide;
**65** 7-[3-(1,3-Dimethyl-ureido)-prop-1-ynyl]-2,3,4,5-tetrahydro-benzo[b]oxepine-9-carboxylic acid [(R)-1-hydroxymethyl-2-(1H-indol-3-yl)-ethyl]-amide;
**66** 7-(4-Amino-but-1-ynyl)-2,3,4,5-tetrahydro-benzo[b]oxepine-9-carboxylic acid [(R)-1-hydroxymethyl-2-(1H-indol-3-yl)-ethyl]-amide;
**67** 6-{9-[(R)-1-Hydroxymethyl-2-(1H-indol-3-yl)-ethylcarbamoyl]-2,3,4,5-tetrahydrobenzo[b]oxepin-7-yl}-hex-5-ynoic acid;
**68** 6-{9-[(R)-1-Hydroxymethyl-2-(1H-indol-3-yl)-ethylcarbamoyl]-2,3,4,5-tetrahydrobenzo[b]oxepin-7-yl}-hex-5-ynoic acid methyl ester;
**69** 7-(6-Hydroxy-hex-1-ynyl)-2,3,4,5-tetrahydro-benzo[b]oxepine-9-carboxylic acid [(R)-1-hydroxymethyl-2-(1H-indol-3-yl)-ethyl]-amide;
**70** 7-(5-Cyclohexyl-pent-1-ynyl)-2,3,4,5-tetrahydro-benzo[b]oxepine-9-carboxylic acid [(R)-1-hydroxymethyl-2-(1H-indol-3-yl)-ethyl]-amide;
**71** 7-(5-Cyano-pent-1-ynyl)-2,3,4,5-tetrahydro-benzo[b]oxepine-9-carboxylic acid [(R)-1-hydroxymethyl-2-(1H-indo)-3-yl]-ethyl]-amide;
**72** 7-(4-Hydroxy-but-1-ynyl)-2,3,4,5-tetrahydro-benzo[b]oxepine-9-carboxylic acid [(R)-1-hydroxymethyl-2-(1H-indol-3-yl)-ethyl]-amide;
**73** 7-{3-[((1R,2R)-2-Hydroxy-1-methyl-propyl)-methyl-amino]-prop-1-ynyl}-2,3,4,5-tetrahydro-benzo[b]oxepine-9-carboxylic acid [(R)-1-hydroxymethyl-2-(1H-indol-3-yl)-ethyl]-amide;
**74** 7-[3-(3-Methyl-ureido)-prop-1-ynyl]-2,3,4,5-tetrahydro-benzo[b]oxepine-9-carboxylic acid [(R)-1-hydroxymethyl-2-(1H-indol-3-yl)-ethyl]-amide;
**75** 7-[4-(3-Methyl-ureido)-but-1-ynyl]-2,3,4,5-tetrahydro-benzo[b]oxepine-9-carboxylic acid [(R)-1-hydroxymethyl-2-(1H-indol-3-yl)-ethyl]-amide;
**76** 7-(4-Methylcarbamoyl-but-1-ynyl)-2,3,4,5-tetrahydro-benzo[b]oxepine-9-carboxylic acid [(R)-1-hydroxymethyl-2-(1H-indol-3-yl)-ethyl]-amide;
**77** 7-(5-Methylcarbamoyl-pent-1-ynyl)-2,3,4,5-tetrahydro-benzo[b]oxepine-9-carboxylic acid [(R)-1-hydroxymethyl-2-(1H-indol-3-yl)-ethyl]-amide.

### Pharmacological investigations

### HTRF assay for measuring cAMP in cells

The method is based on a competitive immunoassay between native cAMP, which has been produced by the cells, and cAMP which is labelled with XL665. The tracer binding was visualized by a monoclonal antibody, anti-cAMP labelled with cryptate [HTRF = homogeneous time-resolved fluorescence].
The specific signal is inversely proportional to the cAMP concentration of the samples employed.
The 665nm/ 620nm fluorescence ratio was evaluated.

*The following material was used: 96-well plates for the tissue culture, 96-well plates with black edge and black base (e.g. Fluotrac 600 from Greiner), 96-well plates for the substance dilutions of polypropylene and cAMP Femtomolar (4000wells Kit, CIS Bio International # 62AM1PEC).*
The following reagents were used: BSA (bovine serum albumin) Fraction V protease-free, IBMX (3-isobutyl-1-methylxanthine), hFSH (human follicle stimulating hormone), Triton X-100 analytical grade, potassium fluoride analytical grade, G 418 (Geneticin) and Accutase.
Buffer 1 (washing and testing buffer) contained PBS, 1 mM CaCl2, 1 mM MgCl₂, 0.2% glucose; 0.1% BSA, 1 mM IBMX.
Buffer 2 (2x lysis buffer) contained 1% Triton X-100 in PBS (without CaCl₂ and MgCl₂).
Buffer 3 (assay buffer) contained 50 mM potassium phosphate buffer (pH 7.0); 800 mM potassium fluoride; 0.2% BSA (always added fresh).

### Procedure:

On day 1, the cells were seeded in 96-well plates (3x10⁴ cells per well hFSHR clone 16 cells (CHO cells stably transfected with the human FSH receptor in 150 µl of medium). The next day, test substance dilutions were made up. For this purpose, all the substances were diluted in ice-cold buffer 1 (with or without hFSH), and the substance dilutions were placed on ice until applied to the cells.
The cell supernatant was then aspirated off, and the cells were washed 2x with 200 µl of buffer 1. The cells were treated with 60 µl of the appropriate substance concentrations at 37°C for 2h. The cells were then lysed with 60 µl of buffer 2 (put onto the supernatant) (on a plate shaker at RT for 30 min).
The test conjugates (XL-665 and anti-cAMP cryptate) were diluted in buffer 3 in accordance with the manufacturers' information. The actual mixture for measurement was pipetted into a black 96-well plate (in each case 15 µl of the cell lysate diluted with 35 µl of buffer 1; firstly 25 µl of XL-665 conjugate were pipetted and, after 10 min, 25 µl of the anti-cAMP cryptate were added). This is followed by incubation at RT for 90 minutes. The measurement was carried out in a PheraStar (BMG).

**Tissue culture conditions**
- 1) hFSHr clone 16: Ham's F12
PSG
10% FCS
700 µg/ml G 418 (Geneticin) from PAA.

Dose-effect curve (hFSH) for the human receptor: 1e-8, 3e-9, 1e-9, 3e-10, 1e-10, 3e-11, 1e-11, 3e-12 mol/l.
The test substances were employed in suitable dilutions in the absence (test for agonism) and in the presence of 1e-9 mol/l hFSH.

### Evaluation

The values of the well ratio were averaged and then entered directly in SigmaPlot versus the concentrations. The maximum and minimum values were determined for each plate, and half the difference is to be regarded as IC₅₀.
The test results (Table 1) show that the compounds according to the invention have an FSH-antagonistic effect.

**Table 1. FSH-antagonistic effect of selected compounds in the HTRF assay**

| **Compound [Ex. #]** | **IC₅₀** |
|---|---|
| **3** | **600 nM** |
| **5** | **4,5 µM** |
| **7** | **200 nM** |
| **8** | **500 nM** |
| **10** | **6 pM** |

Being antagonists of the FSH receptor, compounds of the general formula I or pharmaceutically acceptable salts thereof can thus be used for the manufacture of medicaments to be used for the fertility control in male and/or in a female animals, in particular in men and/or women; as well as for the treatment and/or prevention of osteoporosis.

### Dosage

Satisfactory results are generally to be expected if the daily doses comprise a range from 5 µg to 50 mg of the compound according to the invention per kg of body weight. A recommended daily dose for larger mammals, for example humans, is in the range from 10 µg to 30 mg per kg of body weight. Suitable dosages for the compounds according to the invention are from 0.005 to 50 mg per day per kg of body weight, depending on the age and constitution of the patient, it being possible to administer the necessary daily dose by single or multiple delivery.
Pharmaceutical products based on the novel compounds are formulated in a manner known per se by processing the active ingredient with the carrier substances, fillers, substances which influence disintegration, binders, humectants, lubricants, absorbents, diluents, test modifiers, colorants etc. which are used in pharmaceutical technology, and converting into the desired administration form. Reference should be made in this connection to Remington's Pharmaceutical Science, 15th ed. Mack Publishing Company, East Pennsylvania (1980).
Suitable for oral administration are in particular tablets, coated tablets, capsules, pills, powders, granules, pastilles, suspensions, emulsions or solutions. Preparations for injection and infusion are possible for parenteral administration. Appropriately prepared crystal suspensions can be used for intraarticular injection. Aqueous and oily solutions for injection or suspensions and corresponding depot preparations can be used for intramuscular injection. The novel compounds can be used for rectal administration in the form of suppositories, capsules, solutions (e.g. in the form of enemas) and ointments both for systemic and for local therapy. Formulations possible for topical application are gels, ointments, greasy ointments, creams, pastes, dusting powders, milk and tinctures. The dosage of the compounds of the general formula I in these preparations should be 0.01% - 20% in order to achieve an adequate pharmacological effect. Topical use can also take place by means of a transdermal system, for example a patch.

The invention likewise encompasses the compounds according to the invention of the general formula I as therapeutic active ingredient. The invention further includes the compounds according to the invention of the general formula I as therapeutic active ingredients together with pharmaceutically suitable and acceptable excipients and carriers. The invention likewise encompasses a pharmaceutical composition which comprises one of the pharmaceutically active compounds according to the invention or mixture thereof and a pharmaceutically suitable salt or pharmaceutically suitable excipients and carriers.

The present invention therefore also relates to pharmaceutical compositions which comprise at least one compound of the general formula I, where appropriate together with pharmaceutically suitable excipients and/or carriers.

Suitable for forming pharmaceutically suitable salts of the compounds according to the invention of the general formula I are, by methods known to the skilled person, as inorganic acids inter alia hydrochloric acid, hydrobromic acid, sulphuric acid and phosphoric acid, nitric acid, as carboxylic acids inter alia acetic acid, propionic acid, hexanoic acid, octanoic acid, decanoic acid, oleic acid, stearic acid, maleic acid, fumaric acid, succinic acid, benzoic acid, ascorbic acid, oxalic acid, salicylic acid, tartaric acid, citric acid, lactic acid, glycolic acid, malic acid, mandelic acid, cinnamic acid, glutamic acid, aspartic acid, and as sulphonic acids inter alia methanesulphonic acid, ethanesulphonic acid, toluenesulphonic acid, benzenesulphonic acid and naphthalenesulphonic acid.
These pharmaceutical compositions and medicaments may be intended for oral, rectal, subcutaneous, transdermal, percutaneous, intravenous or intramuscular administration. They comprise besides conventional carriers and/or diluents at least one compound of the general formula I.
The medicaments of the invention are produced using the customary solid or liquid carriers or diluents and the excipients customarily used in pharmaceutical technology, in accordance with the desired mode of administration with a suitable dosage in a known manner. The preferred preparations consist of a dosage form which is suitable for oral administration. Examples of such dosage forms are tablets, film-coated tablets, sugar-coated tablets, capsules, pills, powders, solutions or suspensions or else depot forms.

The pharmaceutical compositions which comprise at least one of the compounds according to the invention are preferably administered orally.
Parenteral preparations such as solutions for injection are also suitable. Preparations which may also be mentioned for example are suppositories.
Appropriate tablets can be obtained for example by mixing the active ingredient with known excipients, for example inert diluents such as dextrose, sugar, sorbitol, mannitol, polyvinylpyrrolidone, disintegrants such as maize starch or alginic acid, binders such as starch or gelatine, lubricants such as magnesium stearate or talc and/ or agents to achieve a depot effect such as carboxylpolymethylene, carboxylmethylcellulose, cellulose acetate phthalate or polyvinyl acetate. The tablets may also consist of a plurality of layers.
Correspondingly, coated tablets can be produced by coating cores which have been produced in analogy to the tablets with agents normally used in tablet coatings, for example polyvinylpyrrolidone or shellac, gum Arabic, talc, titanium oxide or sugar. The tablet coating may also consist of a plurality of layers, it being possible to use the excipients mentioned above for tablets.
Solutions or suspensions with the compounds according to the invention of the general formula I may additionally comprise taste-improving agents such as saccharin, cyclamate or sugar and, for example, flavourings such as vanillin or orange extract. They may additionally comprise suspending aids such as sodium carboxymethylcellulose or preservatives such as p-hydroxybenzoates.
Capsules comprising the compounds of the general formula I can be produced for example by the compound(s) of the general formula I being mixed with an inert carrier such as lactose or sorbitol and encapsulated in gelatine capsules.
Suitable suppositories can be produced for example by mixing with carriers intended for this purpose, such as neutral fats or polyethylene glycol or derivatives thereof.

The present invention also relates to a process for preparing the compounds according to the invention. Compounds of the general formula I can be prepared as shown in Scheme 1 by an amide-formation reaction between the tryptophanol derivative VI and the carboxylic acid VII. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyl-uronium hexafluorophosphate), HBTU (2-(1*H*-benzotriazol-1-yl)-1,1,3,3-tetramethyl-uronium hexafluorophosphate), EDC (*N*-[3-(dimethylamino)propyl]-*N*-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol VI to give the product of the general formula I.

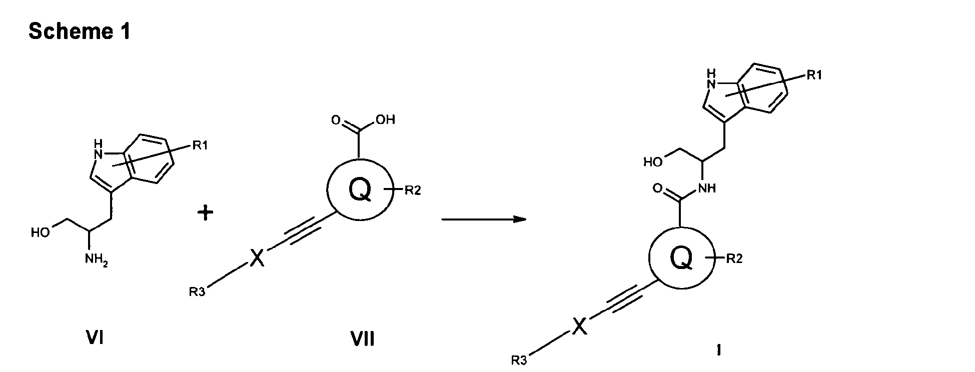

Compounds of general formulae II, III can be prepared as shown in Scheme 2 by an amide-formation reaction between the tryptophanol derivative VI and the appropriate carboxylic acid VIII or IX. Reagents suitable for this purpose are all known peptide-coupling reagents which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H-*benzotriazol-1-yl)-1,1,3,3-tetramethyluronium hexafluorophosphate), HBTU (2-(1*H-*benzotriazol-1-yl)-1,1,3,3-tetramethyluronium hexafluorophosphate), EDC (N-[3-(di-methylamino)propyl]-*N*'-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol VI to give the product of the general formula II or III.

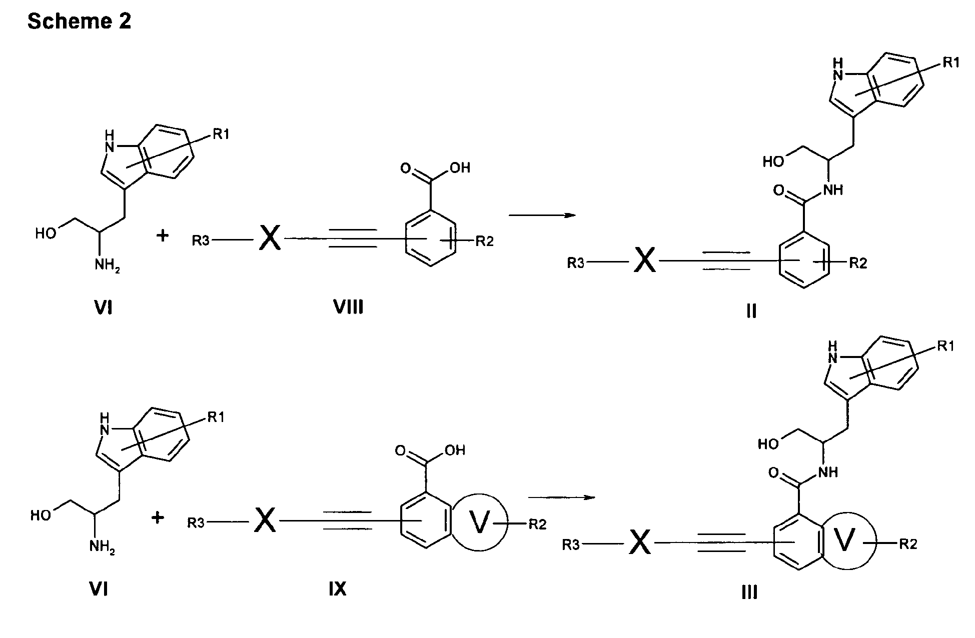

Compounds of general formulae IV and V can be prepared as shown in Scheme 3 by an amide-formation reaction between the tryptophanol derivative VI and the appropriate carboxylic acid X or XI. Reagents suitable for this purpose are all suitable peptide-coupling reagents which are known to the skilled person and which convert the carboxylic acid, where appropriate in the presence of a base, into an intermediate active ester, for example PyBOP ([(1*H*-benzotriazol-1-yl)oxy]tris(pyrrolidin-1-yl)phosphonium hexafluorophosphate), HATU (2-(7-aza-1*H*-benzotriazol-1-yl)-1,1,3,3-tetramethyl-uronium hexafluorophosphate), HBTU (2-(1*H*-benzotriazol-1-yl)-1,1,3,3-tetramethyl-uronium hexafluorophosphate), EDC (*N*-[3-(dimethylamino)propyl]-*N*'-ethylcarbodiimide hydrochloride) / HOBt (1-hydroxy-1*H*-benzotriazole). It is possible as alternative for the carboxylic acid to be converted, where appropriate in the presence of a base, into the carbonyl chloride and reacted with the tryptophanol VI to give the product of the general formula IV or V.

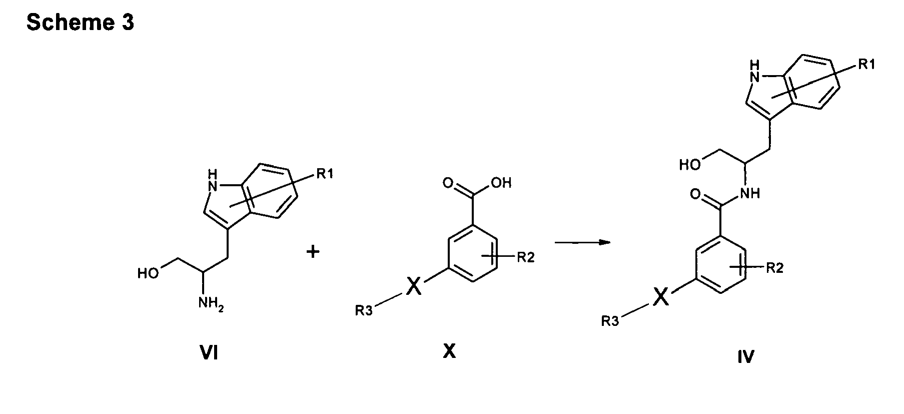

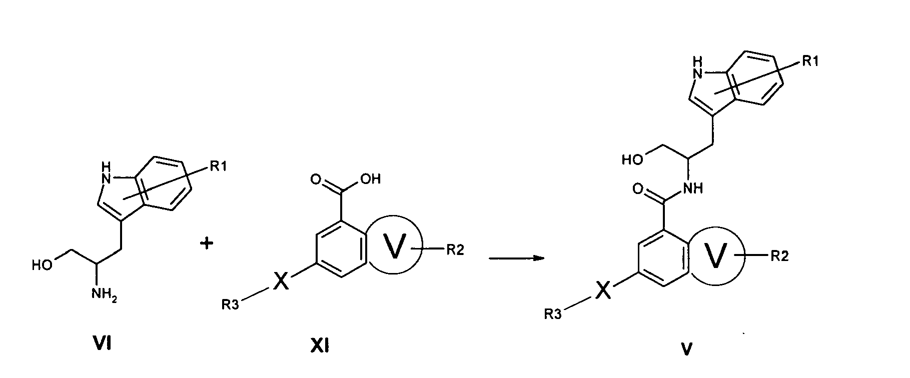

Likewise the present invention relates to a process for preparing the compounds according to the invention. Compounds of the general formula I can be prepared as shown in Scheme 4 by a Sonogashira type coupling reaction between an acetylene derivative XII and an aryl halide XIII. Reagents suitable for this purpose is a palladium catalysts in combination with or without a Cu (I) source , with or without a base in a suitable solvent which are known to the skilled person. Suitable palladium catalysts can be for example (Ph₃P)₂PdCl₂, Pd(PPh₃)₄, Pd(PhCN₂)Cl₂, Pd(OAc)₂, Pd(OAc)₂(PPh₃)₂, PdCl₂ or PhPdl(PPh₃)₂. Suitable bases can be for example amine bases such as triethylamine, diethylamine, diisopropylamine, Hünigs base, pyrrolidine, piperidine, butylamine or tetrabutylammoniumhydroxide, K₂CO₃ or Cs₂CO₃. As Cu (I) source typically Cul is used.

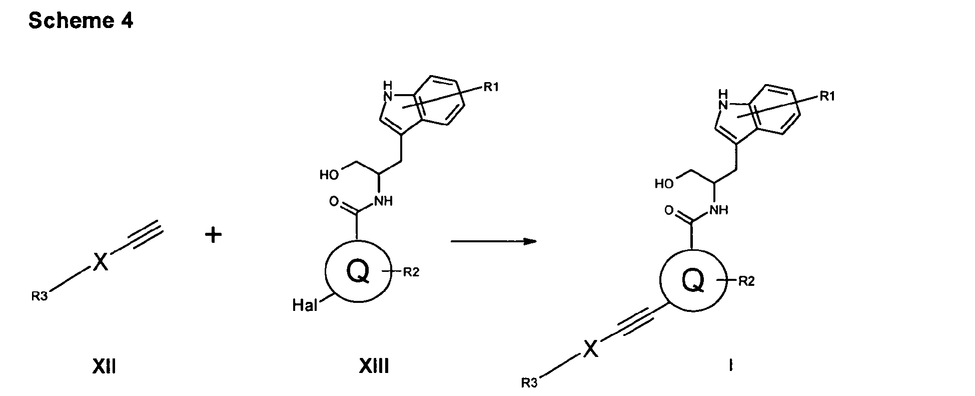

Compounds of the general formula II or III can be prepared as shown in Scheme 5 by a Sonogashira type coupling reaction between an acetylene derivative XII and an aryl halide XIV or XV respectively. Reagents suitable for this purpose is a palladium catalysts in combination with or without a Cu (I) source , with or without a base in a suitable solvent which are known to the skilled person. Suitable palladium catalysts can be for example (Ph₃P)₂PdCl₂, Pd(PPh₃)₄, Pd(PhCN₂)Cl₂, Pd(OAc)₂, Pd(OAc)₂(PPh₃)₂, PdCl₂ or PhPdl(PPh₃)₂. Suitable bases can be for example amine bases such as triethylamine, diethylamine, diisopropylamine, Hünigs base, pyrrolidine, piperidine, butylamine or tetrabutylammoniumhydroxide, K₂CO₃ or Cs₂CO₃. As Cu (I) source typically Cul is used.

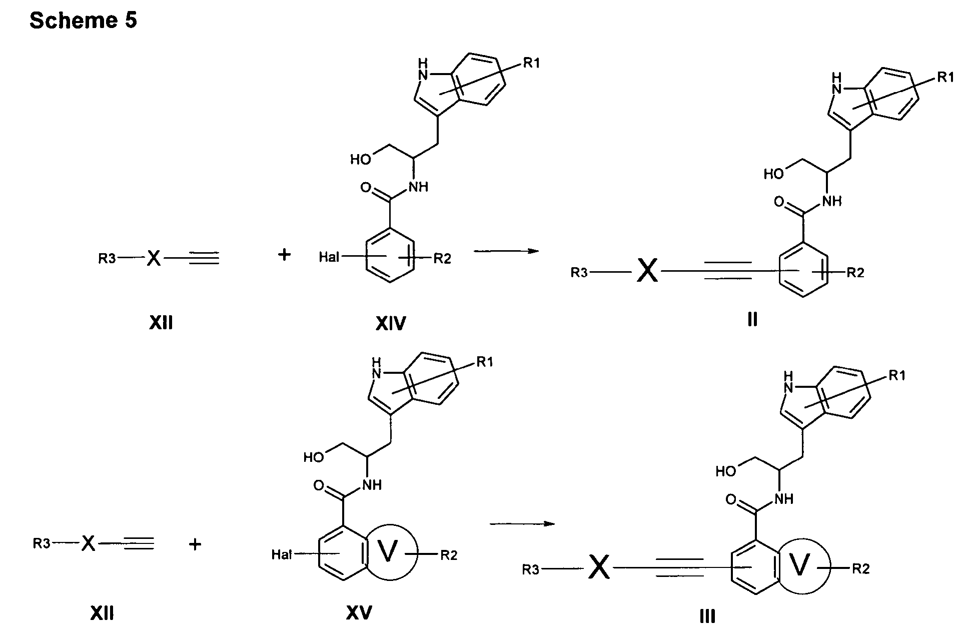

Compounds of the general formula IV or V can be prepared as shown in Scheme 6 by a Sonogashira type coupling reaction between an acetylene derivative XII and an aryl halide XVI or XVII respectively. Reagents suitable for this purpose is a palladium catalysts in combination with or without a Cu (I) source , with or without a base in a suitable solvent which are known to the skilled person. Suitable palladium catalysts can be for example (Ph₃P)₂PdCl₂, Pd(PPh₃)₄, Pd(PhCN₂)Cl₂, Pd(OAc)₂, Pd(OAc)₂(PPh₃)₂, PdCl₂ or PhPdl(PPh₃)₂. Suitable bases can be for example amine bases such as triethylamine, diethylamine, diisopropylamine, Hünigs base, pyrrolidine, piperidine, butylamine or tetrabutylammoniumhydroxide, K₂CO₃ or Cs₂CO₃. As Cu (I) source typically Cul is used.

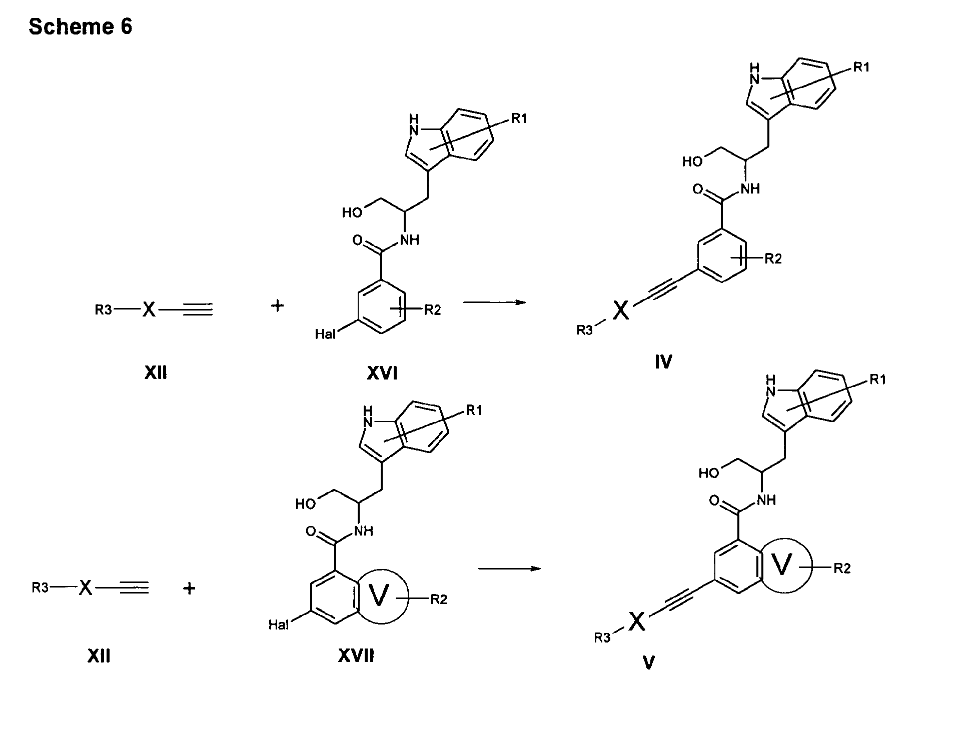

Compounds of the general formula I can in principle be prepared as shown in Scheme 7 by an amide-formation reaction between a tryptophanol derivative VI and a carboxylic acid VII. The reagents typically used for the coupling are EDC and HOBt. 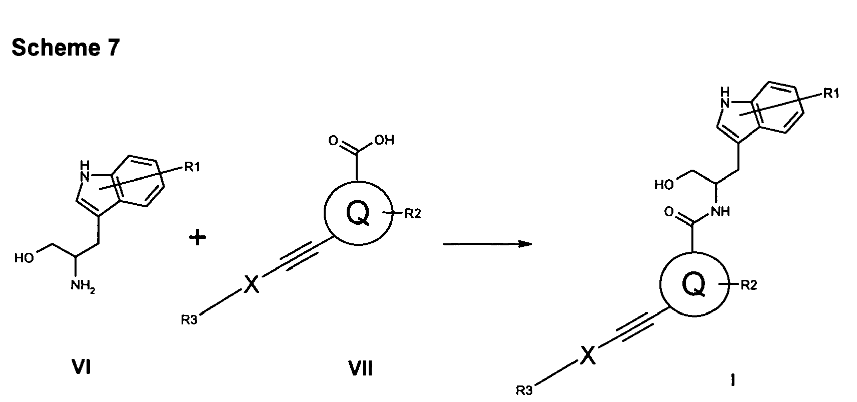 **Reagents:** a) EDCI, HOBt, DMF, Et₃N, DMF.

The tryptophanol derivatives of the formula VI can in principle be prepared as shown in Scheme 8 from the corresponding amino acids which can be purchased or are known from the literature. 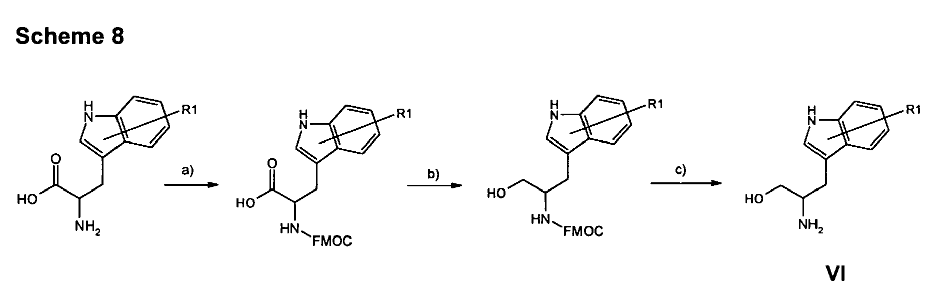 **Reagents:** a) FMOC-Cl, dioxane, 10% Na₂CO₃ solution in water, 0°C-RT; b) i) EtOC(O)Cl, THF, NMM, -10°C; ii) NaBH₄, MeOH, 0°C; c) piperidine, NaOH, RT.

The carboxylic acid derivatives of formula VII can in principle be prepared according to Scheme 9 via a Sonogashira type coupling of acetylenes with their corresponding aryl halides with subsequent hydrolysis of the resulting carboxylic esters. 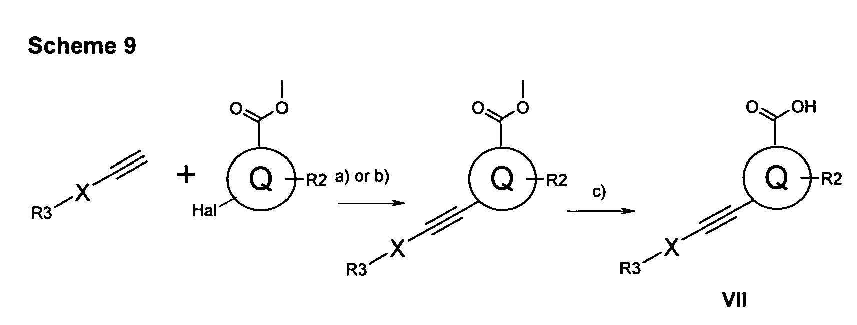 **Reagents:** a) Pd(PPh₃)₂Cl₂, TBAF, neat or THF; b) Pd(PPh₃)₂Cl₂, Cul, Et₂NH; c) KOH, MeOH.

The acetylene derivatives of formula I can in principle also be prepared according to Scheme 10 via a Sonogashira type coupling of terminal acetylenes with their corresponding aryl halides. 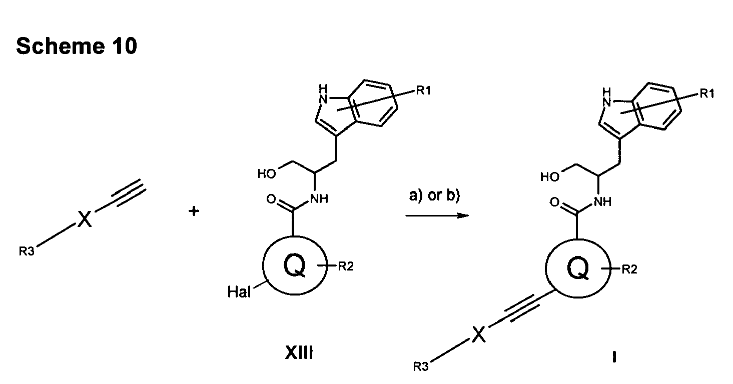 **Reagents:** a) Pd(PPh₃)₂Cl₂, TBAF, neat or THF; b) Pd(PPh₃)₂Cl₂, Cul, Et₂NH.

Compounds of the general formula XV can in principle be prepared according to Scheme 11 by an amide-formation reaction between a tryptophanol derivative VI and their corresponding carboxylic acid. The reagents typically used for the coupling are EDC and HOBt. 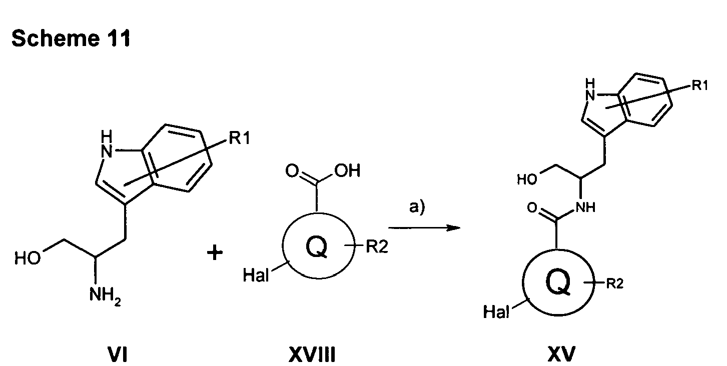 **Reagents:** a) EDCl, HOBt, DMF, Et₃N, DMF.

The compounds according to the invention of the general formula I can be prepared as described below.

### Abbreviations used:

- ACN: Acetonitrile
- DIBAH: Diisobutylaluminium hydride
- DMF: *N,N-*Dimethylformamide
- EDC: *N*-Ethyl-*N*'-(3-dimethylaminopropyl)carbodiimide
- EtOH: Ethanol
- HATU: O-(7-Azabenzotriazol-1-yl)-N,N,N',N'-tetramethyluronium hexafluorophosphate
- FMOC: (9*H*-Fluoren-9-ylmethoxy)carbonyl
- HOBt: 1-Hydroxy-1*H*-benzotriazole
- MeCN: Acetonitrile
- MeOH: Methanol
- MTBE: Methyl tert-butyl ether
- NMM: 4-methylmorpholine
- NMP: N-Methylpyrrolidinone
- Rf: Reflux
- RT: Room temperature
- TBAF: Tetrabutylammonium fluoride
- TFA: Trifluoroacetic acid
- THF: Tetrahydrofuran

### Synthesis of the compounds according to the invention

### Example 1

### 5-(3-Cyclopentyl-prop-1-ynyl)-N-[(R)-1-hydroxymethyl-2-(1H-indol-3-yl)-ethyl]-2-isopropoxy-benzamide

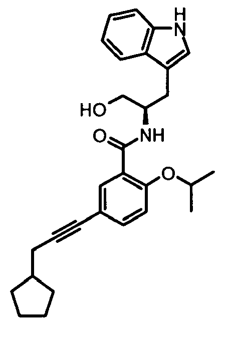

### 1a) 5-lodo-2-isopropoxy-benzoic acid methyl ester

A suspension of 5-lodo-2-hydroxy benzoic acid methyl ester (50 g), potassium carbonate (74,6 g) and *iso-*propyl iodide (89,9 ml) in acetone (500 ml) was stirred under reflux overnight. The reaction mixture was allowed to cool down to ambient temperature and the solid was removed by filtration. The filtrate was evaporated and the title compound was obtained in 96 % yield (55,1 g). **¹H-NMR (CDCl₃):** 8.02 d (*J* = 2.3 Hz, 1H); 7.67 dd (*J* = 2.5 Hz / 8.9 Hz, 1H); 6.74 d (*J* = 8.9 Hz, 1H); 4.54 m (1H); 3.87 s (3H); 1.36 m (6H).

### 1b) 5-lodo-2-isopropoxy-benzoic acid

5-lodo-2-isopropoxy-benzoic acid methyl ester (10g) and KOH (10% in MeOH, 30 mL) in MeOH (200 mL) were stirred at ambient temperature overnight. The reaction mixture was diluted with water (200 mL) and acidified with conc. HCl. The title compound was obtained in 97% yield after filtration of the precipitate and drying in vacuum. **¹H-NMR (DMSO-d₆):** 12.77 s (1H); 7.78 d (*J* = 2.3 Hz, 1H); 7.70 dd (*J* = 2.3 Hz / 8.8 Hz, 1H); 6.94 d (*J* = 8.8 Hz, 1H); 4.59 m (1H); 1.21 d (*J* = 6.1 Hz, 1H).

### 1c) N-[(R)-1-Hydroxymethyl-2-(1H-indol-3-yl)-ethyl]-5-iodo-2-isopropoxy-benzamide

5-lodo-2-isopropoxy-benzoic acid (9.31 g), (D)-tryptophanol (6.94 g), EDCl (7 g), HOBt hydrate (5.6 g) and triethylamine (8.43 mL) in DMF (120 mL) were stirred at ambient temperature overnight. The reaction mixture was poured into water (500 ml), the precipitate was filtered off and washed with water to yield the title compound in quantitative yield. **¹H-NMR (DMSO-d₆):** 10.82 s (1H); 8.38 d (*J* = 8.1 Hz, 1H); 8.14 d (*J* = 2.5 Hz, 1H); 7.75 dd (*J* = 8.9 Hz / 2.5 Hz, 1H); 7.67 d (*J* = 7.9 Hz, 1H); 7.33 d (*J* = 8.1 Hz, 1H); 7.14 d (*J* = 2.3 Hz, 1H); 7.04 m (2H); 4.95 m (1H); 4.74 m (1H); 4.22 m (1H); 3.47 m (2H); 2.98 m (2H); 1.23 m (6H).

### 1d) 5-(3-Cyclopentyl-prop-1-ynyl)-N-[(R)-1-hydroxymethyl-2-(1H-indol-3-yl)-ethyl]-2-isopropoxy-benzamide

N-[(R)-1-Hydroxymethyl-2-(1H-indol-3-yl)-ethyl]-5-iodo-2-isopropoxy-benzamide (168 mg), Prop-2-ynyl-cyclopentane (110 µL), Pd(PPh₃)Cl₂ (12 mg) and Cul (67 mg) in diethylamine (7 mL) were stirred under reflux for 3 hours. The solvent was removed and the crude mixture was purified by flash chromatography to yield 50 % of the title compound. **(DMSO-*d₆*):** 10.77 s (1H); 8.34 d (*J* = 8.3 Hz, 1H); 7.82 d (*J* = 2.3 Hz, 1H); 7.64 d (*J* = 7.9 Hz, 1H); 7.40 dd (*J* = 2.3 Hz / 8.5 Hz, 1H); 7.10 m (2H); 7.02 m (1H); 6.93 m (1H); 4.89 m (1H); 4.72 m (1H); 4.18 m (1H); 3.42 m (2H); 2.92 m (2H); 2.36 d (*J* = 6.7 Hz, 2H); 2.05 m (1H); 1.73 m (2H); 1.53 m (4H); 1.27 m (2H); 1.19 m (6H).

The following compounds were obtained in analogy to the preparation methods described in detail:

| **Ex.** | **Product;** **reagents** | **Method analogous to** | **¹H-NMR (400 MHz) δ** **[ppm]** | **Structure** |
|---|---|---|---|---|
| **2** | 5-Cyclopentylethynyl-N-[(R)-1-hydroxymethyl-2-(1H-indol-3-yl)-ethyl]-2-isopropoxy-benzamide; *N-[(R)-1-Hydroxy-methyl-2-(1H-indol-3-yl)-ethyl]-5-iodo-2-iso-propoxy-benzamide* *and* *Ethynyl-cyclopentane* | **1** | **(DMSO-*d*₆):** 10.78 s (1H); 8.33 d (*J* = 8.1 Hz, 1H); 7.81 d (*J* = 2.5 Hz, 1H); 7.63 d (*J* = 7.8 Hz, 1H); 7.38 dd (*J* = 2.3 Hz / 10.9 Hz, 1H); 7.29 d (*J* = 8.1 Hz, 1H); 7.09 m (2H); 7.02 m (1H); 6.92 m (1H); 4.90 m (1H); 4.72 m (1H); 4.16 m (1H); 3.44 m (2H); 2.91 m (2H); 2.78 m (1H); 1.91 m (2H); 1.67 m (2H); 1.54 m (4H); 1.19 m (6H). | 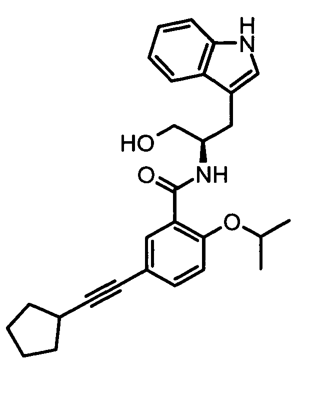 |
| **3** | 5-(3,3-Dimethyl-but-1-ynyl)-N-[(R)-1-hydroxy-methyl-2-(1H-indol-3-yl)-ethyl]-2-isopropoxy-benzamide; *N-[(R)-1-Hydroxy methyl-2-(1H-indol-3-yl)-ethyl]-5-iodo-2-iso-propoxy-benzamide* *and* *3,3-Dimethyl-but-1-yne* | **1** | **(DMSO-*d₆*):** 10.77 s (1H); 8.33 d (*J* = 8.1 Hz, 1H); 7.81 d (*J* = 2.3 Hz, 1H); 7.64 d (*J* = 7.6 Hz, 1H); 7.36 d (*J* = 8.6 Hz / 2.3 Hz, 1H); 7.29 d (*J* = 8.1 Hz, 1H); 7.09 m (2H); 7.02 m (1H); 6.92 m (1H); 4.90 m (1H); 4.74 m (1H); 4.19 m (1H); 3.41 m (2H); 2.92 m (2H); 1.25 s (9H); 1.19 m (6H). | 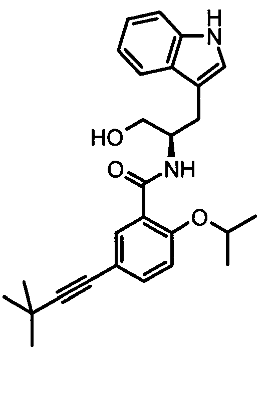 |
| **4** | 5-(4-Hydroxy-but-1-ynyl)-N-[(R)-1-hydroxy-methyl-2-(1H-indol-3-yl)-ethyl]-2-isopropoxy-benzamide; *N-[(R)-1-Hydroxy-methy*/*-2-(1H-indo*/*-3-yl)-ethyl]-5-iodo-2-iso-propoxy-benzamide* *and* *But-3-yn-1-ol* | **1** | **(DMSO-*d₆*):** 10.77 s (1H); 8.33 d (*J* = 8.3 Hz, 1H); 7.86 d (*J* = 2.5 Hz, 1H); 7.63 d (*J* = 8.1 Hz, 1H); 7.41 dd d (*J* = 8.5 Hz / 2.3 Hz, 1H); 7.29 d (*J* = 8_{.}1 Hz, 1H); 7.10 m (2H); 7.02 m (1H); 6.93 m (1H); 4.86 m (2H); 4.72 m (1H); 4.18 m (1H); 3.55 m (2H); 3.39 m (2H); 2.92 m (2H); 1.19 m (6H). | 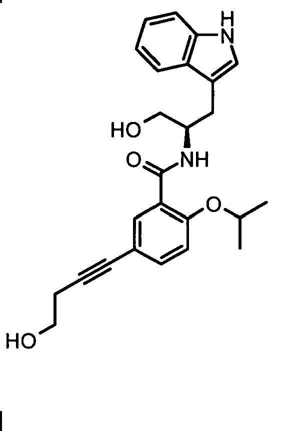 |
| **5** | 5-[3-(1,1-Dioxo-1-lambda*6*-thiomorpho-lin-4-yl)-prop-1-ynyl]-N-[(R)-2-hydroxy-1-(1H-indol-3-ylmethyl)-ethyl]-2-isopropoxy-benzamide; *N-[(R)-1-Hydroxy-methyl-2-(1H-indol-3-yl)-ethyl]-5-iodo-2-iso-propoxy-benzamide* *and* *4-Prop-2-ynyl-thio-morpholine 1,1-dioxide* | **1** | **(DMSO-*d₆*):** 10.78 s (1H); 8.34 d (*J* = 8.3 Hz, 1H); 7.90 d (*J* = 2.3 Hz, 1H); 7.64 d (*J* = 8.1 Hz, 1H); 7.46 dd (*J* = 2.3 Hz / 8.6 Hz, 1H); 7.30 d (*J* = 8.1 Hz, 1H); 7.14 d (*J* = 8.9 Hz, 1H); 7.11 d (*J* = 2.1 Hz, 1H); 7.02 m (1H); 6.93 m (1H); 4.90 m (1H); 4.75 m (1H); 4.18 m (1H); 3.65 s (2H); 3.44 m (2H), 3.13 m (4H); 2.98 m (4H); 1.20 m (6H). | 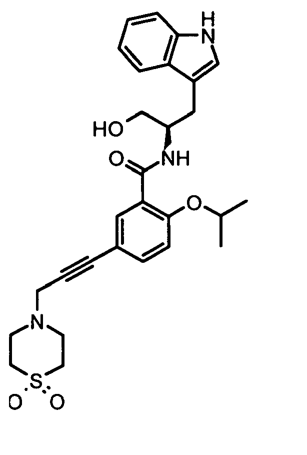 |
| **6** | 6-{3-[(R)-1-Hydroxy-methyl-2-(1H-indol-3-yl)-ethylcarbamoyl]-4-isopropoxy-phenyl}-hex-5-ynoic acid methyl ester; *N-[(R)-1-Hydroxy-methyl-2-(1H-indol-3-yl)-ethyl]-5-iodo-2-iso-propoxy-benzamide* and *Hex-5-ynoic acid methyl ester* | **1** | **(CDCl₃):** 8.43 d (*J* = 7.2 Hz, 1H); 8.27 d (*J* = 2.3 Hz, 1H); 8.12 s (1H); 7.69 d (*J* = 7.7 Hz, 1H); 7.42 dd (*J* = 2.3 Hz / 8.5 Hz, 1H); 7.35 d (*J* = 8.1 Hz, 1H); 7.19 m (1H); 7.11 m (1H); 7.08 d *(J* = 1.0 Hz, 1H); 6.83 d (*J =* 8.7 Hz, 1H); 4.62 m (1H); 4.58 m (1H); 3.81 m (2H); 3.69 s (3H); 3.13 m (2H); 2.51 m (4H); 1.91 m (2H); 1.25 d (*J* = 6.0 Hz, 3H); 1.19 d (*J* = 6.0 Hz, 3H). | 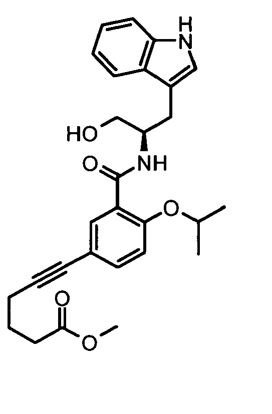 |
| 7 | N-[(R)-1-Hydroxy-methyl-2-(1H-indol-3-yl)-ethyl]-2-isopropoxy-5-[3-(3-p-tolyl-ureido)-prop-1-ynyl]-benzamide; *N-[(R)-1-Hydroxy-methyl-2-(1H-indol-3- yl)-ethyl]-5-iodo-2-iso-propoxy-benzamide* *and* *1-Prop-2-ynyl-3-p-tolyl-urea* | **1** | **(DMSO-*d*₆):** 10.77 s (1H); 8.45 s (1H); 8.33 d (*J* = 8.1 Hz, 1H); 7.87 d (*J* = 2.3 Hz, 1H); 7.63 d (*J* = 8.1 Hz, 1H); 7.44 dd (*J* = 2.3 Hz / 8.7 Hz, 1H); 7.29 d (*J* = 7.9 Hz, 1H); 7.25 d (*J* = 8.5 Hz, 2H); 7.10 m (2H); 7.01 m (3H); 6.92 m (1H); 6.47 m (1H); 4.89 m (1H); 4.73 m (1H); 4.19 m (1H); 4.08 d (*J* = 5.5 Hz, 1H); 3.42 m (2H); 2.92 m (2H); 2.18 s (3H); 1.19 m (6H). | 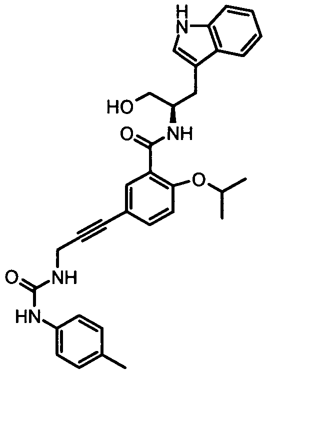 |
| **8** | N-[(R)-1-Hydroxy-methyl-2-(1H-indol-3-yl)-ethyl]-5-(5-hydroxy-pent-1-ynyl)-2-iso-propoxy-benzamide; *N-((R)-1-Hydroxy-methyl-2-(1H-indol-3-yl)-ethyl]-5-iodo-2-iso-propoxy-benzamide* *and* *Pent-4-yn-1-ol* | **1** | **(CDCl*₃*):** 8.46 d (*J* = 7.5 Hz, 1H); 8.26 d (*J* = 2.3 Hz, 1H); 8.10 s (1H); 7.69 d (*J* = 7.7 Hz, 1H); 7.41 dd (*J* = 8.5 Hz / 2.3 Hz, 1H); 7.34 d (*J* = 8.1 Hz, 1H); 7.19 m (1H); 7.14 m (1H); 7.08 s (1H); 6.83 d (*J* = 8.7 Hz, 1H); 4.62 m (2H); 3.82 m (4H); 3.13 m (2H); 2.52 m (2H); 1.87 m (2H); 1.26 m (3H); 1.19 m (3H). | 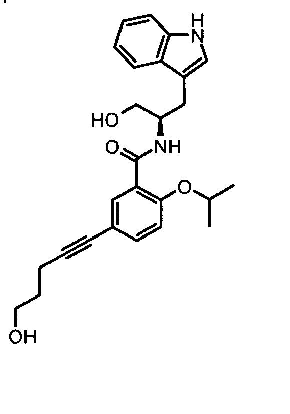 |
| **9** | 5-(5-Cyano-pent-1-ynyl)-N-[(R)-1-hydroxy-methyl-2-(1H-indol-3-yl)-ethyl]-2-isopropoxy-benzamide; *N-[(R)-1-Hydroxy-methyl-2-(1H-indol-3-y*/*)-ethyl]-5-iodo-2-iso-propoxy-benzamide* *and* *Hex-5-ynenitrile* | **1** | **(DMSO-*d₆*):** 10.78 s (1H); 8.33 d (*J* = 8.1 Hz, 1H); 7.86 d (*J* = 2.3 Hz, 1H); 7.63 d (*J* = 7.9 Hz, 1H); 7.45 dd (*J* = 8.5 Hz / 2.3 Hz, 1H); 7.29 d (*J* = 8.1 Hz, 1H); 7.11 m (2H); 7.02 m (1H); 6.93 m (1H); 4.88 m (1H); 4.73 m (1H); 4.18 m (1H); 3.44 m (2H); 2.92 m (2H); 2.62 m (2H); 2.49 m (2H); 1.80 m (2H); 1.20 m (6H). | 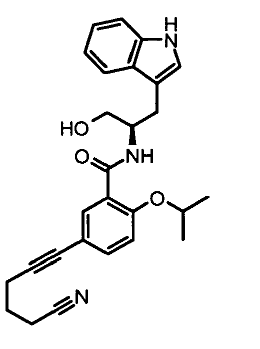 |
| **10** | 5-[3-(1,3-Dimethyl-ureido)-prop-1-ynyl]-N-[(R)-1-hydroxymethyl-2-(1H-indol-3-yl)-ethyl]-2-isopropoxy-benzamide; *N-[(R)-1-Hydroxy-methyl-2-(1H-indol-3-y*/*)-ethy*/*]-5-iodo-2-isopropoxy-benzamide* *and* *1,3-Dimethyl-1-prop-2-ynyl-urea* | **1** | **(DMSO-*d₆*):** 10.78 s (1H); 8.44 d (*J* = 8.3 Hz, 1H); 7.77 d (*J* = 2.0 Hz, 1H); 7.66 d (*J* = 7.8 Hz, 1H); 7.28 m (2H); 7.10 m (2H); 7.02 m (1H); 6,93 m (1H); 4.90 m (1H); 4.69 m (1H); 4.18 m (1H); 3.68 s (2H); 3.44 m (2H); 3.05 s (3H); 2.90 m (5H); 1.20 m (6H). | 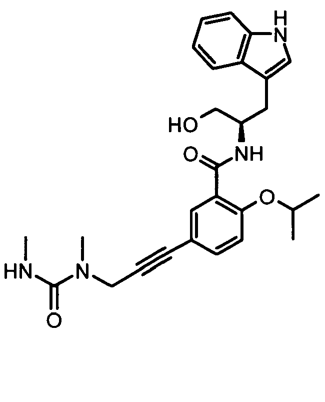 |

### Example 11

### N-[2-(5-Fluoro-1H-indol-3-yl)-1-hydroxymethyl-ethyl]-5-(3-hydroxy-prop-1-ynyl)-2-propoxy-benzamide

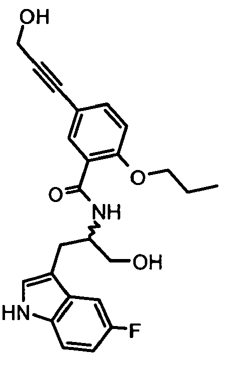

14a) N-[2-(5-Fluoro-1H-indol-3-yl)-1-hydroxymethyl-ethyl]-5-iodo-2-propoxy-benzamide The title compound was prepared in analogy to the procedures described for examples 1a-1c. **(DMSO-*d₆*):** 10.91 s (1H); 8.25 d (*J* = 8.2 Hz, 1H); 8.08 d (*J* = 2.3 Hz, 1H); 7.75 dd (*J* = 2.3 Hz / 8.6 Hz, 1H); 7.39 d (*J* = 10.2 Hz, 1H); 7.30 m (1H); 7.21 s (1H); 6.97 d (*J* = 8.6 Hz, 1H); 6.89 m (1H); 4.93 m (1H); 4.18 m (1H); 4.00 m (2H); 3.45 m (2H); 2.92 m (2H); 1.66 m (2H); 0.91 m (3H).
14b) To a solution of 0.2 mmol of Prop-2-yn-1-ol (0.2M in THF) are added 1.5 eq N-[2-(5-Fluoro-1H-indol-3-yl)-1-hydroxymethyl-ethyl]-5-iodo-2-propoxy-benzamide (0.2M in THF), 0.06 eq PdCl₂(PPh₃)₂ (0.012 M in THF) and 3 eq of TBAF (1M in THF) and the mixture is heated in the microwave oven to 80°C for 45 minutes. After evaporation, the residue is dissolved in 3mL DMSO and subjected to preparative HPLC. Machine: Analytical 4 channel MUX system with CTC Pal injector, Waters 1525 pumps, Waters 2488 UV detector and Waters ZQ 2000 single quad MS detector. HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1% (1.75'). Molecular peak (ESI, M+1): 425; retention time: 3.45 min.

The following compounds were obtained in analogy to the preparation methods described in detail:

| **Ex.** | **Product;*reagents*** | **Method analogous to** | **HPLC-MS** | **Structure** |
|---|---|---|---|---|
| **12** | N-[2-(5-Fluoro-1H-indol-3-yl)-1-hydroxy-methyl-ethyl]-5-(4-hydroxy-pent-1-ynyl)-2-propoxy-benzamide; *N-[2-(5-Fluoro-1H-indol-3-yl)-1-hydroxy-methyl-ethyl]-5-iodo-2-propoxy-benzamide* *and* *Pent-4-yn-2-ol* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 454 Retention time: 3.61 min. | 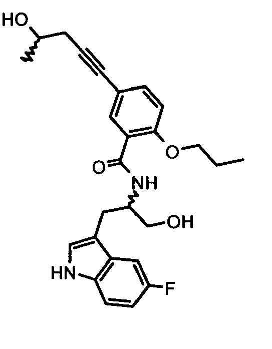 |
| **13** | 5-(3-Dimethylamino-prop-1-ynyl)-N-[2-(5-fluoro-1H-indol-3-yl)-1-hydroxymethyl-ethyl]-2-propoxy-benzamide; *N-[2-(5-Fluoro-1H-indol-3-yl)-1-hydroxy-methyl-ethyl]-5-iodo-2-propoxy-benzamide* *and* *Dimethyl-prop-2-ynyl-amine* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 453 Retention time: 2.99 min. | 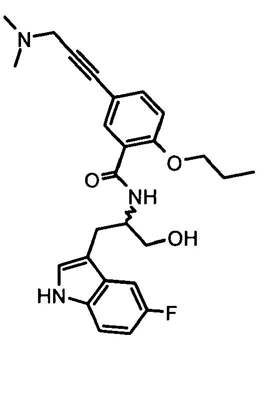 |
| **14** | 5-(5-Cyano-pent-1-ynyl)-N-[2-(5-fluoro-1H-indol-3-yl)-1-hydroxymethyl-ethyl]-2-propoxy-benzamide; *N-[2-(5-Fluoro-1H-indol-3-yl)-1-hydroxy-methyl-ethyl]-5-iodo-2-propoxy-benzamide and Hex-5-ynenitrile* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 463 Retention time: 3.90 min. | 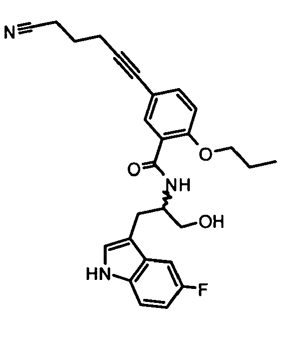 |
| **15** | N-[2-(5-Fluoro-1H-indol-3-yl)-1-hydroxymethyl-ethyl]-5-(5-hydroxy-pent-1-ynyl)-2-propoxy-benzamide; *N-[2-(5-Fluoro-1H-indol-3-yl)-1-hydroxy-methyl-ethyl]-5-iodo-2-propoxy-benzamide* *and* *Pent-4-yn-1-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 454 Retention time: 3.57 min. | 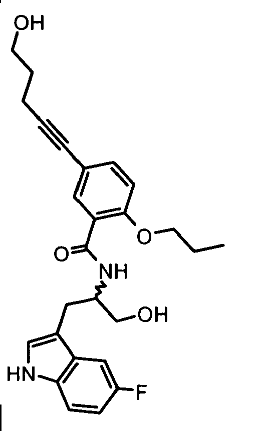 |
| **16** | N-[2-(5-Fluoro-1H-indol-3-yl)-1-hydroxy-methyl-ethyl]-5-(4-hydroxy-but-1-ynyl)-2-propoxy-benzamide; *N-[2-(5-Fluoro-1H-indol-3-yl)-1-hydroxy-methyl-ethyl]-5-iodo-2-propoxy-benzamide* *and* *But-3-yn-1-ol* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 439 Retention time: 3.81 min. | 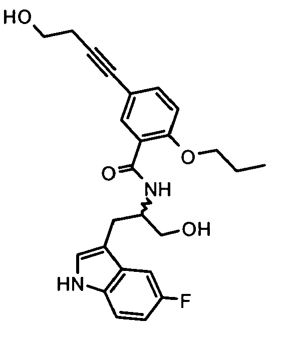 |
| **17** | N-[2-(5-Fluoro-1H-indol-3-yl)-1-hydroxy-methyl-ethyl]-5-{3-[((1R,2R)-2-hydroxy-1-methyl-propyl)-methyl-amino]-prop-1-ynyl}-2-propoxy-benzamide; *N-[2-(5-Fluoro-1H-indol-3-yl)-1-hydroxy-methyl-ethyl]-5-iodo-2-propoxy-benzamide* *and* *(2R,3R)-3-(Methyl-prop-2-ynyl-amino)-butan-2-ol* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 511 Retention time: 3.08 min. | 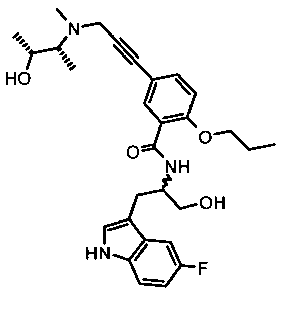 |
| **18** | 5-[4-(1,3-Dimethyl-ureido)-but-1-ynyl]-N-[2-(5-fluoro-1H-indol-3-yl)-1-hydroxymethyl-ethyl]-2-propoxy-benzamide; *N-[2-(5-Fluoro-1H-indol-3-yl)-1-hydroxy-methyl ethyl]-5-iodo-2-propoxy-benzamide* *and* *1-But-3-ynyl-1,3- dimethyl-urea* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 510 Retention time: 3.57 min. | 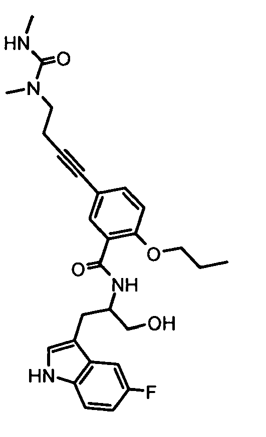 |
| **19** | N-[2-(5-Fluoro-1 H-indol-3-yl)-1-hydroxymethyl-ethyl]-5-(4-methylcarbamoyl-but-1-ynyl)-2-propoxy-benzamide; *N-[2-(5-Fluoro-1H-indol-3-yl)-1-hydroxy-methyl-ethyl]-5-iodo-2-propoxy-benzamide* *and* *Pent-4-ynoic acid me-thylamide* | **11** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1% to 99% (5') to 99% (1') to 1% (0.25') to 1% (1.75'). Molecular peak (ESI, M+1): 481 Retention time: 3.40 min. | 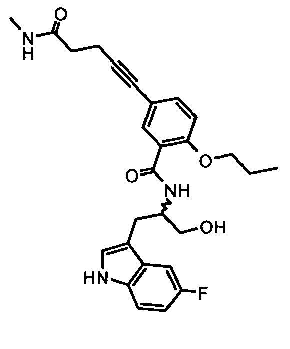 |
| **20** | N-[2-(5-Fluoro-1H-indol-3-yl)-1-hydroxy-methyl-ethyl]-5-(5-methylcarbamoyl-pent-1-ynyl)-2-propoxy-benzamide; *N-[2-(5-F*/*uoro-1H-indol-3-yl)-1-hydroxy-methyl-ethyl]-5-iodo-2-propoxy-benzamide* *and* *Hex-5-ynoic acid me-thylamide* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1 % (1.75'). Molecular peak (ESI, M+1): 495 Retention time: 3.71 min. | 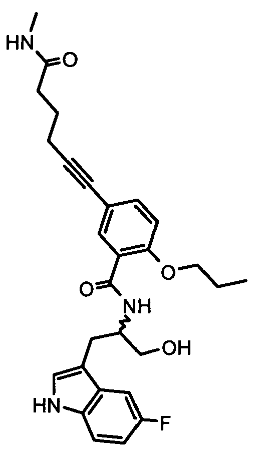 |
| **21** | N-[(R)-1-Hydroxymethyl-2-(1H-indol-3-yl)-ethyl]-5-(3-hydroxy-prop-1-ynyl)-2-isopropoxy-benzamide; *N-[(R)-1-Hydroxy-methy*/*-2-(1H-indo*/*-3-yl)-ethyl]-5-iodo-2-iso-propoxy-benzamide* *and* *Prop-2-yn-1-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 407 Retention time: 3.34 min. | 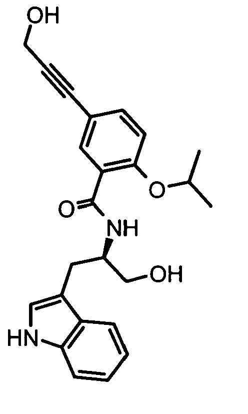 |
| **22** | N-[(R)-1-Hydroxymethyl-2-(1H-indol-3-yl)-ethyl]-5-(4-hydroxy-pent-1-ynyl)-2-isopropoxy-benzamide; *N-[(R)-1-Hydroxy-methyl-2-(1H-indol-3-yl)-ethyl]-5-iodo-2-iso-propoxy-benzamide* *and* *Pent-4-yn-2-ol* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 436 Retention time: 3.52 min. | 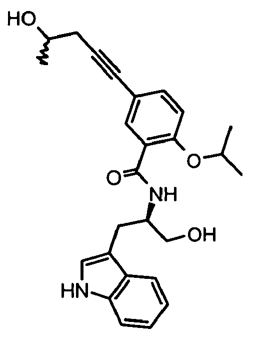 |
| **23** | 5-[3-(Benzyl-methyl-amino)-prop-1-ynyl]-N-[(R)-1-hydroxymethyl-2-(1H-indol-3-yl)-ethyl]-2-isopropoxy-benzamide; *N-[(R)-1-Hydroxy-methyl-2-(1H-indol-3-yl)-ethyl]-5-iodo-2-isopropoxy-benzamide* *and* *Benzyl-methyl-prop-2-ynyl-amine* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 511 Retention time: 3.19 min. | 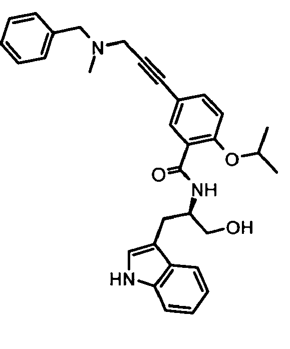 |
| **24** | 5-(3-Dimethylamino-prop-1-ynyl)-N-[(R)-1-hydroxymethyl-2-(1H-indol-3-yl)-ethyl]-2-isopropoxy-benzamide; *N-[(R)-1-Hydroxy-methyl-2-(1H-indol-3-yl)-ethyl]-5-iodo-2-iso-propoxy-benzamide* *and* *Dimethyl-prop-2-ynyl-amine* | **11** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1% to 99% (5') to 99% (1') to 1% (0.25') to 1% (1.75'). Molecular peak (ESI, M+1): 435 Retention time: 2.92 min. | 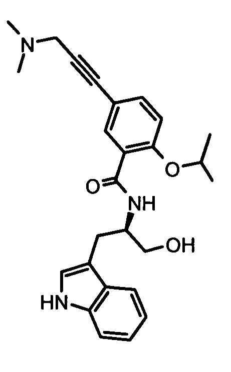 |
| **25** | 5-[3-(1,3-Dimethyl-ureido)-prop-1-ynyl]-N-[(R)-1-hydroxymethyl-2-(1 H-indol-3-yl)-ethyl]-2-isopropoxy-benzamide; *N-[(R)-1-Hydroxy-methyl-2-(1H-indol-3-y*/*)-ethy*/*]-5-iodo-2-isopropoxy-benzamide* *and* *1,3-Dimethyl-1-prop-2-ynyl-urea* | **11** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1 % (1.75'). Molecular peak (ESI, M+1): 478 Retention time: 3.17 min. | 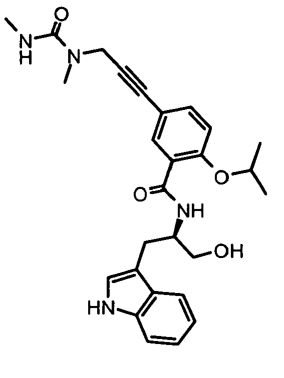 |
| **26** | 6-{3-[(R)-1-Hydroxy-methyl-2-(1H-indol-3-yl)-ethylcarbamoyl]-4-isopropoxy-phenyl}-hex-5-ynoic acid methyl ester; *N-[(R)-1-Hydroxy-methyl-2-(1H-indol-3-yl)-ethyl]-5-iodo-2-isopropoxy-benzamide* *and* *Hex-5-ynoic acid methyl ester* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 478 Retention time: 4.02 min. | 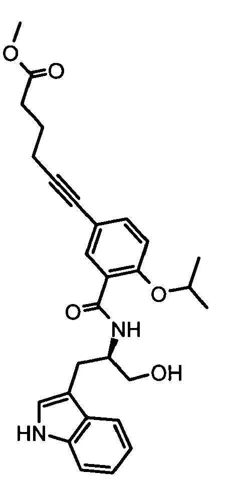 |
| **27** | 5-(6-Hydroxy-hex-1-ynyl)-N-[(R)-1-hydroxymethyl-2-(1H-indol-3-yl)-ethyl]-2-isopropoxy-benzamide; *N-[(R)-1-Hydroxy-methyl-2-(1H-indol-3-yl)-ethyl]-5-iodo-2-isopropoxy-benzamide* *and* *Hex-5-yn-1-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1% (1.75'). Molecular peak (ESI, M+1): 450 Retention time: 3.61 min. | 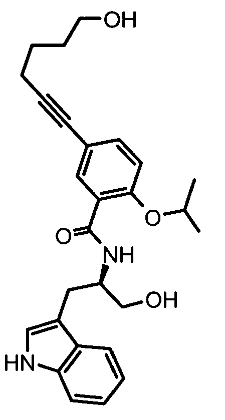 |
| **28** | 5-(5-Cyano-pent-1-ynyl)-N-[(R)-1-hydroxymethyl-2-(1H-indol-3-yl)-ethyl]-2-isopropoxy-benzamide; *N-[(R)-1-Hydroxy-methyl-2-(1H-indol-3-yl)-ethyl]-5-iodo-2-isopropoxy-benzamide* *and* *Hex-5-ynenitrile* | **11** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1% (1.75'). Molecular peak (ESI, M+1): 445 Retention time: 3.85 min. | 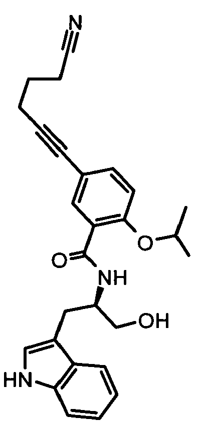 |
| **29** | N-[(R)-1-Hydroxymethyl-2-(1H-indol-3-yl)-ethyl]-5-(5-hydroxy-pent-1-ynyl)-2-isopropoxy-benzamide; *N-[(R)-1-Hydroxy-methyl-2-(1H-indol-3-yl)-ethyl]-5-iodo-2-isopropoxy-benzamide* *and* *Pent-4-yn-1-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1 % (1.75'). Molecular peak (ESI, M+1): 436 Retention time: 3.42 min. | 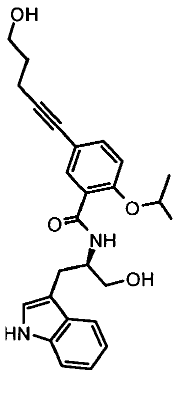 |
| **30** | 5-(4-Hydroxy-but-1-ynyl)-N-[(R)-1-hydroxymethyl-2-(1H-indol-3-yl)-ethyl]-2-isopropoxy-benzamide; *N-[(R)-1-Hydroxy-methyl-2-(1H-indol-3-yl)-ethyl]-5-iodo-2-isopropoxy-benzamide* *and* *But-3-yn-l-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 422 Retention time: 3.39 min. | 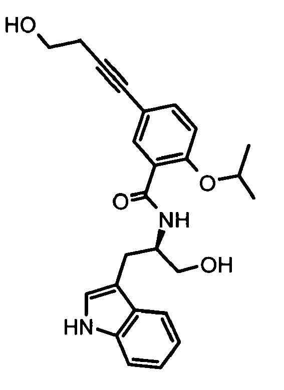 |
| **31** | Acetic acid 3-{3-[(R)-1-hydroxymethyl-2-(1H-indol-3-yl)-ethylcarba-moyl]-4-isopropoxy-phenyl}-prop-2-ynyl ester; *N-[(R)-1-Hydroxy-methyl-2-(1H-indol-3-yl)-ethyl]-5-iodo-2-isopropoxy-benzamide* *and* *Acetic acid prop-2-ynyl ester* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 450 Retention time: 3.71 min. | 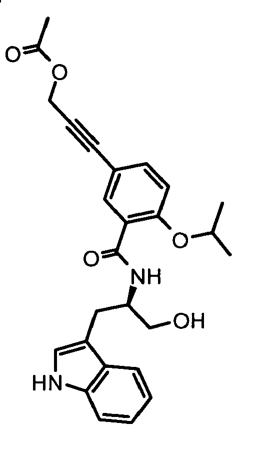 |
| **32** | N-[(R)-1-Hydroxy-methyl-2-(1 H-indol-3-yl)-ethyl]-5-{3-[((1R,2R)-2-hydroxy-1-methyl-propyl)-methyl-amino]-prop-1-ynyl}-2-isopropoxy-benzamide; *N-[(R)-1-Hydroxy-methyl-2-(1H-indol-3-yl)-ethyl]-5-iodo-2-isopropoxy-benzamide* *and (2R,3R)-3-(Methyl-* *prop-2-ynyl-amino)-butan-2-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 493 Retention time: 3.05 min. | 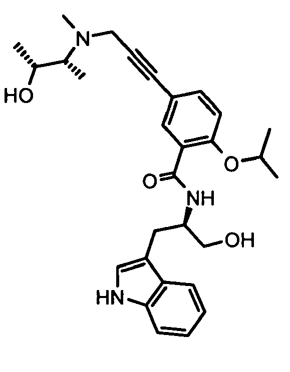 |
| **33** | 9-{3-[(R)-1-Hydroxymethyl-2-(1 H-indol-3-yl)-ethylcarba-moyl]-4-isopropoxy-phenyl}-non-8-ynoic acid; *N-[(R)-1-Hydroxy-methyl-2-(1H-indol-3-yl)-ethyl]-5-iodo-2-isopropoxy-benzamide* *and* *Non-8-ynoic acid* | **11** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1% (1.75'). Molecular peak (ESI, M+1): 506 Retention time: 3.99 min. | 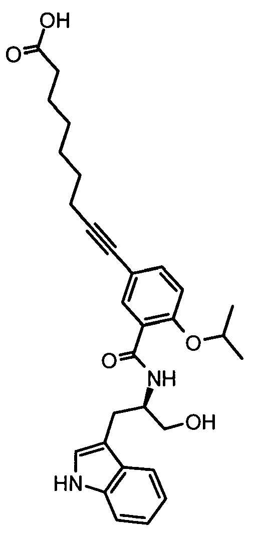 |
| **34** | 5-[4-(1,3-Dimethyl-ureido)-but-1-ynyl]-N-[(R)-1-hydroxymethyl-2-(1 H-indol-3-yl)-ethyl]-2-isopropoxy-benzamide; *N-[(R)-1-Hydroxy methyl-2-(1H-indol-3-yl)-ethyl]-5-iodo-2-isopropoxy-benzamide* *and* *1-But-3-ynyl-1,3-dimethyl-urea* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 492 Retention time: 3.38 min. | 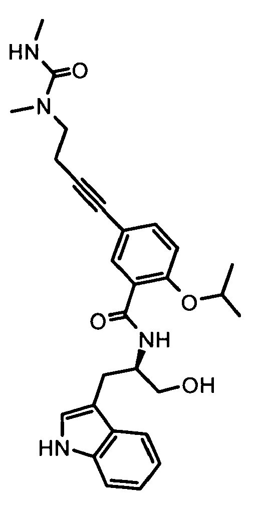 |
| **35** | N-[(R)-1-Hydroxymethyl-2-(1H-indol-3-yl)-ethyl]-2-isopropoxy-5-(4-methylcarbamoyl-but-1-ynyl)-benzamide; *N-[(R)-1-Hydroxy-methyl-2-(1H-indol-3-yl)-ethyl]-5-iodo-2-iso-propoxy-benzamide* *and* *Pent-4-ynoic acid me- thylamide* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 463 Retention time: 3.34 min. | 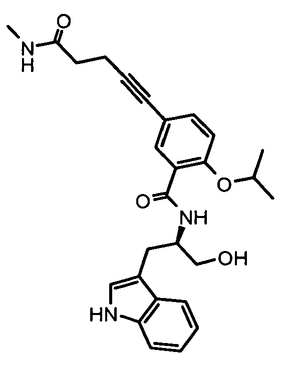 |
| **36** | N-[(R)-1-Hydroxy-methyl-2-(1H-indol-3-yl)-ethyl]-2-isopropoxy-5-(5-methylcarbamoyl-pent-1-ynyl)-benzamide; *N-[(R)-1-Hydroxy-methyl-2-(1H-indol-3-yl)-ethyl]-5-iodo-2-iso-propoxy-benzamide* *and* *Hex-5-ynoic acid me-thylamide* | **11** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1% to 99% (5') to 99% (1') to 1% (0.25') to 1 % (1.75'). Molecular peak (ESI, M+1): 477 Retention time: 3.34 min. | 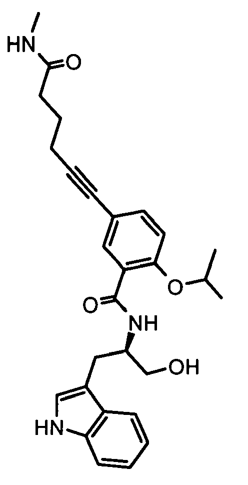 |
| **37** | N-[(R)-1-Hydroxy-methyl-2-(1H-indol-3-yl)-ethyl]-3-(3-hydroxy-prop-1-ynyl)-benzamide; *N-[(R)-1-Hydroxy-methyl-2-(1H-indol-3-yl)-ethyl]-3-iodo-benzamide* *and* *Prop-2-yn-1-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 349 Retention time: 2.91 min. | 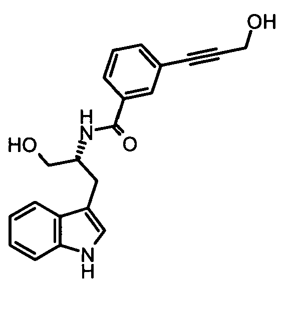 |
| **38** | N-[(R)-1-Hydroxy-methyl-2-(1H-indol-3-yl)-ethyl]-3-(3-hydroxy-prop-1-ynyl)-benzamide; *N-[(R)-1-Hydroxy-methyl-2-(1H-indol-3-y*/*)-ethyl]-3-iodo-benzamide* *and* *Pent-4-yn-2-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 377 Retention time: 3.15 min. | 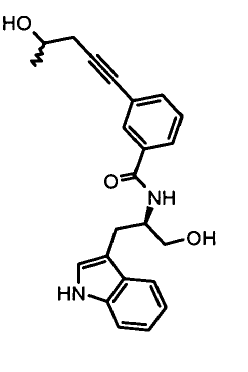 |
| 39 | 3-[3-(Benzyl-methyl-amino)-prop-1-ynyl]-N-[(R)-1-hydroxymethyl-2-(1H-indol-3-yl)-ethyl]-benzamide; *N-[(R)-1-Hydroxy-methyl-2-(1H-indol-3-yl)-ethyl]-3-iodo-benzamide* *and* *Benzyl-methyl-prop-2-ynyl-amine* | 11 | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 453 Retention time: 2.90 min. | 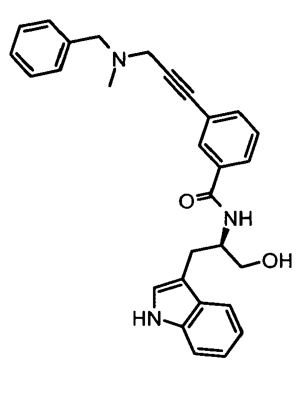 |
| 40 | 3-(3-Dimethylamino-prop-1-ynyl)-N-[(R)-1-hydroxymethyl-2-(1H-indol-3-yl)-ethyl]-benzamide; *N-[(R)-1-Hydroxy-methyl-2-(1H-indol-3-yl)-ethyl]-3-iodo-benzamide* *and* *Dimethyl-prop-2-ynyl-amine* | **11** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1 % (1.75'). Molecular peak (ESI, M+1): 376 Retention time: 2.56 min. | 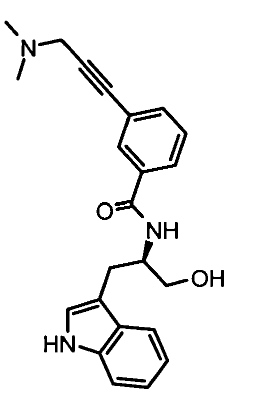 |
| **41** | 3-[3-(1,3-Dimethyl-ureido)-prop-1-ynyl]-N-[(R)-1-hydroxymethyl-2-(1 H-indol-3-yl)-ethyl]-benzamide; *N-[(R)-1-Hydroxy-methyl-2-(1H-indol-3-yl)-ethyl]-3-iodo-benzamide* *and* *1,3-Dimethyl-1-prop-2-ynyl-urea* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 419 Retention time: 2.79 min. | 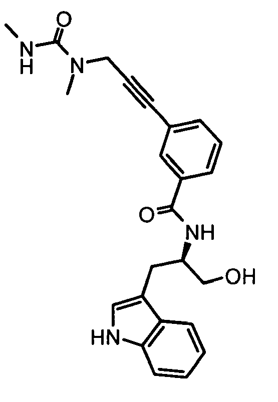 |
| **42** | 6-{3-[(R)-1-Hydroxy-methyl-2-(1H-indol-3-yl)-ethylcarbamoyl]-phenyl}-hex-5-ynoic acid; *N-[(R)-1-Hydroxy-methyl-2-(1H-indol-3-yl)-ethyl]-3-iodo-benzamide* *and* *Hex-5-ynoic acid* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 405 Retention time: 3.21 min. | 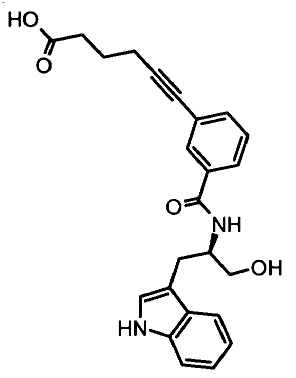 |
| **43** | 6-{3-[(R)-1-Hydroxy-methyl-2-(1H-indol-3-yl)-ethylcarbamoyl]-phenyl}-hex-5-ynoic acid methyl ester; *N-[(R)-1-Hydroxy-methyl-2-(1H-indol-3-yl)-ethyl]-3-iodo-benzamide* *and* *Hex-5-ynoic acid methyl ester* | **11** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1 % (1.75'). Molecular peak (ESI, M+1): 419 Retention time: 3.61 min. | 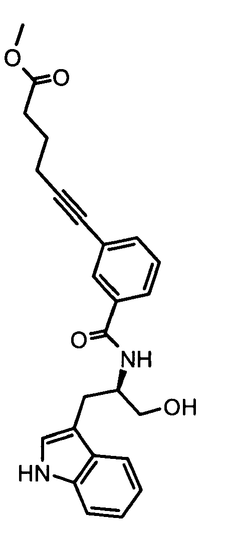 |
| **44** | 3-(6-Hydroxy-hex-1-ynyl)-N-[(R)-1-hydroxymethyl-2-(1H-indol-3-yl)-ethyl]-benzamide; *N-[(R)-1-Hydroxy-methyl-2-(1H-indol-3-yl)-ethyl]-3-iodo-benzamide* *and* *Hex-5-yn-1-ol* | **11** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1% to 99% (5') to 99% (1') to 1% (0.25') to 1 % (1.75'). Molecular peak (ESI, M+1): 391 Retention time: 3.25 min. | 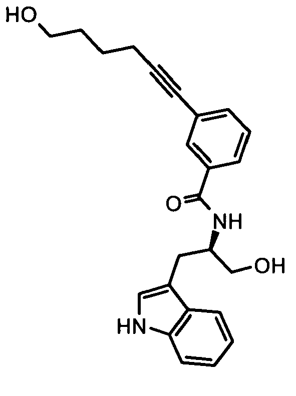 |
| **45** | 3-(5-Cyclohexyl-pent**-** 1-ynyl)-N-[(R)-1-hydroxymethyl-2-(1H-indol-3-yl)-ethyl]-benzamide; *N-[(R)-1-Hydroxy-methyl-2-(1H-indol-3-yl)-ethyl]-3-iodo-benzamide* *and* *Pent-4-ynyl-cyclohexane* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 444 Retention time: 5.12 min. | 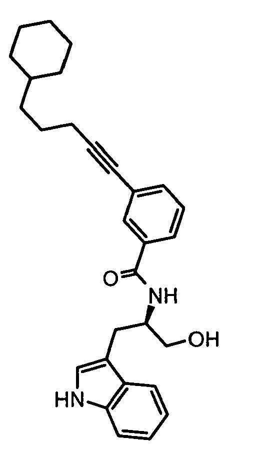 |
| **46** | 3-(5-Cyano-pent-1-ynyl)-N-[(R)-1-hydroxymethyl-2-(1 H-indol-3-yl)-ethyl]-benzamide; *N-[(R)-1-Hydroxy-methyl-2-(1H-indol-3-yl)-ethyl]-3-iodo-benzamide* *and* *Hex-5-ynenitrile* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 386 Retention time: 3.39 min. | 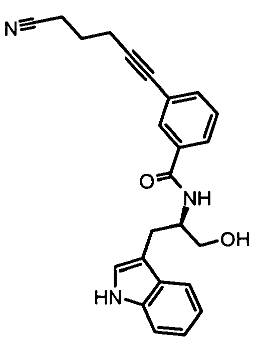 |
| **47** | N-[(R)-1-Hydroxy-methyl-2-(1H-indol-3-yl)-ethyl]-3-(5-hydroxy-pent-1-ynyl)-benzamide; *N-[(R)-1-Hydroxy-methyl-2-(1H-indol-3-y*/*)-ethyl]-3-iodo-benzamide* *and* *Pent-4-yn-1-ol* | **11** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1% to 99% (5') to 99% (1') to 1% (0.25') to 1 % (1.75'). Molecular peak (ESI, M+1): 377 Retention time: 3.08 min. | 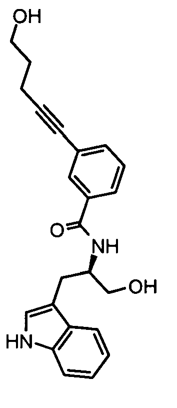 |

| **Ex.** | **Product; *reagents*** | **Method analogous to** | **HPLC-MS** | **Structure** |
|---|---|---|---|---|
| **48** | 3-(4-Hydroxy-but-1-ynyl)-N-[(R)-1-hydroxymethyl-2-(1 H-indol-3-yl)-ethyl]-benzamide; *N-[(R)-1-Hydroxy-methyl-2-(1H-indol-3-y*/*)-ethy*/*]-3-iodo-benzamide* *and* *But-3-yn-1-o*/ | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 363 Retention time: 2.96 min. | 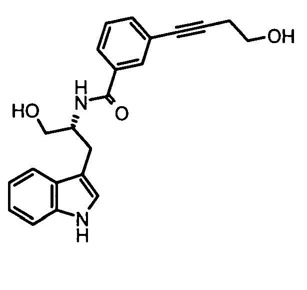 |
| **49** | N-[(R)-1-Hydroxy-methyl-2-(1 H-indol-3-yl)-ethyl]-3-{3-[((1R,2R)-2-hydroxy-1-methyl-propyl)-methyl-amino]-prop-1-ynyl}-benzamide; *N-[(R)-1-Hydroxy-methyl-2-(1H-indol-3-yl)-ethyl]-3-iodo-benzamide* *and* *(2R,3R)-3-(Methyl-prop-2-ynyl-amino)-butan-2-ol* | **11** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1% (1.75'). Molecular peak (ESI, M+1): 435 Retention time: 2.72 min. | 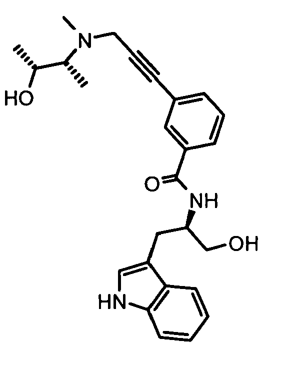 |
| **50** | N-[(R)-1-Hydroxy-methyl-2-(1H-indol-3-yl)-ethyl]-3-[3-(3-methyl-ureido)-prop-1-ynyl]-benzamide; *N-[(R)-1-Hydroxy-methyl-2-(1H-indol-3-y*/*)-ethyl]-3-iodo-benzamide* *and* *1-Methyl-3-prop-2-ynyl-urea* | **11** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1% to 99% (5') to 99% (1') to 1% (0.25') to 1 % (1.75'). Molecular peak (ESI, M+1): 405 Retention time: 2.79 min. | 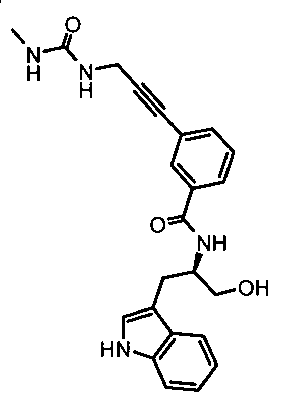 |
| **51** | 9-{3-[(R)-1-Hydroxy-methyl-2-(1H-indol-3-yl)-ethylcarbamoyl]-phenyl}-non-8-ynoic acid; *N-[(R)-1-Hydroxy-methyl-2-(1H-indol-3-yl)-ethyl]-3-iodo-benzamide* *and* *Non-8-ynoic acid* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 448 Retention time: 3.63 min. | 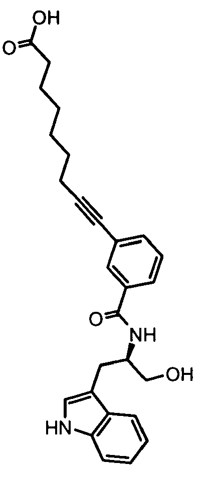 |
| **52** | 3-[4-(1,3-Dimethyl-ureido)-but-1-ynyl]-N-[(R)-1-hydroxymethyl-2-(1 H-indol-3-yl)-ethyl]-benzamide; *N-[(R)-1-Hydroxy-methyl-2-(1H-indol-3-yl)-ethyl]-3-iodo-benzamide* *and* *1-But-3-ynyl-1,3-dimethyl-urea* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 434 Retention time: 2.99 min. | 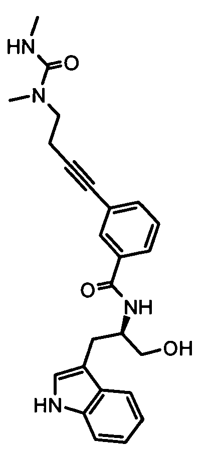 |
| **53** | N-[(R)-1-Hydroxymethyl-2-(1 H-indol-3-yl)-ethyl]-3-[4-(3-methyl-ureido)-but-1-ynyl]-benzamide; *N-[(R)-1-Hydroxy-methyl-2-(1H-indol-3-yl)-ethyl]-3-iodo-benzamide* *and* *1-But-3-ynyl-3-methyl-urea* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 419 Retention time: 2.99 min. | 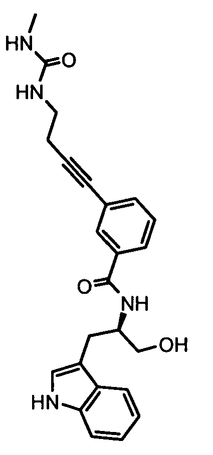 |
| **54** | N-[(R)-1-Hydroxy-methyl-2-(1H-indol-3-yl)-ethyl]-3-(4-methyl-carbamoyl-but-1-ynyl)-benzamide; *N-[(R)-1-Hydroxy-methyl-2-(1H-indol-3-yl)-ethyl]-3-iodo-benzamide* *and* *Pent-4-ynoic acid me-thylamide* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 404 Retention time: 2.99 min. | 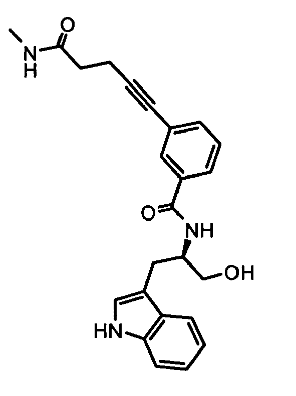 |
| **55** | N-[(R)-1-Hydroxy-methyl-2-(1H-indol-3-yl)-ethyl]-3-(5-methyl-carbamoyl-pent-1-yn-yl)-benzamide; *N-[(R)-1-Hydroxy-methyl-2-(1H-indol-3-yl)-ethyl]-3-iodo-benzamide* *and* *Hex-5-ynoic acid methylamide* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 419 Retention time: 3.03 min. | 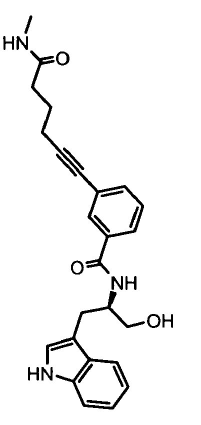 |
| **56** | 5-[3-(Benzyl-methyl-amino)-prop-1-ynyl]-2-bromo-N-[(R)-1-hydroxymethyl-2-(1 H-indol-3-yl)-ethyl]-benzamide; *2-Bromo-N-[(R)-1-hydroxymethyl-2-(1H-indol-3-yl)-ethyl]-5-iodo-benzamide* *and* *Benzyl-methyl-prop-2-ynyl-amine* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 531 Retention time: 2.99 min. | 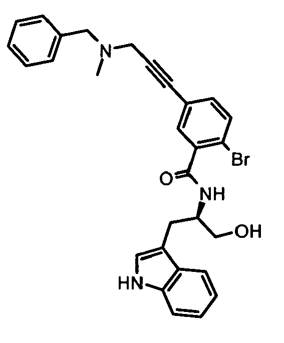 |
| **57** | 2-Bromo-5-(3-dimethylamino-prop-1-ynyl)-N-[(R)-1-hydroxymethyl-2-(1 H-indol-3-yl)-ethyl]-benzamide; *2-Bromo-N-[(R)-1-hydroxymethyl-2-(1H-indol-3-yl)-ethyl]-5-iodo-benzamide* *and* *Dimethyl-prop-2-ynyl-amine* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1 % (1.75'). Molecular peak (ESI, M+1): 455 Retention time: 2.65 min. | 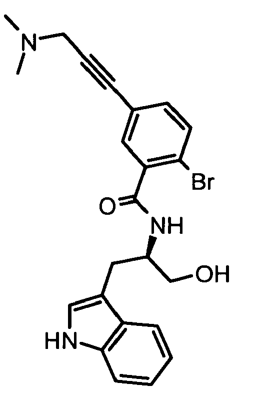 |
| **58** | 2-Bromo-5-[3-(1,3-dimethyl-ureido)-prop-1-ynyl]-N-[(R)-2-hydroxy-1-(1H-indol-3-ylmethyl)-ethyl]-benzamide; *2-Bromo-N-[(R)-1-hydroxymethyl-2-(1H-indol-3-yl)-ethyl]-5-iodo-benzamide* *and* *1,3-Dimethyl-1-prop-2-ynyl-urea* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 499 Retention time: 4.13 min. | 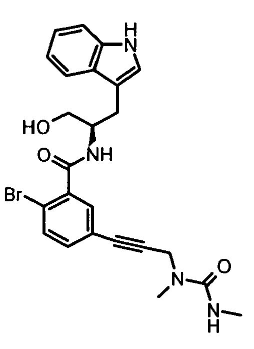 |
| **59** | 2-Bromo-5-(5-cyano-pent-1-ynyl)-N-[(R)-1-hydroxymethyl-2-(1 H-indol-3-yl)-ethyl]-benzamide; *2-Bromo-N-[(R)-1-hydroxymethyl-2-(1H-indol-3-yl)-ethyl]-5-iodo-benzamide* *and* *Hex-5-ynenitrile* | **11** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 465 Retention time: 3.49 min. | 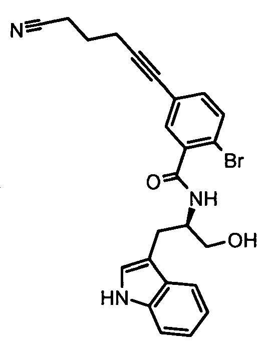 |
| **60** | 2-Bromo-N-[(R)-1-hydroxymethyl-2-(1 H-indol-3-yl)-ethyl]-5-(5-methylcarbamoyl-pent-1-ynyl)-benzamide; *2-Bromo-N-[(R)-1-hydroxymethyl-2-(1H-indol-3-yl)-ethyl]-5-iodo-benzamide* *and* *Hex-5-ynoic acid me-thylamide* | **11** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 497 Retention time: 3.13 min. | 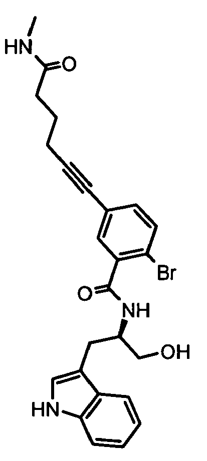 |

### Example 61

### 7-(3-Hydroxy-prop-1-ynyl)-2,3,4,5-tetrahydro-benzo[b]oxepine-9-carboxylic acid [(R)-1-hydroxymethyl-2-(1H-indol-3-yl)-ethyl]-amide

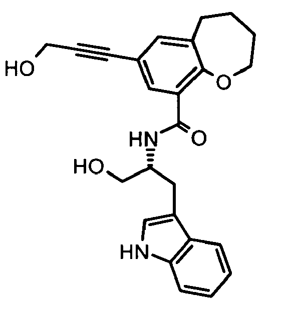

65a) 2-Allyloxy-5-bromo-benzoic acid methyl ester
   The title compound was prepared from 2-Hydroxy-5-bromo-benzoic acid methyl ester in analogy to the described literature procedure via an O-alkylation. See Eur. J. Med. Chem. 1997, 32, page 385.
65b) 3-Allyl-5-bromo-2-hydroxy-benzoic acid methyl ester
   The title compound was prepared via a Claisen rearrangement from 2-Allyloxy-5-bromobenzoic acid methyl ester in analogy to the described literature procedure. See J. Med. Chem. 1992, 35, page 310.
65c) 3-Allyl-2-allyloxy-5-bromo-benzoic acid methyl ester
   The title compound was prepared from -Allyl-5-bromo-2-hydroxy-benzoic acid methyl ester in analogy to the described literature procedure via an O-alkylation. See Eur. J. Med. Chem. 1997, 32, page 385.
65d) 7-Bromo-2,5-dihydro-benzo[b]oxepine-9-carboxylic acid methyl ester
   The title compound was prepared from 3-Allyl-2-allyloxy-5-bromo-benzoic acid methyl ester in analogy to the described literature procedure via an olefin metathesis reaction. See Heterocycles 2002, 57, page 1997.
65e) 7-Bromo-2,3,4,5-tetrahydro-benzo[b]oxepine-9-carboxylic acid methyl ester
   The title compound was prepared from 7-Bromo-2,5-dihydro-benzo[b]oxepine-9-carboxylic acid methyl ester in analogy to the described literature procedure via a hydrogenation reaction. See Org. Lett. 2006, 15, page 3279.
65f) 7-Bromo-2,3,4,5-tetrahydro-benzo[b]oxepine-9-carboxylic acid 7-Bromo-2,3,4,5-tetrahydro-benzo[b]oxepine-9-carboxylic acid methyl ester (10g) and
   KOH (10g) in MeOH (100 mL) were strirred at ambient temperature overnight. The solvent was distilled off and water was added. The solution was acidified by addition of conc. HCl and the precipitate filtered off. After drying in vacuum the title compound was obtained in 95% yield. **H-NMR (DMSO-d₆):** 7.53 d (J = 2.8 Hz, 1 H); 7.48 d (J = 2.5 Hz, 1H); 3.90 m (2H); 2.72 m (2H); 1.85 m (2H); 1.60 m (2H).
65g) 7-Bromo-2,3,4,5-tetrahydro-benzo[b]oxepine-9-carboxylic acid [(R)-1-hydroxymethyl-2-(1H-indol-3-yl)-ethyl]-amide
   7-Bromo-2,3,4,5-tetrahydro-benzo[b]oxepine-9-carboxylic acid (2.2 g), (D)-tryptophanol (1.54 g), HOBt x H₂O (1.49 g), EDCI (1.87 g), triethylamine (2.25 mL) were dissolved in DMF (8 mL) and stirred at ambient temperature overnight. The reaction mixture was poured into water and the precipitate was purified by flash chromatography to obtain the title compound in 69% yield. **H-NMR (DMSO-d₆):** 10.78 s (1 H); 8.33 d (J = 8.1 Hz, 1 H); 7.62 m (2H); 7.50 d (J = 2.5 Hz, 1 H); 7.29 d (J = 8.1 Hz, 1 H); 7.12 d (J = 2.3 Hz, 1 H); 7.02 m (1 H); 6.94 m (1H); 4.87 m (1 H); 4.16 m (1 H); 3.81 m (2H); 3.46 m (2H); 2.96 m (2H); 2.71 m (2H); 1.83 m (2H); 1.58 m (2H).
65h) 7-(3-Hydroxy-prop-1-ynyl)-2,3,4,5-tetrahydro-benzo[b]oxepine-9-carboxylic acid [(R)-1-hydroxymethyl-2-(1H-indol-3-yl)-ethyl]-amide
   To a solution of 0.2 mmol of Prop-2-yn-1-ol (0.2M in THF) are added 1.5 eq 7-Bromo-2,3,4,5-tetrahydro-benzo[b]oxepine-9-carboxylic acid [(R)-1-hydroxymethyl-2-(1H-indol-3-yl)-ethyl]-amide (0.2M in THF), 0.06 eq PdCl₂(PPh₃)₂ (0.012 M in THF) and 3 eq of TBAF (1 M in THF) and the mixture is heated in the microwave oven to 80°C for 45 minutes. After evaporation, the residue is dissolved in 3mL DMSO and subjected to preparative HPLC. Machine: Analytical 4 channel MUX system with CTC Pal injector, Waters 1525 pumps, Waters 2488 UV detector and Waters ZQ 2000 single quad MS detector. HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 419; retention time: 3.37 min.

The following compounds were obtained in analogy to the preparation methods described in detail:

| **Ex.** | **Product; *reagents*** | **Method analogous to** | **HPLC-MS** | **Structure** |
|---|---|---|---|---|
| **62** | 7-(4-Hydroxy-pent-1-ynyl)-2,3,4,5-tetra-hydro-benzo[b]-oxepine-9-carboxylic acid [(R)-1-hydroxy-methyl-2-(1 H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Pent-4-yn-2-ol* | **62** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 448 Retention time: 3.54 min. | 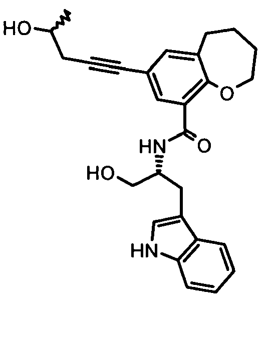 |
| **63** | 7-[3-(Benzyl-methyl-amino)-prop-1-ynyl]-2,3,4,5-tetrahydro-benzo[b]oxepine-9-carboxylic acid [(R)-1-hydroxymethyl-2-(1 H-indol-3-yl)-ethyl]-amide; *7-Bromo-2,3,4,5-tetrahydro-benzo[b]-oxepine-9-carboxylic acid [(R)-1-hydroxy-methy*/*-2-(1H-indo*/*-3-yl)-ethyl]-amide* *and* *Benzyl-methyl-prop-2-ynyl-amine* | **62** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 523 Retention time: 3.24 min. | 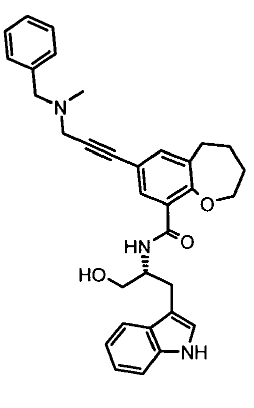 |
| **64** | 7-(3-Dimethylamino-prop-1-ynyl)-2,3,4,5-tetrahydro-benzo[b]-oxepine-9-carboxylic acid [(R)-1-hydroxy-methyl-2-(1H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Dimethyl-prop-2-ynyl-amine* | **62** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 447 Retention time: 2.91 min. | 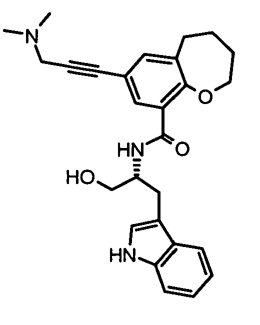 |
| **65** | 7-[3-(1,3-Dimethyl-ureido)-prop-1-ynyl]-2,3,4,5-tetrahydro-benzo[b]oxepine-9-carboxylic acid [(R)-1-hydroxymethyl-2-(1 H-indol-3-yl)-ethyl]-amide; *7-Bromo-2,3,4,5-tetrahydro-benzo[b]- oxepine-9-carboxylic acid [(R)-1-hydroxy-methyl-2-(1H-indol-3-yl)-ethyl]-amide* *and* *1, 3-Dimethyl-1-prop-2-ynyl-urea* | **62** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 490 Retention time: 3.22 min. | 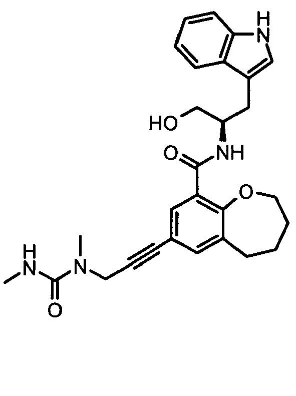 |
| **66** | 7-(4-Amino-but-1- ynyl)-2,3,4,5-tetrahydro-benzo[b]-oxepine-9-carboxylic acid [(R)-1-hydroxy-methyl-2-(1 H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *But-3-ynylamine* | **62** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 433 Retention time: 2.92 min. | 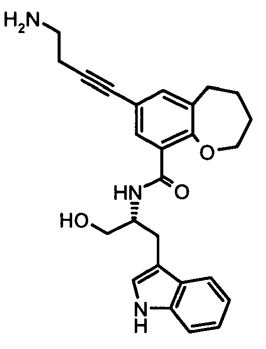 |
| **67** | 6-{9-[(R)-1-Hydroxy-methyl-2-(1H-indol-3-yl)-ethylcarbamoyl]-2,3,4,5-tetrahydro-benzo[b]oxepin-7-yl}-hex-5-ynoic acid; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Hex-5-ynoic acid* | **62** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1 % (1.75'). Molecular peak (ESI, M+1): 476 Retention time: 3.61 min. | 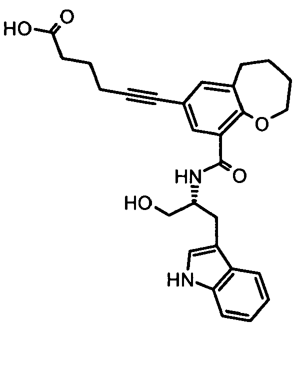 |
| **68** | 6-{9-[(R)-1-Hydroxymethyl-2-(1H-indol-3-yl)-ethylcarba-moyl]-2,3,4,5-tetra-hydro-benzo[b]oxepin-7-yl}-hex-5-ynoic acid methyl ester; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Hex-5-ynoic acid methyl ester* | **62** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 490 Retention time: 4.07 min. | 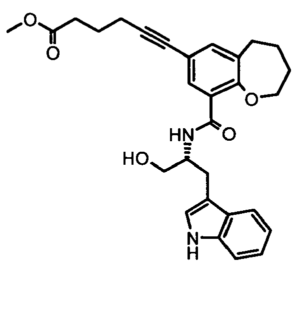 |
| **69** | 7-(6-Hydroxy-hex-1-ynyl)-2,3,4,5-tetra-hydro-benzo[b]-oxepine-9-carboxylic acid [(R)-1-hydroxy-methyl-2-(1 H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Hex-5-yn-1-ol* | **62** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 462 Retention time: 5.12 min. | 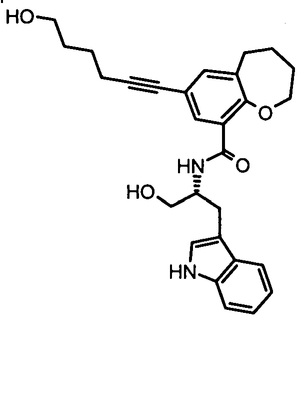 |
| **70** | 7-(5-Cyclohexyl-pent-1-ynyl)-2,3,4,5-tetra-hydro-benzo[b]-oxepine-9-carboxylic acid [(R)-1-hydroxy-methyl-2-(1 H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Pent-4-ynyl-cyclo-hexane* | **62** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 514 Retention time: 5.63 min. | 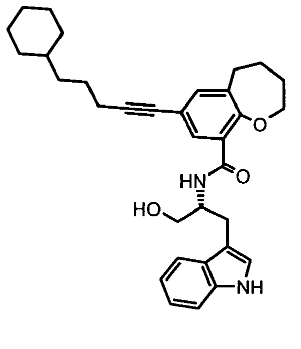 |
| **71** | 7-(5-Cyano-pent-1-y- nyl)-2,3,4,5-tetrahydro-benzo[b]oxepine-9-carboxylic acid [(R)-1-hydroxymethyl-2-(1 H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Hex-5-ynenitrile* | **62** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1% to 99% (5') to 99% (1') to 1% (0.25') to 1% (1.75'). Molecular peak (ESI, M+1): 457 Retention time: 5.28 min. | 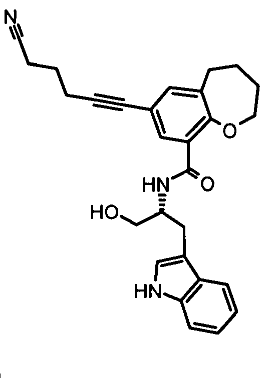 |
| **72** | 7-(4-Hydroxy-but-1-ynyl)-2,3,4,5-tetrahydro-benzo[b]oxepine-9-carboxylic acid [(R)-1-hydroxymethyl-2-(1 H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide and But-3-yn-1-ol* | **62** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1 % (1.75'). Molecular peak (ESI, M+1): 434 Retention time: 3.42 min. | 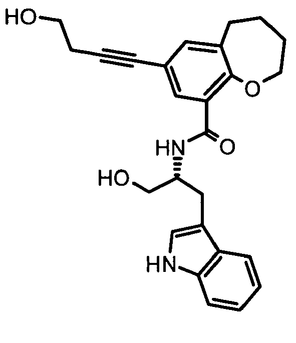 |
| **73** | 7-{3-[((1R,2R)-2-Hydroxy-1-methyl-propyl)-methyl-amino]-prop-1-ynyl}-2,3,4,5-tetrahydro-benzo[b]-oxepine-9-carboxylic acid [(R)-1-hydroxy-methyl-2-(1H-indol-3-yl)-ethyl]-amide; *7-Bromo-2,3,4,5-tetrahydro-benzo[b]-oxepine-9-carboxylic acid [(R)-1-hydroxy₋ methyl-2-(1H-indol-3-yl)-ethyl]-amide* *and* *(2R, 3R)-3-(Methyl-prop-2-ynyl-amino)-butan-2-ol* | **62** | HPLC purification: Column X-Bridge RP C18 4.6 x 50 3.5µM; detection wavelength 214 nm; flow rate 2 ml/min; eluents A: 0.1% TFA in H₂O, B 0.1 % TFA in ACN; gradient in each case based on B: 1 % to 99% (5') to 99% (1') to 1 % (0.25') to 1 % (1.75'). Molecular peak (ESI, M+1): 505 Retention time: 3.00 min. | 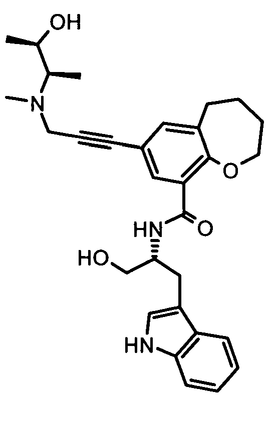 |
| **74** | 7-[3-(3-Methyl-ureido)-prop-1-ynyl]-2,3,4,5-tetrahydro-benzo[b]-oxepine-9-carboxylic acid [(R)-1-hydroxy-methyl-2-(1 H-indol-3-yl)-ethyl]-amide; *7-Bromo-2,3,4,5-tetra-* on *hydro-benzo[b]-oxepine-9-carboxylic acid [(R)-1-hydroxy-methyl-2-(1H-indol-3-yl)-ethyl]-amide* *and* *1-Methyl-3-prop-2-ynyl-urea* | **62** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based B: 1% to 99% (5') to 99% (1') to 1% (0.25') to 1% (1.75'). Molecular peak (ESI, M+1): 476 Retention time: 3.25 min. | 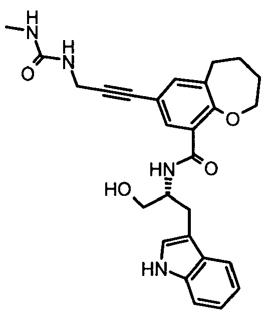 |
| **75** | 7-[4-(3-Methyl-ureido)-but-1-ynyl]-2,3,4,5-tetrahydro-benzo[b]-oxepine-9-carboxylic acid [(R)-1-hydroxy-methyl-2-(1 H-indol-3-yl)-ethyl]-amide; *7-Bromo-2,3,4,5-tetra-hydro-benzo[b]-oxepine-9-carboxylic acid [(R)-1-hydroxy-methyl-2-(1H-indol-3-yl)-ethyl]-amide* *and* *1-But-3-ynyl-3-methyl-urea* | **62** | HPLC purification: Column X-Bridge RP C18 4.6 x 50 3.5µM; detection wavelength 214 nm; flow rate 2 ml/min; eluents A: 0.1 % TFA in H₂O, B 0.1% TFA in ACN; gradient in each case based on B: 1% to 99% (5') to 99% (1') to 1% (0.25') to 1% (1.75'). Molecular peak (ESI, M+1): 490 Retention time: 3.33 min. | 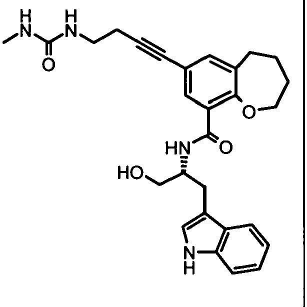 |
| **76** | 7-(4-Methylcarbamoyl-but-1-ynyl)-2,3,4,5-tetrahydro-benzo[b]-oxepine-9-carboxylic acid [(R)-1-hydroxy-methyl-2-(1H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Pent-4-ynoic acid me-thylamide* | **62** | HPLC purification: Column X-Bridge RP C18 4.6 x 50 3.5µM; detection wavelength 214 nm; flow rate 2 ml/min; eluents A: 0.1% TFA in H₂O, B 0.1% TFA in ACN; gradient in each case based on B: 1% to 99% (5') to 99% (1') to 1% (0.25') to 1% (1.75'). Molecular peak (ESI, M+1): 475 Retention time: 3.50 min. | 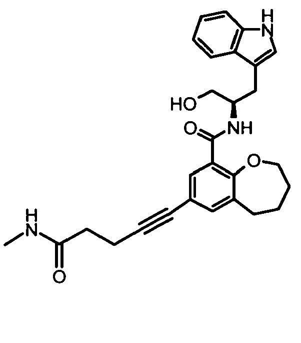 |
| **77** | 7-(5-Methylcarbamoyl-pent-1-ynyl)-2,3,4,5-tetrahydro-benzo[b]-oxepine-9-carboxylic acid [(R)-1-hydroxy-methyl-2-(1 H-indol-3-yl)-ethyl]-amide; *7-Bromo-2,3,4,5-tetrahydro-benzo[b]-oxepine-9-carboxylic acid [(R)-1-hydroxy-methyl-2-(1H-indol-3-yl)-ethyl]-amide* *and* *Hex-5-ynoic acid me-thylamide* | **62** | HPLC purification: Column X-Bridge RP C18 4.6 x 50 3.5µM; detection wavelength 214 nm; flow rate 2 ml/min; eluents A: 0.1 % TFA in H₂O, B 0.1 % TFA in ACN; gradient in each case based on B: 1% to 99% (5') to 99% (1') to 1% (0.25') to 1% (1.75'). Molecular peak (ESI, M+1): 489 Retention time: 4.93 min. | 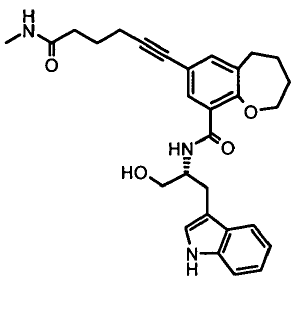 |

## Claims

1. Compounds of the formula I 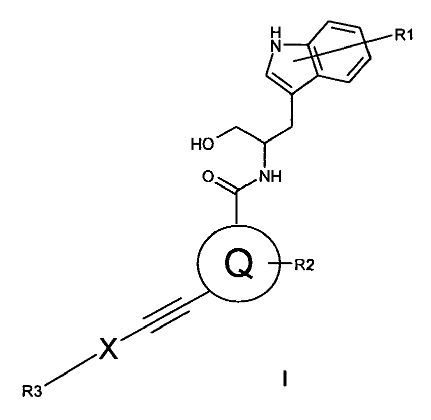 in which
R1 is hydrogen, halogen, cyano, -SO₂Me, C₁-C₆-alkyl, C₂-C₆-alkenyl, C₂-C₆-alkynyl or C₁-C₆-alkyloxy,
where the hydrocarbon chains therein may optionally be substituted one or more times by fluorine and/or cyano;
R2 is hydrogen, halogen, nitro, amino, cyano, C₁-C₆-alkyl, C₂-C₆-alkenyl or C₂-C₆-alkynyl, C₃-C₇-cycloalkyl, hydroxy-C₁-C₆-alkylene, hydroxy-C₃-C₆-alkenylene, hydroxy-C₃-C₆-alkynylene, C₁-C₆-alkyloxy, C₁-C₆-alkyloxy-C₁-C₆-alkylene, C₃-C₇-cycloalkyloxy, C₃-C₇-cycloalkyl-C₁-C₆-alkylenoxy, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkyloxyphenyl-C₁-C₆-alkylene, C₁-C₆-alkylamino-C₁-C₆-alkylene, di(C₁-C₆-alkyl)amino-C₁-C₆-alkylene, phenyloxy-C₁-C₆-alkylene;
where the hydrocarbon chains therein may optionally be substituted one or more times by fluorine, cyano or hydroxy;
R3 is hydroxy, amino, cyano, C₁-C₆-alkyl, C₂-C₆-alkenyl or C₂-C₆-alkynyl, C₃-C₇-cycloalkyl, C₃-C₇-cycloalkenyl, C₃-C₇-heterocycloalkyl,
where the hydrocarbon chains therein may optionally be substituted one or more times by fluorine or cyano
or hydroxy-C₁-C₆-alkylene, hydroxy-C₃-C₆-alkenylene, hydroxy-C₃-C₆-alkynylene, C₁-C₆-alkyloxy, C₃-C₇-cycloalkyloxy, C₃-C₇-cydoalkyl-C₁-C₆-alkylenoxy, C₁-C₆-alkyloxy-C₁-C₆-alkylene, C₃-C₇-cycloalkyloxy-C₁-C₆-alkylene, C₁-C₆-alkyloxy-C₃-C₆-alkenylene, C₁-C₆-alkyloxy-C₃-C₆-alkynylene, C₁-C₆-alkylamino, di(C₁-C₆-alkyl)amino, C₁-C₆-alkylamino-C₁-C₆-alkylene, di(C₁-C₆)-alkylamino-C₁-C₆-alkylene, C₃-C₇-cycloalkyl-(C₀-C₆-alkyl)amino, C₁-C₆-acyl-(C₀-C₆-alkyl)amido, C₁-C₆-alkylaminocarbonyl, di(C₁-C₆-alkyl)aminocarbonyl, (C₃-C₇-cycloalkyl)aminocarbonyl, di(C₃-C₇-cycloalkyl)aminocarbonyl, C₃-C₇-cycloalkyl-C₁-C₆-alkyleneamino-carbonyl, C₁-C₆-alkylcarbonyl, C₃-C₇-cycloalkylcarbonyl, C₁-C₆-acyloxy, carboxy, carboxamido [-C(O)NH₂], C₁-C₆-alkyloxycarbonyl, C₁-C₃-alkylsulphanyl, C₁-C₆-alkysulphonyl, C₃-C₇-cycloalkylsulphonyl, C₃-C₇-cycloalkyl-C₁-C₆-alkylenesulphonyl, C₁-C₆-alkylaminosulphonyl, di(C₁-C₆-alkyl)aminosulphonyl, (C₃-C₇-cycloalkyl)aminosulphonyl, di(C₃-C₇-cycloalkyl)aminosulphonyl, C₃-C₇-cycloalkyl-C₁-C₆-alkyleneaminosulphonyl, C₁-C₆-alkylsulphonylamido, -N(C₀-C₆-alkyl)-C(O)-C₁-C₆-alkyl, -N(C₀-C₆-alkyl)-C(O)-C₃-C₇-cycloalkyl, -N(C₀-C₆-alkyl)-C(O)-N-di(C₀-C₆-alkyl), -N(C₀-C₆-alkyl)-C(O)-O-(C₀-C₆)alkyl, -N(C₀-C₆-alkyl)-C(O)-NH-C₃-C₇-cycloalkyl, -N(C₀-C₆-alkyl)-SO₂-C₁-C₆-alkyl, -N(C₀-C₆-alkyl)-SO₂-C₃-C₇-cycloalkyl, -N(C₀-C₆-alkyl)-SO₂-N-di(C₀-C₆-alkyl), -N(C₀-C₆-alkyl)-SO₂-NH-(C₃-C₇)-cycloalkyl, -C(O)-N(H)-C₂-C₆-alkylene-(C₁-C₆-alkyl)amine, -C(O)-N(H)-C₂-C₆-alkylene-[di(C₁-C₆-alkyl)]amine, -C(O)-N(H)-C₂-C₆-alkylene-(C₃-C₇-cycloalkyl)amine, -C(O)-N(H)-C₂-C₆-alkylene-(C₃-C₇-cycloalkyl-C₁-C₆-alkyl)amine, -S(O₂)-N(H)-C₂-C₆-alkylene-(C₁-C₆-alkyl)amine, -S(O₂)-N(H)-C₂-C₆-alkylene-[di(C₁-C₆-alkyl)]amine, -S(O₂)-N(H)-C₂-C₆-alkylene-(C₃-C₇-cycloalkyl)amine, -S(O₂)-N(H)-C₂-C₆-alkylene-(C₃-C₇-cycloalkyl-C₁-C₆-alkylene)amine, -O-C₂-C₆-alkylene-(C₁-C₆-alkyl)amine, -O-C₂-C₆-alkylene-[di(C₁-C₆-alkylene)]amine,
or the radicals: 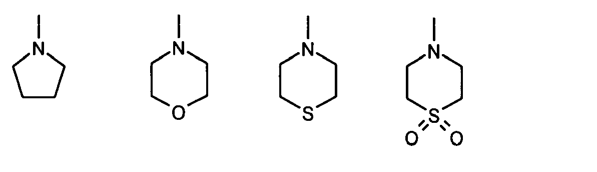 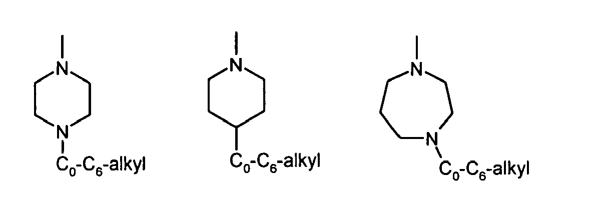 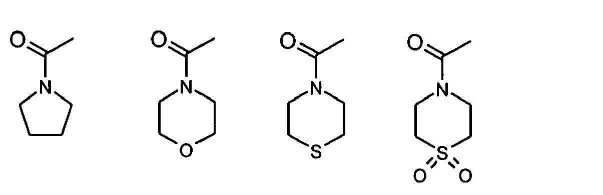 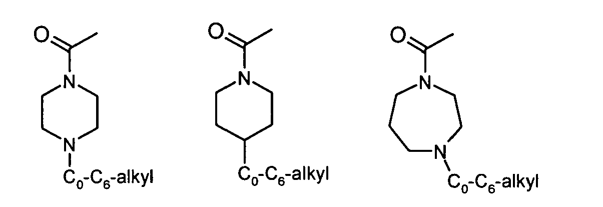 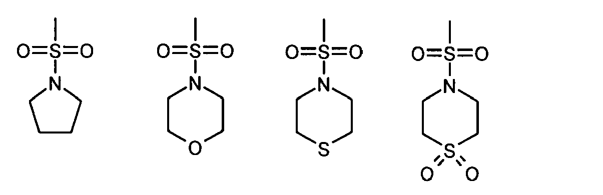 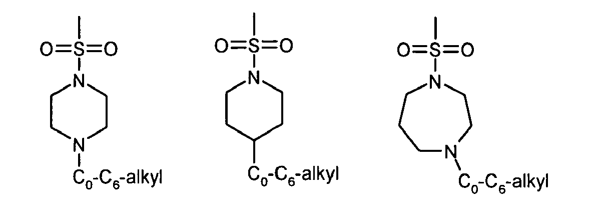 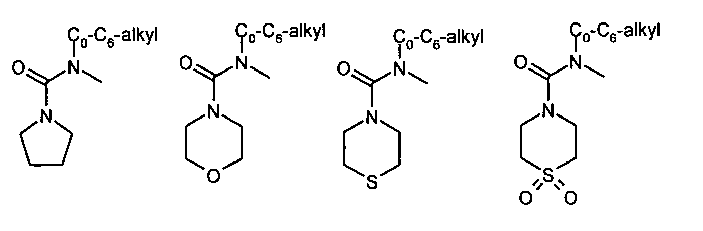 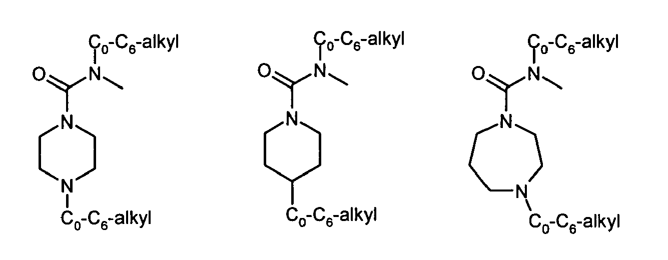 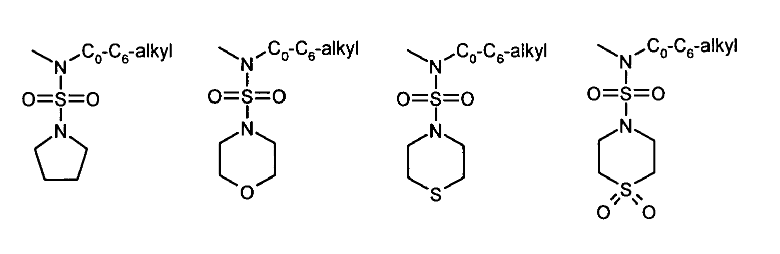 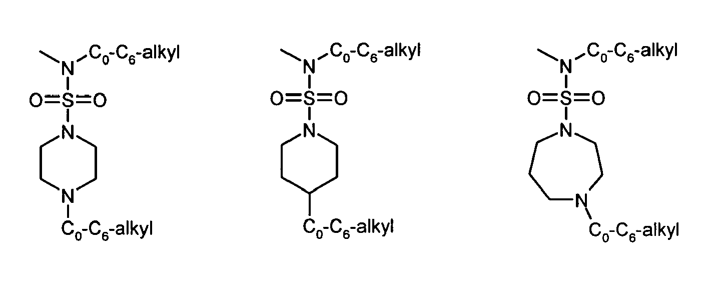 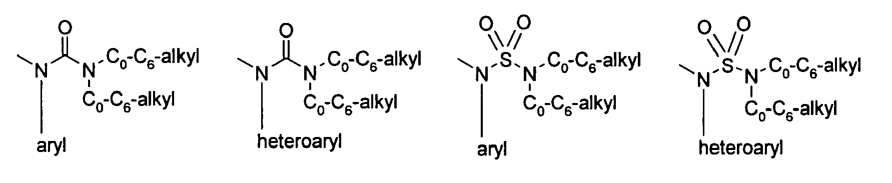 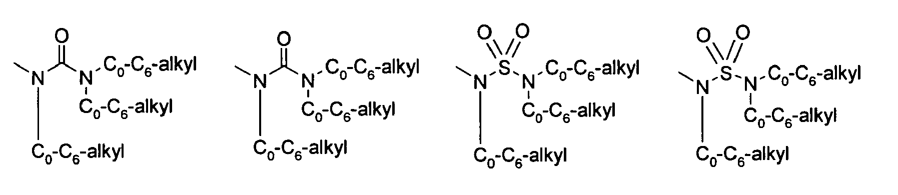 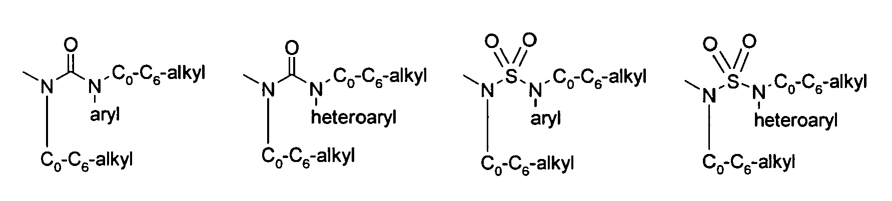 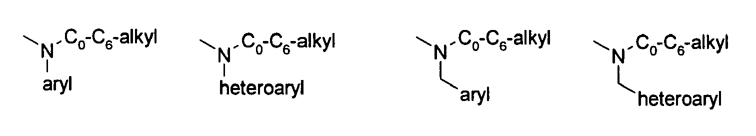 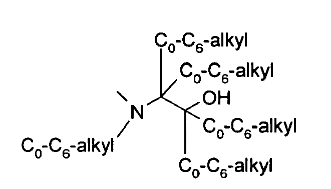 in which the aryl or heteroarylgroup may optionally be substituted with halogen, cyano, SO₂Me, C₁-C₆-alkyl, C₂-C₆-alkenyl, C₂-C₆-alkynyl or C₁-C₆-alkyloxy, where the hydrocarbon chains therein may optionally be substituted one or more times by fluorine and/or cyano;
and
X is a bond, C₁-C₄-alkylene, C₂-C₄-alkenylene or C₂-C₄-alkynylene;
Q is an aryl or heteroaryl group,
or the group AV 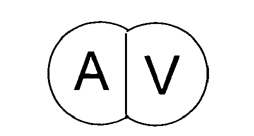
in which
A is a monocyclic aryl or a monocyclic heteroaryl group;
V is a cycloalkylen, cycloalkenylen, heterocycloalkylen or heterocycloalkenylen group;
where
R1 substitutes one or more positions of the aryl or heteroaryl ring in the indole residue;
R2 substitutes one or more positions of the aryl or heteroaryl ring in the radicals Q, A or V;
R3 substitutes one or more positions of the group X.

2. Compounds according to claim 1, namely acyltryptophanols of the formula II 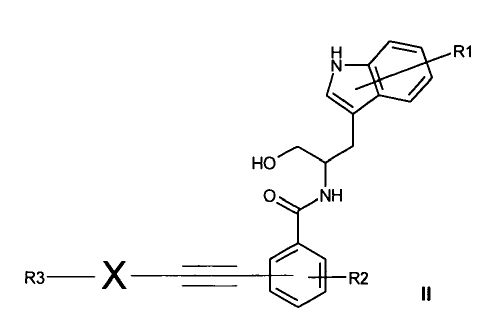 in which
R1, R2, R3 and X have the same meaning as defined in claim 1.

3. Compounds according to claim 1, namely acyltryptophanols of the formula III 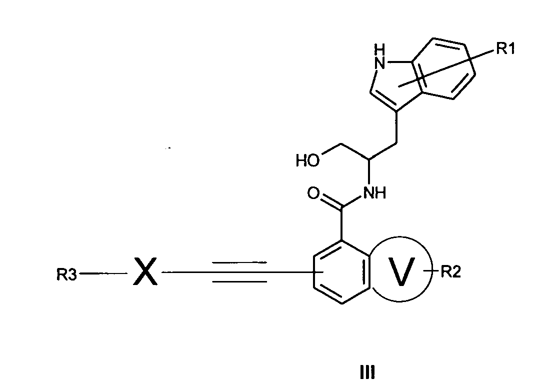 in which
R1, R2, R3, X and V have the same meaning as defined in claim 1.

4. Compounds according to claims 1 or 2, namely acyltryptophanols of the formula IV 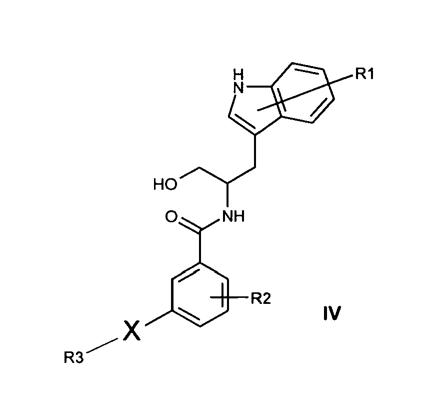 in which the radicals R1 to R3 and X have the same meaning as defined in claim 1.

5. Compounds according to claims 1 or 3, namely acyltryptophanols of the formula V 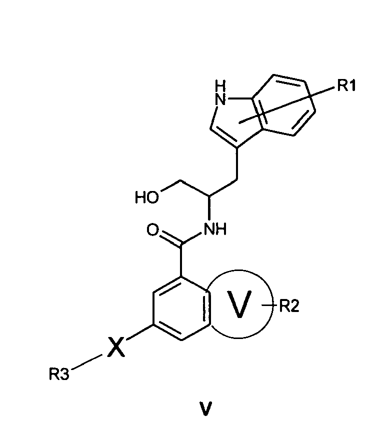 in which the radicals R1 to R3, X and V have the same meaning as defined in claim 1.

6. Compounds according to any of the preceding claims, namely
**1** 5-(3-Cyclopentyl-prop-1-ynyl)-N-[(R)-1-hydroxymethyl-2-(1H-indol-3-yl)-ethyl]-2-isopropoxy-benzamide;
**2** 5-Cyclopentylethynyl-N-[(R)-1-hydroxymethyl-2-(1H-indol-3-yl)-ethyl]-2-isopropoxy-benzamide;
**3** 5-(3,3-Dimethyl-but-1-ynyl)-N-[(R)-1-hydroxymethyl-2-(1H-indol-3-yl)-ethyl]-2-isopropoxy-benzamide;
**4** 5-(4-Hydroxy-but-1-ynyl)-N-[(R)-1-hydroxymethyl-2-(1H-indol-3-yl)-ethyl]-2-isopropoxy-benzamide;
**5** 5-[3-(1,1-Dioxo-1lambda*6*-thiomorpholin-4-yl)-prop-1-ynyl]-N-[(R)-2-hydroxy-1-(1H-indol-3-ylmethyl)-ethyl]-2-isopropoxy-benzamide;
**6** 6-{3-[(R)-1-Hydroxymethyl-2-(1H-indol-3-yl)-ethylcarbamoyl]-4-isopropoxy-phenyl}-hex-5-ynoic acid methyl ester;
**7** N-[(R)-1-Hydroxymethyl-2-(1H-indol-3-yl)-ethyl]-2-isopropoxy-5-[3-(3-p-tolyl-ureido)-prop-1-ynyl]-benzamide;
**8** N-[(R)-1-Hydroxymethyl-2-(1H-indol-3-yl)-ethyl]-5-(5-hydroxy-pent-1-ynyl)-2-isopropoxy-benzamide;
**9** 5-(5-Cyano-pent-1-ynyl)-N-[(R)-1-hydroxymethyl-2-(1H-indol-3-yl)-ethyl]-2-isopropoxy-benzamide;
**10** 5-[3-(1,3-Dimethyl-ureido)-prop-1-ynyl]-N-[(R)-1-hydroxymethyl-2-(1H-indol-3-yl)-ethyl]-2-isopropoxy-benzamide;
**11** N-[2-(5-Fluoro-1H-indol-3-yl)-1-hydroxymethyl-ethyl]-5-(3-hydroxy-prop-1-ynyl)-2-propoxy-benzamide;
**12** N-[2-(5-Fluoro-1H-indol-3-yl)-1-hydroxymethyl-ethyl]-5-(4-hydroxy-pent-1-ynyl)-2-propoxy-benzamide;
**13** 5-(3-Dimethylamino-prop-1-ynyl)-N-[2-(5-fluoro-1H-indol-3-yl)-1-hydroxymethyl-ethyl]-2-propoxy-benzamide;
**14** 5-(5-Cyano-pent-1-ynyl)-N-[2-(5-fluoro-1H-indol-3-yl)-1-hydroxymethyl-ethyl]-2-propoxy-benzamide;
**15** N-[2-(5-Fluoro-1H-indol-3-yl)-1-hydroxymethyl-ethyl]-5-(5-hydroxy-pent-1-ynyl)-2-propoxy-benzamide;
**16** N-[2-(5-Fluoro-1 H-indol-3-yl)-1-hydroxymethyl-ethyl]-5-(4-hydroxy-but-1-ynyl)-2-propoxy-benzamide;
**17** N-[2-(5-Fluoro-1H-indol-3-yl)-1-hydroxymethyl-ethyl]-5-{3-[((1R,2R)-2-hydroxy-1-methyl-propyl)-methyl-amino]-prop-1-ynyl}-2-propoxy-benzamide;
**18** 5-[4-(1,3-Dimethyl-ureido)-but-1-ynyl]-N-[2-(5-fluoro-1H-indol-3-yl)-1-hydroxymethyl-ethyl]-2-propoxy-benzamide;
**19** N-[2-(5-Fluoro-1 H-indol-3-yl)-1-hydroxymethyl-ethyl]-5-(4-methylcarbamoyl-but-1-ynyl)-2-propoxy-benzamide;
**20** N-[2-(5-Fluoro-1H-indol-3-yl)-1-hydroxymethyl-ethyl]-5-(5-methylcarbamoyl-pent-1-ynyl)-2-propoxy-benzamide;
**21** N-[(R)-1-Hydroxymethyl-2-(1H-indol-3-yl)-ethyl]-5-(3-hydroxy-prop-1-ynyl)-2-isopropoxy-benzamide;
**22** N-[(R)-1-Hydroxymethyl-2-(1H-indol-3-yl)-ethyl]-5-(4-hydroxy-pent-1-ynyl)-2-isopropoxy-benzamide;
**23** 5-[3-(Benzyl-methyl-amino)-prop-1-ynyl]-N-[(R)-1-hydroxymethyl-2-(1H-indol-3-yl)-ethyl]-2-isopropoxy-benzamide;
**24** 5-(3-Dimethylamino-prop-1-ynyl)-N-[(R)-1-hydroxymethyl-2-(1H-indol-3-yl)-ethyl]-2-isopropoxy-benzamide;
**25** 5-[3-(1,3-Dimethyl-ureido)-prop-1-ynyl]-N-[(R)-1-hydroxymethyl-2-(1H-indol-3-yl)-ethyl]-2-isopropoxy-benzamide;
**26** 6-{3-[(R)-1-Hydroxymethyl-2-(1H-indol-3-yl)-ethylcarbamoyl]-4-isopropoxy-phenyl}-hex-5-ynoic acid methyl ester;
**27** 5-(6-Hydroxy-hex-1-ynyl)-N-[(R)-1-hydroxymethyl-2-(1H-indol-3-yl)-ethyl]-2-isopropoxy-benzamide;
**28** 5-(5-Cyano-pent-1-ynyl)-N-[(R)-1-hydroxymethyl-2-(1H-indol-3-yl)-ethyl]-2-isopropoxy-benzamide;
**29** N-[(R)-1-Hydroxymethyl-2-(1H-indol-3-yl)-ethyl]-5-(5-hydroxy-pent-1-ynyl)-2-isopropoxy-benzamide;
**30** 5-(4-Hydroxy-but-1-ynyl)-N-[(R)-1-hydroxymethyl-2-(1H-indol-3-yl)-ethyl]-2-isopropoxy-benzamide;
**31** Acetic acid 3-{3-[(R)-1-hydroxymethyl-2-(1H-indol-3-yl)-ethylcarbamoyl]-4-isopropoxy-phenyl}-prop-2-ynyl ester;
**32** N-[(R)-1-Hydroxymethyl-2-(1H-indol-3-yl)-ethyl]-5-{3-[((1R,2R)-2-hydroxy-1-methyl-propyl)-methyl-amino]-prop-1-ynyl}-2-isopropoxy-benzamide;
**33** 9-{3-[(R)-1-Hydroxymethyl-2-(1H-indol-3-yl)-ethylcarbamoyl]-4-isopropoxy-phenyl}-non-8-ynoic acid;
**34** 5-[4-(1,3-Dimethyl-ureido)-but-1-ynyl]-N-[(R)-1-hydroxymethyl-2-(1H-indol-3-yl)-ethyl]-2-isopropoxy-benzamide;
**35** N-[(R)-1-Hydroxymethyl-2-(1H-indol-3-yl)-ethyl]-2-isopropoxy-5-(4-methylcarbamoyl-but-1-ynyl)-benzamide;
**36** N-[(R)-1-Hydroxymethyl-2-(1H-indol-3-yl)-ethyl]-2-isopropoxy-5-(5-methylcarbamoyl-pent-1-ynyl)-benzamide;
**37** N-[(R)-1-Hydroxymethyl-2-(1H-indol-3-yl)-ethyl]-3-(3-hydroxy-prop-1-ynyl)-benzamide;
**38** N-[(R)-1-Hydroxymethyl-2-(1H-indol-3-yl)-ethyl]-3-(3-hydroxy-prop-1-ynyl)-benzamide;
**39** 3-[3-(Benzyl-methyl-amino)-prop-1-ynyl]-N-[(R)-1-hydroxymethyl-2-(1H-indol-3-yl)-ethyl]-benzamide;
**40** 3-(3-Dimethylamino-prop-1-ynyl)-N-[(R)-1-hydroxymethyl-2-(1H-indol-3-yl)-ethyl]-benzamide;
**41** 3-[3-(1,3-Dimethyl-ureido)-prop-1-ynyl]-N-[(R)-1-hydroxymethyl-2-(1H-indol-3-yl)-ethyl]-benzamide;
**42** 6-{3-[(R)-1-Hydroxymethyl-2-(1H-indol-3-yl)-ethylcarbamoyl]-phenyl}-hex-5-ynoic acid;
**43** 6-{3-[(R)-1-Hydroxymethyl-2-(1H-indol-3-yl)-ethylcarbamoyl]-phenyl}-hex-5-ynoic acid; methyl ester;
**44** 3-(6-Hydroxy-hex-1-ynyl)-N-[(R)-1-hydroxymethyl-2-(1H-indol-3-yl)-ethyl]-benzamide;
**45** 3-(5-Cyclohexyl-pent-1-ynyl)-N-[(R)-1-hydroxymethyl-2-(1H-indol-3-yl)-ethyl]-benzamide;
**46** 3-(5-Cyano-pent-1-ynyl)-N-[(R)-1-hydroxymethyl-2-(1H-indol-3-yl)-ethyl]-benzamide;
**47** N-[(R)-1-Hydroxymethyl-2-(1H-indol-3-yl)-ethyl]-3-(5-hydroxy-pent-1- ynyl)-benzamide;
**48** 3-(4-Hydroxy-but-1-ynyl)-N-[(R)-1-hydroxymethyl-2-(1H-indol-3-yl)-ethyl]-benzamide;
**49** N-[(R)-1-Hydroxymethyl-2-(1H-indol-3-yl)-ethyl]-3-{3-[((1R,2R)-2-hydroxy-1-methyl-propyl)-methyl-amino]-prop-1-ynyl}-benzamide;
**50** N-[(R)-1-Hydroxymethyl-2-(1H-indol-3-yl)-ethyl]-3-[3-(3-methyl-ureido)-prop-1-ynyl]-benzamide;
**51** 9-{3-[(R)-1-Hydroxymethyl-2-(1H-indol-3-yl)-ethylcarbamoyl]-phenyl}-non-8-ynoic acid;
**52** 3-[4-(1,3-Dimethyl-ureido)-but-1-ynyl]-N-[(R)-1-hydroxymethyl-2-(1H-indol-3-yl)-ethyl]-benzamide;
**53** N-[(R)-1-Hydroxymethyl-2-(1H-indol-3-yl)-ethyl]-3-[4-(3-methyl-ureido)-but-1-ynyl]-benzamide;
**54** N-[(R)-1-Hydroxymethyl-2-(1H-indol-3-yl)-ethyl]-3-(4-methylcarbamoyl-but-1-ynyl)-benzamide;
**55** N-[(R)-1-Hydroxymethyl-2-(1H-indol-3-yl)-ethyl]-3-(5-methylcarbamoyl-pent-1-ynyl)-benzamide;
**56** 5-[3-(Benzyl-methyl-amino)-prop-1-ynyl]-2-bromo-N-[(R)-1-hydroxymethyl-2-(1H-indol-3-yl)-ethyl]-benzamide;
**57** 2-Bromo-5-(3-dimethylamino-prop-1-ynyl)-N-[(R)-1-hydroxymethyl-2-(1 H-indol-3-yl)-ethyl]-benzamide;
**58** 2-Bromo-5-[3-(1,3-dimethyl-ureido)-prop-1-ynyl]-N-[(R)-2-hydroxy-1-(1 H-indol-3-ylmethyl)-ethyl]-benzamide;
**59** 2-Bromo-5-(5-cyano-pent-1-ynyl)-N-[(R)-1-hydroxymethyl-2-(1H-indol-3-yl)-ethyl]-benzamide;
**60** 2-Bromo-N-[(R)-1-hydroxymethyl-2-(1H-indol-3-yl)-ethyl]-5-(5-methylcarbamoyl-pent-1-ynyl)-benzamide;
**61** 7-(3-Hydroxy-prop-1-ynyl)-2,3,4,5-tetrahydro-benzo[b]oxepine-9-carboxylic acid [(R)-1-hydroxymethyl-2-(1H-indol-3-yl)-ethyl]-amide;
**62** 7-(4-Hydroxy-pent-1-ynyl)-2,3,4,5-tetrahydro-benzo[b]oxepine-9-carboxylic acid [(R)-1-hydroxymethyl-2-(1H-indol-3-yl)-ethyl]-amide;
**63** 7-[3-(Benzyl-methyl-amino)-prop-1-ynyl]-2,3,4,5-tetrahydro-benzo[b]oxepine-9-carboxylic acid [(R)-1-hydroxymethyl-2-(1H-indol-3-yl)-ethyl]-amide;
**64** 7-(3-Dimethylamino-prop-1-ynyl)-2,3,4,5-tetrahydro-benzo[b]oxepine-9-carboxylic acid [(R)-1-hydroxymethyl-2-(1H-indol-3-yl)-ethyl]-amide;
**65** 7-[3-(1,3-Dimethyl-ureido)-prop-1-ynyl]-2,3,4,5-tetrahydro-benzo[b]oxepine-9-carboxylic acid [(R)-1-hydroxymethyl-2-(1H-indol-3-yl)-ethyl]-amide;
**66** 7-(4-Amino-but-1-ynyl)-2,3,4,5-tetrahydro-benzo[b]oxepine-9-carboxylic acid [(R)-1-hydroxymethyl-2-(1H-indol-3-yl)-ethyl]-amide;
**67** 6-{9-[(R)-1-Hydroxymethyl-2-(1H-indol-3-yl)-ethylcarbamoyl]-2,3,4,5-tetrahydro-benzo[b]oxepin-7-yl}-hex-5-ynoic acid;
**68** 6-{9-[(R)-1-Hydroxymethyl-2-(1H-indol-3-yl)-ethylcarbamoyl]-2,3,4,5-tetrahydro-benzo[b]oxepin-7-yl}-hex-5-ynoic acid methyl ester;
**69** 7-(6-Hydroxy-hex-1-ynyl)-2,3,4,5-tetrahydro-benzo[b]oxepine-9-carboxylic acid [(R)-1-hydroxymethyl-2-(1H-indol-3-yl)-ethyl]-amide;
**70** 7-(5-Cyclohexyl-pent-1-ynyl)-2,3,4,5-tetrahydro-benzo[b]oxepine-9-carboxylic acid [(R)-1-hydroxymethyl-2-(1H-indol-3-yl)-ethyl]-amide;
**71** 7-(5-Cyano-pent-1-ynyl)-2,3,4,5-tetrahydro-benzo[b]oxepine-9-carboxylic acid [(R)-1-hydroxymethyl-2-(1H-indol-3-yl)-ethyl]-amide;
**72** 7-(4-Hydroxy-but-1-ynyl)-2,3,4,5-tetrahydro-benzo[b]oxepine-9-carboxylic acid [(R)-1-hydroxymethyl-2-(1H-indol-3-yl)-ethyl]-amide;
**73** 7-{3-[((1R,2R)-2-Hydroxy-1-methyl-propyl)-methyl-amino]-prop-1-ynyl}-2,3,4,5-tetrahydro-benzo[b]oxepine-9-carboxylic acid [(R)-1- hydroxymethyl-2-(1H-indol-3-yl)-ethyl]-amide;
**74** 7-[3-(3-Methyl-ureido)-prop-1-ynyl]-2,3,4,5-tetrahydro-benzo[b]oxepine-9-carboxylic acid [(R)-1-hydroxymethyl-2-(1H-indol-3-yl)-ethyl]-amide;
**75** 7-[4-(3-Methyl-ureido)-but-1-ynyl]-2,3,4,5-tetrahydro-benzo[b]oxepine-9-carboxylic acid [(R)-1-hydroxymethyl-2-(1H-indol-3-yl)-ethyl]-amide;
**76** 7-(4-Methylcarbamoyl-but-1-ynyl)-2,3,4,5-tetrahydro-benzo[b]oxepine-9-carboxylic acid [(R)-1-hydroxymethyl-2-(1H-indol-3-yl)-ethyl]-amide;
**77** 7-(5-Methylcarbamoyl-pent-1-ynyl)-2,3,4,5-tetrahydro-benzo[b]oxepine-9-carboxylic acid [(R)-1-hydroxymethyl-2-(1H-indol-3-yl)-ethyl]-amide.

7. Process for preparing compounds of the formula I of claim 1, wherein a tryptophanol derivative of the formula VI 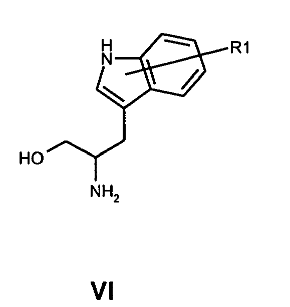 in which the radical R1 has the same meaning as defined in claim 1,
is coupled with a carboxylic acid of the formula VII 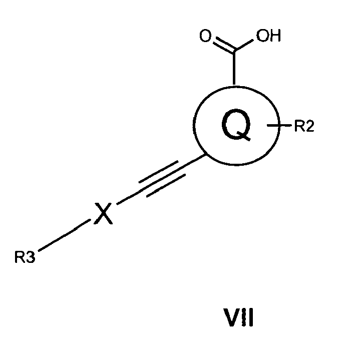 in which R2, R3, Q and X have the same meaning as defined in claim 1,
in an amide forming reaction comprising
a) conversion of said carboxylic acids into an intermediate active ester or carbonyl chloride with a suitable peptide-coupling reagent, or with thionyl chloride, oxalyl chloride, phosgene or derivatives thereof, where appropriate in the presence of a base,
b) reacting the active intermediate resulting from step a) with said tryptophanol.

8. Process according to claim 7 for preparing compounds of the formula II of claim 2, wherein a tryptophanol derivative of the formula VI is coupled with a carboxylic acid of the formula VIII 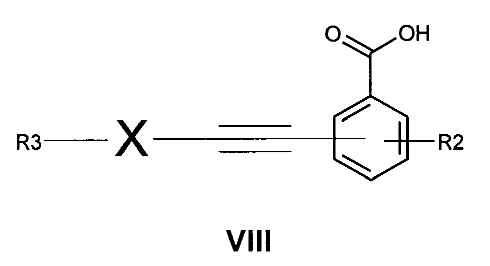 in which R2, R3 and X have the same meaning as defined in claim 1.

9. Process according to claim 7 for preparing compounds of the formula III of claim 3, wherein a tryptophanol derivative of the formula VI is coupled with a carboxylic acid of the formula IX 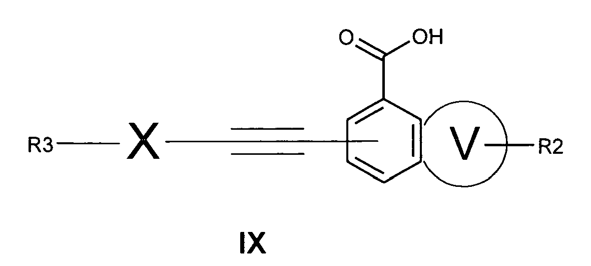 In which R2, R3, X and V have the same meaning as defined in claim 1.

10. Process according to claim 7 for preparing compounds of the formula IV of claim 4, wherein a tryptophanol derivative of the formula VI is coupled with a carboxylic acid of the formula X 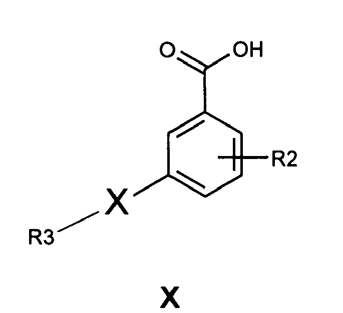 in which R2, R3 and X have the same meaning as defined in claim 1.

11. Process according to claim 7 for preparing compounds of the formula V of claim 5, wherein a tryptophanol derivative of the formula VI is coupled with a carboxylic acid of the formula XI 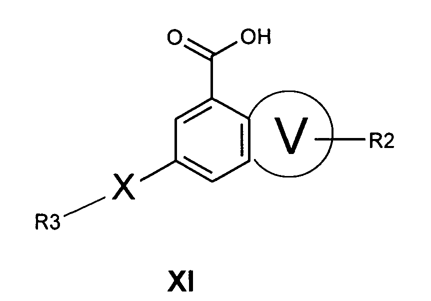 In which R2, R3, X and V have the same meaning as defined in claim 1.

12. Process for preparing compounds of the formula I of claim 1, wherein the acetylene derivative of the formula XII 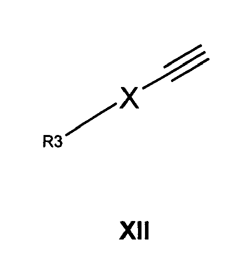 in which R3 and X have the same meaning as defined in claim 1,
is coupled in a Sonogoshira type coupling reaction
in the presence of a palladium-catalyst
with an aryl halide of the formula XIII 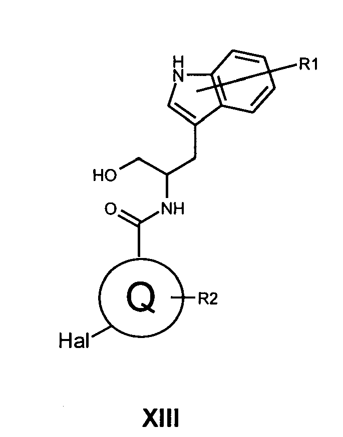 in which R1, R2 and Q have the same meaning as defined in claim 1,
and
Hal stands for a chlorine, bromine or iodine.

13. Process according to claim 12 for preparing compounds of the formula II of claim 2, wherein the acetylene derivative of the formula XII is coupled with an aryl halide of the formula XIV 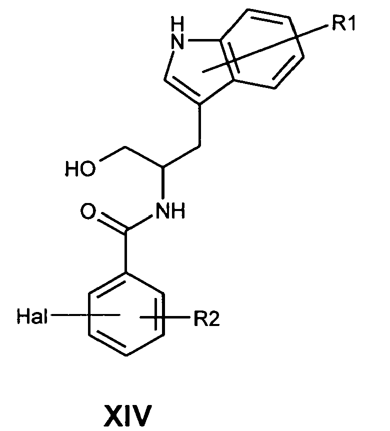 in which R1 and R2 have the same meaning as defined in claim 1,
and
Hal stands for a chlorine, bromine or iodine.

14. Process according to claim 12 for preparing compounds of the formula III of claim 3, wherein the acetylene derivative of the formula XII is coupled with an aryl halide of the formula XV 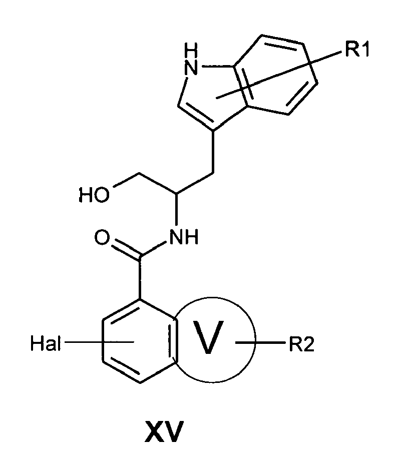 in which R1 and R2 have the same meaning as defined in claim 1,
and
Hal stands for a chlorine, bromine or iodine.

15. Process according to claim 12 for preparing compounds of the formula IV of claim 4, wherein the acetylene derivative of the formula XII is coupled with an aryl halide of the formula XVI 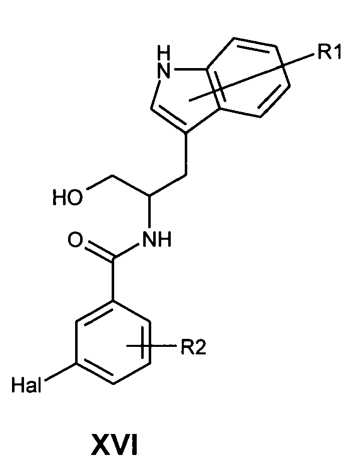 in which R1 and R2 have the same meaning as defined in claim 1,
and
Hal stands for a chlorine, bromine or iodine.

16. Process according to claim 12 for preparing compounds of the formula V of claim 5, wherein the acetylene derivative of the formula XII is coupled with an aryl halide of the formula XVII 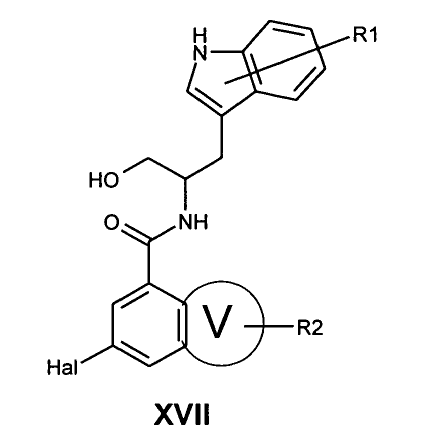 in which R1 and R2 have the same meaning as defined in claim 1,
and
Hal stands for a chlorine, bromine or iodine.

17. Pharmaceutical compositions comprising at least one of the compounds according to any of claims 1 to 6 with pharmaceutically suitable excipients and/or carriers.

18. Use of the compounds of the general formula I according to any of claims 1 to 6 for the fertility control in men or in women.

19. Process for producing medicaments comprising at least one of the compounds of the general formula I according to any of claims 1 to 6 for the prevention and/or treatment of osteoporosis.
